(19) Europäisches Patentamt
European Patent Office
Office européen des brevets

(11) **EP 3 309 553 A1**

(12) **EUROPEAN PATENT APPLICATION**
published in accordance with Art. 153(4) EPC

(43) Date of publication:
**18.04.2018 Bulletin 2018/16**

(51) Int Cl.:
**G01N 33/68** (2006.01)

(21) Application number: **15894866.1**

(86) International application number:
**PCT/JP2015/002892**

(22) Date of filing: **09.06.2015**

(87) International publication number:
**WO 2016/199180 (15.12.2016 Gazette 2016/50)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA**

(71) Applicant: **Osaka University**
**Suita-shi, Osaka 565-0871 (JP)**

(72) Inventors:
• **YOSHIZAKI, Kazuyuki**
**Suita-shi**
**Osaka 565-0871 (JP)**

• **UNO, Kazuko**
**Kyoto-shi**
**Kyoto 606-8225 (JP)**
• **IWAHASHI, Mitsuhiro**
**Higashihiroshima-shi**
**Hiroshima 739-0002 (JP)**
• **YAGI, Katsumi**
**Kyoto-shi**
**Kyoto 606-8225 (JP)**

(74) Representative: **Uexküll & Stolberg**
**Partnerschaft von**
**Patent- und Rechtsanwälten mbB**
**Beselerstraße 4**
**22607 Hamburg (DE)**

(54) **METHOD FOR PREDICTING/EVALUATING THERAPEUTIC EFFECT OF BIOLOGICAL PREPARATION ON RHEUMATOID ARTHRITIS**

(57) The present invention provides a method of predicting and determining a therapeutic effect (especially whether complete remission is reached) or the level of improvement prior to administration of a biological formulation such as an anti-IL-6 agent or an anti-TNF-$\alpha$ agent, which is simple and cost-effective, and accurate. Sgp130, IP-10, sTNFRI, sTNFRII, GM-CSF, IL-I$\beta$, IL-2, IL-5, IL-6, IL-7, IL-8, IL-9, IL-10, IL-12, IL-13, IL-15, Eotaxin, VEGF, MCP-1, TNF-$\alpha$, IFN-$\gamma$, FGFbasic, PDGF-bb, sIL-6R, MIP-1$\alpha$ and the like can be utilized as a specific marker used in the method. Since a therapeutic effect (level of improvement in a symptom or possibility of remission) on a rheumatoid arthritis patient can be determined prior to the administration of a biological formulation using such a specific marker, rheumatoid arthritis therapy is possible at a precision that could not be achieved conventionally.

EP 3 309 553 A1

**Description**

[Technical Field]

The present invention relates to a method of predicting and determining a therapeutic effect of a biological formulation on a rheumatoid arthritis patient. More specifically, the present invention relates to a method of predicting and determining a therapeutic effect on rheumatoid arthritis due to a biological formulation comprising an anti-IL-6 agent and an anti-TNF-a agent. Even more specifically, the present invention relates to a method of predicting and determining a therapeutic effect, such as the level of improvement in a symptom or the possibility of remission, prior to the administration of a biological formulation to a rheumatoid arthritis patient. Furthermore, the present invention relates to a diagnostic agent for predicting and determining a therapeutic effect due to a biological formulation on a rheumatoid arthritis patient. More specifically stated, the present invention relates to a technique for determining in advance a therapy with a biological formulation comprising an anti-interleukin-6 (IL-6) agent and an anti-tumor necrosis factor-$\alpha$ (TNF-$\alpha$) agent to provide an effective therapeutic agent to a patient.

[Background Art]

Rheumatoid arthritis is a systemic inflammatory disease, which is predominantly a lesion in the articular synovial membrane. It is estimated that approximately 700,000 people suffer from rheumatoid arthritis in Japan. Many biological formulations that target inflammatory cytokines have been developed for rheumatoid arthritis therapy. In recent years, anti-TNF-$\alpha$ agents or anti-IL-6 agents, which inhibit TNF-$\alpha$ or IL-6 action, have been used in clinical practices.

Conventionally, biological formulations targeting an inflammatory cytokine, such as tocilizumab, sirukumab, etanercept, adalimumab, or infliximab, have been used in rheumatoid arthritis therapy. Tocilizumab is a humanized IL-6 receptor antibody, which is an agent that causes rheumatoid arthritis to subside by the action of binding to a membrane-binding IL-6 receptor and a soluble IL-6 receptor to suppress IL-6 signaling. Sirukumab is a human anti-IL-6 antibody, which is an agent that causes rheumatoid arthritis to subside by the action of binding to IL-6 to suppress IL-6 signaling. Although such formulations that target IL-6 signaling have various targets, the working mechanism after the formulation binds to a target is similar. Thus, the inhibition mechanism is similar regardless of the type of agent. For this reason, such formulations are generally called anti-IL-6 agents. For example, tocilizumab, sarilumab, olokizumab, sirukumab and the like are classified as an anti-IL-6 agent. Meanwhile, etanercept is a fully human soluble TNF/LT$\alpha$ receptor formulation consisting of a subunit dimer of an extracellular domain of a human tumor necrosis factor II receptor and an Fc region of a human IgG1. Etanercept is an agent that binds to both TNF$\alpha/\beta$ to inhibit signaling to a TNF receptor to cause rheumatoid arthritis to subside. Adalimumab and infliximab are human and chimeric TNF-$\alpha$ antibodies, which are agents that cause rheumatoid arthritis to subside by specifically binding to excessively produced TNF-$\alpha$ and inhibiting the binding of TNF-$\alpha$ to a TNF-$\alpha$ receptor. Although such formulations that target TNF-$\alpha$ signaling have various targets, the working mechanism after the formulation binds to a target is similar. Thus, the inhibition mechanism is similar regardless of the type of agent. For this reason, such formulations are generally called anti-TNF-a agents. For example, etanercept, adalimumab, infliximab, golimumab, certolizumab and the like are classified as an anti-TNF-$\alpha$ agent.

For such biological formulations, a certain level of effectiveness in rheumatoid arthritis therapy is verified. The biological formulations have seen increase in variety and popularity. Meanwhile, such formulations have disadvantages such as the formulations being expensive and time-intensive for determining a therapeutic effect. In addition, there are certain percentages of cases with no effect, and expression of side effects, such as an infectious disease or an interstitial pneumonia, has been observed in some cases. For this reason, cases where the biological formulation is usable are limited. Thus, if the effectiveness of a biological formulation targeting an inflammatory cytokine can be estimated in advance for each rheumatoid arthritis patient, this would be a boon to rheumatoid arthritis patients and provide contribution to medical business. That is, this can be a logical and excellent tool for reducing social security expenses for government agencies in charge of social security expenses, such as the Ministry of Health, Labour and Welfare. For patients, it would be possible to receive high quality therapy by avoiding a situation where a patient realizes that a medicament is ineffective after a symptom is exacerbated. For pharmaceutical manufacturers, reliability of biological formulations is enhanced, so that the possibility of a patient, who have conventionally not selected a biological formulation, using such a formulation would drastically increase.

Markers for predicting the effectiveness of a biological formulation targeting an inflammatory cytokine on a rheumatoid arthritis patient have been intensively investigated. For example, a method of using a microDNA chip, a method of using a CRP value at baseline as an indicator, a method of using blood soluble ICAMl concentration and CXCL13 concentration as indicators, a method of using leukocyte ADAMT5 gene expression amount as an indicator (Non Patent Literature 4), a method of comprehensively analyzing genetic polymorphisms (Patent Literatures 1 and 2), a method of analyzing a genetic mutation of an IL-6 receptor (Patent Literature 3) and the like have been reported as a method of predicting the therapeutic effectiveness of tocilizumab on rheumatoid arthritis. Further, a method of analyzing the IL10RB gene, the IRF5 gene, and polymorphisms of the IRF5 gene (Patent Literature 4) has been reported as a method of predicting the therapeutic effectiveness of infliximab on rheumatoid arthritis. Furthermore, a method of comprehensively analyzing genetic polymorphisms (Patent literature 5) has been reported as a method of predicting the therapeutic effectiveness of an anti-TNF-α agent such as etanercept, adalimumab, or infliximab on rheumatoid arthritis.

However, conventional methods of determining a therapeutic effect on rheumatoid arthritis have disadvantages such as: genetic analysis or the like is required, in addition to the operation being complicated; analysis is time and cost-intensive; there is little versatility; proper diagnosis rate is low; and the like. Furthermore, conventional approaches cannot accurately determine whether rheumatoid arthritis can be in full remission prior to the administration of a biological formulation. Thus, conventional approaches have a problem in that an appropriate therapeutic plan which takes into consideration the therapeutic effect thereof cannot be established prior to administration of a biological formulation.

Such background conventional techniques elicit a desire for the establishment of a technique for predicting a therapeutic effect of biological formulation administration to a rheumatoid arthritis patient, which is simple and cost-efficient, highly versatile and highly accurate.

[Citation List]

[Patent Literature]

[PTL 1] International Publication No. WO 2011/128096
[PTL 2] Japanese Laid-Open Publication No. 2011-182780
[PTL 3] International Publication No. WO 2012/41332
[PTL 4] Japanese Laid-Open Publication No. 2009-225713
[PTL 5] Japanese Laid-Open Publication No. 2010-088432

[Summary of Invention]

[Solution to Problem]

The present invention provides a method of predicting and determining a therapeutic effect (level of improvement in a symptom or possibility of remission) prior to administration of a biological formulation such as an anti-IL-6 agent or an anti-TNF-α agent, which is simple and cost-effective, highly versatile and highly accurate. The present invention also provides a diagnostic agent for carrying out the above-described method and a therapeutic agent comprising a biological formulation such as an anti-IL-6 agent or an anti-TNF-α agent characterized in carrying out the above-described method.

The inventors have discovered that it is possible to predict and determine the possibility of remission, level of improvement in a symptom and disease activity indicator for rheumatoid arthritis due to the use of a biological formulation such as an anti-IL-6 agent or an anti-TNF-α agent by analyzing the prognostic state of a rheumatoid arthritis patient administered with the biological formulation and the concentrations of cytokines, chemokines and soluble receptors thereof in a bodily fluid sample such as a serum of the patient prior to administration of the biological formulation and conducting a backward-looking analysis. As a result of the analysis, the inventors have discovered that the following markers can be utilized as such a cytokine, chemokine or a soluble receptor for determination: sgp130, IP-10, sTNFRI, sTNFRII, GM-CSF, IL-1β, IL-2, IL-5, IL-6, IL-7, IL-8, IL-9, IL-10, IL-12, IL-13, IL-15, Eotaxin, VEGF, MCP-1, TNF-α, IFN-γ, FGFbasic, PDGF-bb, sIL-6R, and MIP-1α. The inventors have discovered that a therapeutic effect on a rheumatoid arthritis patient (e.g., possibility of remission, level of improvement in a symptom, and disease activity indicator) can be determined (predicted and determined) in advance in a simple and cost-effective manner at any facility with high accuracy, prior to administering a biological formulation targeting an inflammatory cytokine such as IL-6 or TNF-α by utilizing the concentration of these specific markers in a body sample (e.g., in serum).

More specifically, the inventors have obtained the following knowledge.

(1) Analysis of level of improvement (level of improvement in DAS-28 value) after therapy

(1-1) It was discovered through simple linear regression analysis that when therapy is applied by administering an anti-IL-6 agent (e.g., tocilizumab or the like) to a rheumatism patient who has not received anti-cytokine therapy (administration of a biological formulation such as infliximab, etanercept, adalimumab, or tocilizumab) in the past (referred to as a "naive patient" herein), the level of improvement in the DAS-28 value (DAS-28 value prior to therapy - DAS-28 value after 16 weeks of therapy) is significantly correlated with the log values of serum concentrations of IL-7, IL-8, IL-12, IL-13, IP-10, and VEGF prior to the administration of the anti-IL-6 agent (e.g., tocilizumab or the like) to the patient. In this regard, DAS is a specific value representing not only complaint regarding pain from a patient, but also the intensity of a symptom of rheumatoid arthritis which is comprehensively evaluated by combining joint and blood tests. DAS-28 refers to a result of examining 28 joints, while indicators such as DAS-44, which examines 44 joints, are also known.

(1-2) It was discovered through simple linear regression analysis that when therapy is applied by administering an anti-IL-6 agent (e.g., tocilizumab or the like) to a rheumatism patient who has received anti-cytokine therapy in the past (referred to as a "switch patient" herein; also referred to as a "non-naive patient" in the art), the level of improvement in the DAS-28 value is significantly correlated with the log values of serum concentrations of IL-1$\beta$, IL-5, IL-6, IL-7, IL-10, IL-12, IL-13, IL-15, FGFbasic, GM-CSF, IFN-$\gamma$, TNF-$\alpha$, and VEGF prior to the administration of the anti-IL-6 agent (e.g., tocilizumab or the like) to the patient.

(1-3) It was discovered through simple linear regression analysis that when therapy is applied by administering an anti-TNF-$\alpha$ agent (e.g., etanercept or the like) to a naive patient, the level of improvement in DAS-28 value is significantly correlated with the log values of serum concentrations of IL-6 and IP-10 prior to the administration of the anti-TNF-$\alpha$ agent (e.g., etanercept or the like) to the patient.

(1-4) It was discovered through multiple linear regression analysis that when therapy is applied by administering an anti- IL-6 agent (e.g., tocilizumab or the like) to a naive patient, the level of improvement in DAS-28 value is significantly correlated with a combination of log values of serum concentrations of IL-1$\beta$, IL-7, TNF-$\alpha$, and IL-6R prior to the administration of the anti-IL-6 agent (e.g., tocilizumab or the like) to the patient.

(1-5) It was discovered through multiple linear regression analysis that when therapy is applied by administering an anti-TNF-$\alpha$ agent (e.g., etanercept or the like) to a naive patient, the level of improvement in DAS-28 value is significantly correlated with a combination of log values of serum concentrations of IL-2, IL-15, sIL-6R, and sTNFRI prior to the administration of the anti-TNF-$\alpha$ agent (e.g., etanercept or the like) to the patient.

(2) Analysis of disease activity indicator (DAS-28 value after 16 weeks of therapy)

(2-1) It was discovered through simple linear regression analysis that when therapy is applied by administering an anti-IL-6 agent (e.g., tocilizumab or the like) to a naive patient, the DAS-28 value after 16 weeks of therapy is significantly correlated with a serum concentration of sgp130 prior to the administration of the anti-IL-6 agent (e.g., tocilizumab or the like) to the patient.

(2-2) It was discovered through simple linear regression analysis that when therapy is applied by administering an anti-IL-6 agent (e.g., tocilizumab or the like) to a switch patient, the DAS-28 value after 16 weeks of therapy is significantly correlated with the log values of serum concentrations of IL-1$\beta$, IL-2, IL-5, IL-15, GM-CSF, IFN-$\gamma$, and TNF-$\alpha$ and a serum concentration of sgp130 prior to the administration of the anti-IL-6 agent (e.g., tocilizumab or the like) to the patient.

(2-3) It was discovered through simple linear regression analysis that when therapy is applied by administering an anti-TNF-a agent (e.g., etanercept or the like) to a naive patient, the DAS-28 value after 16 weeks of therapy is significantly correlated with the log value of a serum concentration of IL-9 prior to the administration of the anti-TNF-$\alpha$ agent (e.g., etanercept or the like) to the patient.

(2-4) It was discovered through multiple linear regression analysis that when therapy is applied by administering an anti-IL-6 agent (e.g., tocilizumab or the like) to a naive patient, the DAS-28 value after 16 weeks of therapy is significantly correlated with a combination of log values of serum concentrations of IL-8, Eotaxin, IP-10, sTNRFI, sTNFRII, IL-6 and VEGF and a serum concentration of sgp130 prior to the administration of the anti-IL-6 agent (e.g., tocilizumab or the like) to the patient.

(2-5) It was discovered through multiple linear regression analysis that when therapy is applied by administering an anti-IL-6 agent (e.g., tocilizumab or the like) to a naive patient, the DAS-28 value after 16 weeks of therapy is significantly correlated with a combination of log values of serum concentrations of IL-8, Eotaxin, IP-10, sTNFRI, sTNRFII, and IL-6 and a serum concentration of sgp130 prior to the administration of the anti-IL-6 agent (e.g., tocilizumab or the like) to the patient.

(2-6) It was discovered through multiple linear regression analysis that when therapy is applied by administering an anti-IL-6 agent (e.g., tocilizumab or the like) to a switch patient, the DAS-28 value after 16 weeks of therapy is significantly correlated with a combination of log values of serum concentrations of IP-10 and GM-CSF and a serum concentration of sgp130 prior to the administration of the anti-IL-6 agent (e.g., tocilizumab or the like) to the patient.

(2-7) It was discovered through multiple linear regression analysis that when therapy is applied by administering an anti-TNF-$\alpha$ agent (e.g., etanercept or the like) to a naive patient, the DAS-28 value after 16 weeks of therapy is significantly correlated with a combination of log values of serum concentrations of IL-6 and IL-13 and the DAS-28 value prior to the administration of the anti-TNF-a agent (e.g., etanercept or the like).

(2-8) It was discovered through multiple linear regression analysis that when therapy is applied by administering an anti-TNF-a agent (e.g., etanercept or the like) to a naive patient, the DAS-28 value after 16 weeks of therapy is significantly correlated with a combination of log values of serum concentrations of IL-9, TNF-$\alpha$, and VEGF prior to the administration of the anti-TNF-$\alpha$ agent (e.g., etanercept or the like).

(3) Analysis of possibility of remission

(3-1) It was discovered through multiple logistic regression analysis that the possibility of remission, when therapy is applied by administering an anti-IL-6 agent (e.g., tocilizumab or the like) to a naive patient can be predicted and determined by combining a serum concentration of sgp130, a log value of a serum concentration of IP-10, a log value of a serum concentration of sTNFRII, and a log value of a serum concentration of IL-6, IL-7, MCP-1, or IL-1$\beta$ prior to the administration of the anti-IL-6 agent (e.g., tocilizumab or the like).

(3-2) It was discovered through multiple logistic regression analysis that the possibility of remission, when therapy is applied by administering an anti-IL-6 agent (e.g., tocilizumab or the like) to a switch patient, can be predicted and determined by combining a serum concentration of sgp130, a log value of a serum concentration of IP-10, a log value of a serum concentration of sTNFRII, and a log value of a serum concentration of IL-6 or IL-1$\beta$ prior to the administration of the anti-IL-6 agent (e.g., tocilizumab or the like).

(3-3) It was discovered through multiple logistic regression analysis that the possibility of remission, when therapy is applied by administering an anti-TNF-$\alpha$ agent (e.g., etanercept or the like) to a naive patient, can be predicted and determined by combining the DAS-28 value and log values of serum concentrations of VEGF and PDGF-bb prior to the administration of the anti-TNF-$\alpha$ agent (e.g., etanercept or the like).

(3-4) It was discovered through multiple logistic regression analysis that the possibility of remission, when therapy is applied by administering an anti-TNF-$\alpha$ agent (e.g., etanercept or the like) to a naive patient, can be predicted and determined by combining the DAS-28 value and log values of serum concentrations of MIP-1$\alpha$ and PDGF-bb prior to the administration of the anti-TNF-$\alpha$ agent (e.g., etanercept or the like).

(3-5) It was discovered through multiple linear regression analysis that the possibility of remission, when therapy is applied by administering an anti-TNF-$\alpha$ agent (e.g., etanercept or the like) to a naive patient, can be predicted and determined by combining log values of serum concentrations of IL-9 and TNF-$\alpha$ prior to the administration of the anti-TNF-$\alpha$ agent (e.g., etanercept or the like).

The present invention was completed by additional repeated examinations based on such knowledge. Specifically, the present invention provides inventions in the following embodiments.

(Item 1) A method of determining in advance remission for a rheumatoid arthritis patient due to a specific biological formulation by measuring a concentration of a specific marker in a body sample of the patient.

(Item 2) The method of item 1, wherein the specific biological formulation is an anti-IL-6 agent, and the specific marker comprises a combination of sgp130 and at least one selected from the group consisting of IP-10, sTNFRII, IL-6, IL-7, MCP-1 and IL-1$\beta$.

(Item 3) The method of item 1 or 2, wherein the specific biological formulation is an anti-IL-6 agent, and the specific marker comprises a combination of (i) sgp130, (ii) IP-10, (iii) sTNFRII, and (iv) IL-6, IL-7, MCP-1 or IL-I$\beta$.

(Item 4) The method of any one of items 1-3, wherein the specific biological formulation is an anti-IL-6 agent, the patient is a rheumatoid arthritis patient who has received anti-cytokine therapy in the past, and the marker is a combination of (i) sgp130, (ii) IP-10, (iii) sTNFRII, and (iv) IL-6 or IL-1$\beta$.

(Item 5) The method of item 1, wherein the specific biological formulation is an anti-TNF-$\alpha$ agent, and the specific marker comprises a combination of IL-9 and TNF-$\alpha$ or a combination of VEGF or MIP-1a, PDGFbb and an indicator of a condition prior to therapy of the patient.

(Item 6) The method of item 1 or 5, wherein the specific biological formulation is an anti-TNF-$\alpha$ and the specific marker comprises a combination of IL-9 and TNF-$\alpha$.

(Item 7) The method of any one of items 1-6, wherein the body sample is a serum.

(Item 7A) The method of any one of items 1-7, wherein the patient is a rheumatoid arthritis patient who has received anti-cytokine therapy in the past.

(Item 7B) The method of any one of items 1-3 and 5-7, wherein the patient is a rheumatoid arthritis patient who has not received anti-cytokine therapy in the past.

(Item 8) The method of any one of items 1-7, 7A, and 7B, wherein remission for the patient is determined in advance based on a probability of remission calculated from a regression equation using a value of a concentration of the specific marker or a log value thereof or an indicator of a condition of the patient prior to therapy.

(Item 9) The method of item 8, wherein calculation with the regression equation is performed by using a value of a concentration of the sgpl30 and a log value of a concentration for the other specific markers.

(Item 10) The method of item 9, wherein the regression equation is selected from one of regression equations (8)-(16). The details for regression equations (8)-(16) are described in "1. Determining method" described in the present specification.

(Item 11) A method of selecting a biological formulation that is effective for the patient by determining in advance remission due to the specific biological formulation in accordance with the method of any one of items 1-7, 7A, 7B, and 8-10 and selecting a specific biological formulation with a high probability of remission.

(Item 12) A method of treating a rheumatoid arthritis patient comprising (A) measuring a concentration of a specific marker in a body sample of the rheumatoid arthritis patient to determine in advance remission for the patient due to a specific biological formulation, and (B) when it is determined that remission would occur due to the specific biological formulation by step (A), administering to the patient the specific biological formulation.

(Item 13) A method of treating a rheumatoid arthritis patient comprising (A) measuring a concentration of a specific marker in a body sample of the rheumatoid arthritis patient to calculate in advance a probability of remission for the patient due to a plurality of specific biological formulations, and (B) administering to the patient a specific biological formulation with a high probability of remission obtained from step (A).

(Item 13A) The method of item 12 or 13, comprising a feature of any one of items 2-7, 7A, 7B and 8-10.

(Item 14) A diagnostic agent comprising a reagent for detecting a specific marker, wherein the diagnostic agent is used in a method of measuring a concentration of the specific marker in a body sample of a rheumatoid arthritis patient to determine in advance remission for the patient due to a specific biological formulation.

(Item 15) A diagnostic agent comprising a reagent for detecting a specific marker, wherein the diagnostic agent is used in a method of selecting a biological formulation that is effective for a rheumatoid arthritis patient by measuring a concentration of the specific marker in a body sample of the patient, calculating in advance a probability of remission for the patient due to a plurality of specific biological formulations, and selecting a specific biological formulation with a high probability of remission.

(Item 15A) The diagnostic agent of item 14 or 15, comprising a feature of any one of items 2-7, 7A, 7B and 8-10.

(Item 16) A therapeutic agent for treating a rheumatoid arthritis patient comprising a specific biological formulation, characterized in that a concentration of a specific marker in a body sample of the patient is measured to determine in advance remission for the patient due to the specific biological formulation and when it is determined that remission would occur, the specific biological formulation is administered.

(Item 16A) A set of therapeutic agents 'for treating a rheumatoid arthritis patient comprising a plurality of specific biological formulations, characterized in that a concentration of a specific marker in a body sample of the patient is measured to calculate in advance a probability of remission for the patient due to the specific biological formulations and a specific biological formulation with a high probability of remission is administered to the patient.

(Item 16B) The therapeutic agent of item 16 or the set of therapeutic agents of item 16A, comprising a feature of any one of items 2-7, 7A, 7B and 8-10.

(Item 17) A method of measuring a concentration of a specific marker in a body sample of a rheumatoid arthritis patient to determine in advance a level of improvement in a symptom after therapy for the patient due to a specific biological formulation.

(Item 18) The method of item 17, wherein the specific biological formulation is an anti-IL-6 agent and the specific marker comprises a combination of IL-1$\beta$, IL-7, TNF-$\alpha$, and sIL-6R.

(Item 19) The method of item 17, wherein the specific biological formulation is an anti-TNF-$\alpha$ agent and the specific marker comprises a combination of IL-2, IL-15, sIL-6R, and sTNFRI or a combination of IL-6 and IL-13.

(Item 20) The method of any one of items 17-19, wherein the body sample is a serum.

(Item 20A) The method of any one of items 17-20, wherein the patient is a rheumatism patient who has not received anti-cytokine therapy in the past.

(Item 20B) The method of any one of items 17-20, wherein the patient is a rheumatism patient who has received anti-cytokine therapy in the past.

(Item 21) The method of any one of items 17-20, 20A and 20B, wherein the level of improvement in a symptom after therapy is determined in advance based on a probability of remission calculated from a regression equation using a value of the concentration of the specific marker or a log value thereof or an indicator of a condition of the patient prior to therapy.

(Item 22) The method of item 21, wherein calculation with the regression equation is performed by using a value of a concentration of the sgp130 and a log value of a concentration for the other specific markers.

(Item 23) The method of item 22, wherein the regression equation is selected from one of regression equations (1)-(2). The details for regression equations (1)-(2) are described in "1. Determining method" described in the present specification.

(Item 24) A method of selecting a biological formulation that is effective for the patient by determining in advance a level of improvement in a symptom after therapy due to the specific biological formulation in accordance with the method of any one of items 17-20, 20A, 20B, and 21-23 and selecting a specific biological formulation with a high level of improvement in a symptom after therapy.

(Item 25) A method of treating a rheumatoid arthritis patient comprising (A) measuring a concentration of a specific marker in a body sample of the rheumatoid arthritis patient to determine in advance a level of improvement in a symptom after therapy for the patient due to a specific biological formulation, and (B) when the level of improvement determined by step (A) is at or above a predetermined baseline, administering to the patient the specific biological formulation.

(Item 26) A method of treating a rheumatoid arthritis patient comprising (A) measuring a concentration of a specific marker in a body sample of the rheumatoid arthritis patient to determine in advance a level of improvement in a symptom after therapy for the patient due to a plurality of specific biological formulations, and (B) administering to the patient a specific biological formulation with a high level of improvement obtained from step (A).

(Item 26A) The method of item 25 or 26, comprising a feature of any one of items 17-20, 20A, 20B and 21-23.

(Item 27) A diagnostic agent comprising a reagent for detecting a specific marker, wherein the diagnostic agent is used in a method of measuring a concentration of the specific marker in a body sample of a rheumatoid arthritis patient to determine in advance a level of improvement in a symptom after therapy for the patient due to a specific biological formulation.

(Item 28) A diagnostic agent comprising a reagent for detecting a specific marker, wherein the diagnostic agent is used in a method of selecting a biological formulation that is effective for a rheumatoid arthritis patient by measuring a concentration of the specific marker in a body sample of the patient, determining in advance a level of improvement in a symptom after therapy for the patient due to a plurality of specific biological formulations, and selecting a specific biological formulation with a high level of improvement.

(Item 28A) The diagnostic agent of item 27 or 28, comprising a feature of any one of items 17-20, 20A, 20B and 21-23.

(Item 29) A therapeutic agent for treating a rheumatoid arthritis patient comprising a specific biological formulation, characterized in that a concentration of a specific marker in a body sample of the rheumatoid arthritis patient is measured to determine in advance a level of improvement in a symptom after therapy for the patient due to the specific biological formulation and when the level of improvement is at or above a predetermined baseline, the specific biological formulation is administered.

(Item 29A) A set of therapeutic agents for treating a rheumatoid arthritis patient comprising a plurality of specific biological formulations, characterized in that a concentration of a specific marker in a body sample of the patient is measured to calculate in advance a level of improvement in a symptom after therapy for the patient due to the specific biological formulations and a specific biological formulation with a high level of improvement is administered to the patient.

(Item 29B) The therapeutic agent of item 29 or the set of therapeutic agents of item 29A, comprising a feature of any one of items 17-20, 20A, 20B and 21-23.

(Item 30) A method of measuring a concentration of a specific marker in a body sample of a rheumatoid arthritis patient to determine in advance a disease activity indicator after therapy for the patient due to a specific biological formulation to the patient.

(Item 31) The method of item 30, wherein the specific biological formulation is an anti-IL-6 agent and the specific marker comprises a combination of sgp130, IP-10, and at least one selected from IL-8, Eotaxin, sTNFRI, sTNFRII, IL-6, VEGF, and GM-CSF.

(Item 32) The method of item 30 or 31, wherein the specific biological formulation is an anti-IL-6 agent and the specific marker comprises a combination of sgp130, IL-8, Eotaxin, IP-10, sTNFRI, sTNFRII, and IL-6, or a combination of sgp130, IL-8, Eotaxin, IP-10, sTNFRI, sTNFRII, IL-6, and VEGF, and wherein the patient is a rheumatism patient who has not received anti-cytokine therapy in the past.

(Item 33) The method of item 30 or 31, wherein the specific biological formulation is an anti-IL-6 agent and the specific marker comprises a combination of sgp130, IL-10, and GM-CSF, and wherein the patient is a rheumatism patient who has received anti-cytokine therapy in the past.

(Item 34) The method of item 30, wherein the specific biological formulation is an anti-TNF-$\alpha$ agent and the specific marker comprises a combination of IL-9, TNF-$\alpha$, and VEGF or a combination of IL-6 and IL-13.

(Item 34A) The method of claim 34, wherein the patient is a rheumatism patient who has not received anti-cytokine therapy in the past.

(Item 34B) The method of claim 34, wherein the patient is a rheumatism patient who has received anti-cytokine therapy in the past.

(Item 35) The method of any one of items 30-34, 34A and 34B, wherein the body sample is a serum.

(Item 36) The method of any one of items 30-34, 34A, 34B, and 35, wherein the disease activity indicator after therapy is determined in advance based on a disease activity indicator after therapy calculated with a regression equation using a value of a concentration of the specific marker or a log value thereof or an indicator of a condition of the patient prior to therapy.

(Item 37) The method of item 36, wherein calculation with the regression equation is performed by using a value of a concentration of the sgp130 and a log value of a concentration for the other specific markers.

(Item 38) The method of item 37, wherein the regression equation is selected from one of regression equations (3)-(7). The details for regression equations (3)-(7) are described in "1. Determining method" described in the present specification.

(Item 39) A method of selecting a biological formulation that is effective for the patient by determining in advance a disease activity indicator after therapy for the patient due to a specific biological formulation in accordance with the method of any one of items 30-34, 34A, '34B, and 35 and selecting a specific biological formulation with the disease activity indicator that is lower than a predetermined baseline.

(Item 40) A method of treating a rheumatoid arthritis patient comprising (A) measuring a concentration of a specific marker in a body sample of the rheumatoid arthritis patient to determine in advance a disease activity indicator after therapy for the patient due to a specific biological formulation, and (B) when the disease activity indicator is at or below a predetermined baseline according to step (A), administering to the patient the specific biological formulation.

(Item 41) A method of treating a rheumatoid arthritis patient comprising (A) measuring a concentration of a specific marker in a body sample of the rheumatoid arthritis patient to determine in advance a disease activity indicator after therapy for the patient due to a plurality of specific biological formulations, and (B) administering to the patient a specific biological formulation with a low disease activity indicator obtained from step (A).

(Item 41A) The method of item 40 or 41, comprising a feature of any one of items 30-34, 34A, 34B and 35.

(Item 42) A diagnostic agent comprising a reagent for detecting a specific marker, wherein the diagnostic agent is used in a method of measuring a concentration of the specific marker in a body sample of a rheumatoid arthritis patient to determine in advance a disease activity indicator after therapy for the patient due to a specific biological formulation.

(Item 43) A diagnostic agent comprising a reagent for detecting a specific marker, wherein the diagnostic agent is used in a method of selecting a biological formulation that is effective for a rheumatoid arthritis patient by measuring a concentration of the specific marker in a body sample of the patient, determining in advance a disease activity indicator after therapy for the patient due to a plurality of specific biological formulations, and selecting a specific biological formulation with a low disease activity indicator.

(Item 43A) The diagnostic agent of item 42 or 43, comprising a feature of any one of items 30-34, 34A, 34B and 35.

(Item 44) A therapeutic agent for treating a rheumatoid arthritis patient comprising a specific biological formulation, characterized in that a concentration of a specific marker in a body sample of the patient is measured to determine in advance a disease activity indicator after therapy for the patient due to the specific biological formulation and when the disease activity indicator is at or below a predetermined baseline, the specific biological formulation is administered.

(Item 44A) A set of therapeutic agents for treating a rheumatoid arthritis patient comprising a plurality of specific biological formulations, characterized in that a concentration of a specific marker in a body sample of the patient is measured to determine in advance a disease activity indicator after therapy for the patient due to the plurality of specific biological formulations and a specific biological formulation with a low disease activity indicator is administered.

(Item 44B) The therapeutic agent of item 44 or the set of therapeutic agents of item 44A, comprising a feature of any one of items 30-34, 34A, 34B and 35.

In another aspect, the present invention also provides the following.

Item A1. A method of predicting and determining a therapeutic effect of a biological formulation targeting an inflammatory cytokine on a rheumatoid arthritis patient, characterized in comprising the step of measuring a concentration of at least one type of specific marker selected from the group consisting of sgp130, IP-10, sTNFRI, sTNFRII, GM-CSF, IL-1$\beta$, IL-2, IL-5, IL-6, IL-7, IL-8, IL-9, IL-10, IL-12, IL-13, IL-15, Eotaxin, VEGF, MCP-1, TNF-$\alpha$, IFN-$\gamma$, FGFbasic, PDGF-bb, sIL-6R, and MIP-1$\alpha$ in a serum collected from the rheumatoid arthritis patient prior to the administration of the biological formulation.

Item A2. The method of item A1, wherein

the method is a method of predicting and determining a possibility of remission with tocilizumab, and
the specific marker is at least one type selected from the group consisting of sgp130, IP-10, sTNFRII, IL-6, IL-7, MCP-1, and IL-1$\beta$.

Item A3. The method of determining of item A2, wherein at least sgp130 is used as the specific marker.
Item A4. The method of determining of item A2 or A3, wherein

a patient to be administered with tocilizumab is a rheumatoid arthritis patient who has not received anti-cytokine therapy in the past, and
the specific marker is a combination of (i) sgp130, (ii) IP-10, (iii) sTNFRII, and (iv) IL-6, IL-7, MCP-1 or IL-1β.
Item A5. The method of determining of item A2 or A3, wherein
a patient to be administered with tocilizumab is a rheumatoid arthritis patient who has received anti-cytokine therapy in the past, and
the specific marker is a combination of (i) sgp130, (ii) IP-10, (iii) sTNFRII, and (iv) IL-6 or IL-1β.

Item A6. The method of determining of item A1, wherein

the method is a method of predicting and determining a possibility of remission with etanercept in a rheumatism patient who has not received anti-cytokine therapy in the past, and
the specific marker is at least one type selected from the group consisting of IL-9, TNF-α, VEGF, PDGF-bb, and MIP-1α.

Item A7. The method of determining of item A6, wherein the specific marker is a combination of IL-9 and TNF-α, a combination of VEGF and PDGF-bb, or a combination of MIP-1α and PDGF-bb.
Item A8. The method of determining of item A1, wherein

the method is a method of predicting and determining a disease activity indicator after therapy with tocilizumab in a rheumatism patient who has not received anti-cytokine therapy in the past, and
the specific marker is at least one type selected from the group consisting of sgp130, IP-8, Eotaxin, IP-10, sTNFRI, sTNFRII, IL-6, and VEGF.

Item A9. The method of determining of item A8, wherein the specific marker is a combination of sgp130, IL-8, Eotaxin, IP-10, sTNFRI, sTNFRII, and IL-6 or a combination of sgp130, IL-8, Eotaxin, IP-10, sTNFRI, sTNFRII, IL-6 and VEGF.
Item A10. The method of determining of item A1, wherein

the method is a method of predicting and determining a value of a disease activity indicator after therapy with tocilizumab in a rheumatism patient who has received anti-cytokine therapy in the past, and
the specific marker is at least one type selected from the group consisting of sgp130, IL-1β, IL-2, IL-5, IL-15, GM-CSF, IFN-γ, TNF-α, and IP-10.

Item A11. The method of determining of item A10, wherein the specific marker is a combination of sgp130, IP-10, and GM-CSF.
Item A12. The method of determining of item A1, wherein

the method is a method of predicting and determining a value of a disease activity indicator after therapy with etanercept in a rheumatism patient who has not received anti-cytokine therapy in the past, and
the specific marker is at least one type selected from the group consisting of IL-9, IL-6, IL-13, TNF-α, and VEGF.
Item A13. The method of determining of item A12, wherein
the specific marker is a combination of TNF-α and VEGF or a combination of IL-6 and IL-13.

Item A14. The method of determining of item A1, wherein

the method is a method of predicting and determining a level of improvement in a symptom after therapy with tocilizumab in a rheumatism patient who has not received anti-cytokine therapy in the past, and
the specific marker is at least one type selected from the group consisting of IL-7, IL-8, IL-12, IL-13, IP-10, VEGF, IL-1β, TNF-α, and sIL-6R.

Item A15. The method of determining of item A14, wherein the specific marker is a combination of IL-1β, IL-7, TNF-α, and sIL-6R.
Item A16. The method of determining of item A1, wherein

the method is a method of predicting and determining a level of improvement in a symptom after therapy with tocilizumab in a rheumatism patient who has received anti-cytokine therapy in the past, and
the specific marker is at least one type selected from the group consisting of IL-1β, IL-5, IL-6, IL-7, IL-10, IL-12, IL-13, IL-15, FGFbasic, GM-CSF, IFN-γ, TNF-α, and VEGF. Item A17. The method of determining of item A1, wherein

the method is a method of predicting and determining a level of improvement in a symptom after therapy with etanercept in a rheumatism patient who has not received anti-cytokine therapy in the past, and
the specific marker is at least one type selected from the group consisting of IL-6, IP-10, IL-2, IL-13, IL-15, sIL-6R, and sTNFRI.

Item A18. The method of determining of item A17, wherein the specific marker is a combination of IL-2, IL15, sIL-6R, and sTNFRI or a combination of IL-6 and IL-13.

Item A19. A method of selecting a more effective biological formulation for therapy in a rheumatism patient who has not received anti-cytokine therapy in the past from among biological formulations consisting of tocilizumab and etanercept, comprising:

predicting and determining a possibility of remission with tocilizumab in accordance with the method of determining of item A4;
predicting and determining a possibility of remission with etanercept in accordance with the method of determining of item A6; and
comparing the possibility of remission with tocilizumab with the possibility of remission with etanercept that were predicted determined in the aforementioned steps to select a biological formulation with a high possibility of remission. Item A20. A method of selecting a more effective biological formulation for therapy in a rheumatism patient who has not received anti-cytokine therapy in the past from among biological formulations consisting of tocilizumab and etanercept, comprising:
predicting and determining a disease activity indicator after therapy with tocilizumab in accordance with the method of determining of item A10 or All;
predicting and determining a disease activity indicator after therapy with etanercept in accordance with the method of determining of item A12 or A13; and
comparing the disease activity indicator after therapy with tocilizumab with the disease activity indicator after therapy with etanercept that were predicted and determined in the aforementioned steps to select a biological formulation with a low disease activity indicator after therapy.

Item A21. A method of selecting a more effective biological formulation for therapy in a rheumatism patient who has not received anti-cytokine therapy in the past from among biological formulations consisting of tocilizumab and etanercept, comprising:

predicting and determining a level of improvement in a symptom after therapy with tocilizumab in accordance with the method of determining of item A14 or A15;
predicting and determining a level of improvement in a symptom after therapy with etanercept in accordance with the method of determining of item A17 or A18; and
comparing the level of improvement in a symptom after therapy with tocilizumab with the level of improvement in a symptom after therapy with etanercept that were predicted in the aforementioned steps to select a biological formulation with a high level of improvement in a symptom after therapy. Item A22. A diagnostic agent for predicting and determining a therapeutic effect due to a biological formulation targeting an inflammatory cytokine on a rheumatoid arthritis patient, comprising a reagent capable of detecting at least one type of marker selected from the group consisting of sgp130, IP-10, sTNFRI, sTNFRII, GM-CSF, IL-1β, IL-2, IL-5, IL-6, IL-7, IL-8, IL-9, IL-10, IL-12, IL-13, IL-15, Eotaxin, VEGF, MCP-1, TNF-α, IFN-γ, FGFbasic, PDGF-bb, sIL-6R, and MIP-1α.

The present invention is intended such that one or more of the features described in each of the above-described items can be provided in more combinations, in addition to the explicitly described combinations. Those skilled in the art can understand further embodiments and advantages of the present invention by referring to the following Detailed Description of the Invention as needed.

[Advantageous Effects of Invention]

Accordingly to the present invention, a therapeutic effect on a rheumatoid arthritis patient can be accurately estimated, and whether rheumatoid arthritis would enter a state of complete remission due to a biological formulation can be determined with high precision, prior to the administration of the biological formulation targeting an inflammatory cytokine such as IL-6 or TNF-$\alpha$ (unless noted otherwise, referred to as a "biological formulation" herein). Furthermore, according to the present invention, a level of improvement in a symptom for a rheumatoid arthritis patient can be accurately determined prior to the administration of the biological formulation, thus allowing the establishment of a suitable treatment plan, which takes into consideration the therapeutic effect of the biological formulation. Further, according to the present invention, it is possible to predict which biological formulation is the most effective when administered for a rheumatoid arthritis patient prior to therapy. Thus, the most effective treatment plan can be established for each patient by selecting the optimal biological formulation for each patient. In the present invention, it is possible to clearly understand and predict that effects on a patient are different between an anti-IL-6 agent and an anti-TNF-$\alpha$ agent, which allows administration of an anti-IL-6 agent to a patient for whom an anti-IL-6 agent is effective and administration of an anti-TNF-$\alpha$ agent to a patient for whom an anti-TNF-a agent is effective. The working mechanisms are different for an anti-IL-6 agent and an anti-TNF-a agent. However, since the present invention can clearly determine the difference thereof prior to therapy, a high quality rheumatoid arthritis therapy that was impossible with prior art can be achieved.

In this manner, a rheumatoid arthritis patient for whom administration of a biological formulation is effective can be identified by utilizing the present invention. Thus, for patients, the present invention is beneficial in terms of medical cost containment, sense of security from the prediction of a therapeutic effect and the like. For physicians, the present invention enables the establishment of a suitable treatment plan based on an accurate prediction of effectiveness of a biological formulation.

Furthermore, the present invention does not require complex and time-consuming genetic analysis which lacks versatility. In addition, the present invention uses the concentration of a specific cytokine, chemokine, and/or soluble receptor in a serum as an indicator. Thus, the present invention can estimate in advance the effectiveness of a biological formulation targeting an inflammatory cytokine for each patient in a simple and cost-effective manner by using an existing method of measurement.

[Brief Description of Drawings]

[Figure 1] Figure **1** is a diagram showing results of comparing predicted DAS-28 values after 16 weeks of therapy calculated from regression equation (4) prior to therapy and actual DAS-28 values after 16 weeks of therapy subjecting naive patients who received anti-IL-6 agent (tocilizumab) therapy.

[Figure 2] Figure **2** is a diagram showing results of comparing predicted DAS-28 values after 16 weeks of therapy calculated from regression equation (5) prior to therapy and actual DAS-28 values after 16 weeks of therapy subjecting switch patients who received anti-IL-6 agent (tocilizumab) therapy.

[Figure 3] Figure **3** is a diagram showing results of comparing predicted DAS-28 values after 16 weeks of therapy calculated from regression equation (7) prior to therapy and actual DAS-28 values after 16 weeks of therapy subjecting naive patients who received anti-TNF-a agent (etanercept) therapy.

[Figure 4] Figure **4** is a diagram showing the relationship between DAS-28 values prior to therapy (PreDAS-28 score) and values obtained from subtracting a DAS-28 value after 16 weeks from a DAS-28 value prior to therapy (PreDAS-28score - 16W DAS-28 score) in naive patients who received anti-IL-6 agent (tocilizumab) therapy.

[Figure 5] Figure **5** is a diagram showing a trial profile of an anti-IL-6 agent (tocilizumab) therapy patient and an anti-TNF-$\alpha$ agent (etanercept) therapy patient.

[Figure 6-1] Figures **6-1** to **6-4** are diagrams showing clinical baseline individual group statistics for healthy individuals and rheumatoid arthritis patients with respect to serum concentrations of cytokines/chemokines/soluble receptors.

[Figure 6-2] Figures **6-1** to **6-4** are diagrams showing clinical baseline individual group statistics for healthy individuals and rheumatoid arthritis patients with respect to serum concentrations of cytokines/chemokines/soluble receptors.

[Figure 6-3] Figures **6-1** to **6-4** are diagrams showing clinical baseline individual group statistics for healthy individuals and rheumatoid arthritis patients with respect to serum concentrations of cytokines/chemokines/soluble receptors.

[Figure 6-4] Figures **6-1** to **6-4** are diagrams showing clinical baseline individual group statistics for healthy individuals and rheumatoid arthritis patients with respect to serum concentrations of cytokines/chemokines/soluble receptors.

[Figure 7] Figure 7 is a diagram showing the relationship between DAS-28 values prior to therapy and DAS-28 values after 16 weeks of therapy in anti-IL-6 agent (tocilizumab) therapy patients and anti-TNF-$\alpha$ agent (etanercept) therapy patients.

[Figure 8] Figure **8** is a diagram showing the relationship between actual values of DAS-28 after 16 weeks of etanercept therapy and predicted DAS-28 values after 16 weeks of therapy estimated by assuming a patient has received anti-IL-6 agent (tocilizumab) therapy in naive patients who received anti-TNF-$\alpha$ agent (etanercept) therapy.

[Figure 9] Figure **9** is a diagram showing results of analyzing the relationship between serum sgp130 concentrations and DAS-28 values prior to therapy for patients in remission and non-remission.

[Figure 10] Figure **10** is a correlation diagram comparing values of DAS28-CRP and values of DAS28-ESR with respect to the same sample.

[Figure 11] Figure **11** shows an ROC curve for sgp130 alone and an ROC graph calculated while also taking logIL-6, logIP-10 and logTNFRII into consideration in addition thereto. A is for a naive patient and B is for a switch patient. Thick lines indicate an ROC curve for sgp130, logIL-6, logIP-10 and logTNFRII and thin lines indicate an ROC curve for sgp130 alone (also indicated by an arrow in the drawings).

[Description of Embodiments]

The present invention is described below while disclosing the best mode of the invention. Throughout the entire specification, a singular expression should be understood as encompassing the concept thereof in a plural form unless specifically noted otherwise. Thus, terms using singular articles (e.g., "a", "an", "the" and the like in case of English) should be understood as encompassing the concept thereof in a plural form unless specifically noted otherwise. Further, the terms used herein should be understood as being used in the meaning that is commonly used in the art, unless specifically noted otherwise. Thus, unless defined otherwise, all terminologies and scientific technical terms that are used herein have the same meaning as the terms commonly understood by those skilled in the art to which the present invention belongs. In case of a discrepancy between the explanation of the present specification and the explanation in the art, the present specification (including the definitions) takes precedence.

1. Determining method

The present invention provides a method of determining effectiveness of therapy due to a biological formulation targeting an inflammatory cytokine on a rheumatoid arthritis patient (e.g., possibility of remission, level of improvement in a symptom after therapy, disease activity indicator or the like).

In one embodiment, the method of the present invention includes a method of measuring the concentration of a specific marker in a body sample of a rheumatoid arthritis patient to determine in advance remission for the patient due to a biological formulation (also referred to as a "specific biological formulation" herein when mentioned in comparison to an "specific marker" discussed below). In another embodiment, the method of the present invention includes a method of measuring the concentration of a specific marker in a body sample of a rheumatoid arthritis patient to determine in advance a level of improvement in a symptom after therapy for the patient due to a specific biological formulation. In other embodiments, the present invention includes a method of measuring the concentration of a specific marker in a body sample of a rheumatoid arthritis patient to determine in advance a disease activity indicator after therapy due to a specific biological formulation.

As used herein, a "body sample" refers to any sample collected from the body of a patient. Examples thereof include, but are not limited to, serum, blood, urine, saliva and the like. A serum is preferably used in the present invention. Thus, in a preferred embodiment of the present invention, the method of the present invention is carried out by using a serum collected from a rheumatoid arthritis patient prior to the administration of a biological formulation.

In the present invention, examples of a marker used for determining the effectiveness of therapy for a specific biological formulation (also referred to as a "specific marker" in the present invention) include one or two or more types of markers selected from the group consisting of sgp130, IP-10, sTNFRI, sTNFRII, GM-CSF, IL-1$\beta$, IL-2, IL-5, IL-6, IL-7, IL-8, IL-9, IL-10, IL-12, IL-13, IL-15, Eotaxin, VEGF, MCP-1, TNF-$\alpha$, IFN-$\gamma$, FGFbasic, PDGF-bb, sIL-6R, and MIP-1$\alpha$. If necessary, an indicator for a condition of a patient prior to therapy (e.g., DAS28 score prior to therapy or the like) may be used as a "specific marker". Thus, a "specific marker" encompasses indicators for a condition of a patient prior to therapy in the present invention.

In a preferred embodiment, a specific marker that can be used for an anti-IL-6 agent includes sgp130, logIL-6, logIL-8, logEotaxin, logIP-10, logVEGF, logsTNFR-I, and logsTNFR-II. These markers can be used to predict an indicator related to future therapy, such as a DAS28-CRP score in 16 weeks in a naive patient.

In another preferred embodiment, a specific marker that can be used for an anti-IL-6 agent includes sgp130, logGM-CSF, and logIP-10. These markers can be used to predict an indicator related to future therapy, such as a DAS28-CRP score in 16 weeks in a switch patient.

In a preferred embodiment, a specific maker that can be used for an anti-TNF-a agent includes logIL-9, logVEGF, and logTNF-$\alpha$. These markers are effective in the prediction of a DAS28-CRP score in 16 weeks in a naïve patient.

In the determining method of the present invention, determination is made by using the concentration of a specific marker of the present invention in a body sample. Thus, the method of the present invention encompasses measuring a concentration of a specific marker in a body sample (e.g., in a serum). Hereinafter, the determining method of the present invention is discussed in detail.

Biological formulation subjected to determination

The determining method of the present invention is a method of predicting and determining a therapeutic effect of a biological formulation targeting an inflammatory cytokine on a rheumatoid arthritis patient.

In the determining method of the present invention, a biological formulation targeting an inflammatory cytokine is not limited to a biological formulation used in rheumatoid arthritis therapy. A therapeutic effect can be predicted and determined in accordance with the type of biological formulation to be used. Examples of a biological formulation targeting an inflammatory cytokine include anti-IL-6 agents, anti-TNF-$\alpha$ agents and the like.

A preferred embodiment predicts and determines a therapeutic effect of a biological formulation comprising an anti-IL-6 agent and an anti-TNF-a agent. Hereinafter, anti-IL-6 agents and anti-TNF-a agents are discussed in further detail.

As used herein, an "anti-IL-6 agent" refers to a medicament capable of treating rheumatoid arthritis by suppressing an IL-6 signaling pathway. Specific examples of anti-IL-6 agent include humanized anti-IL-6 receptor antibodies, anti-IL-6 antibodies and the like. Specific examples of humanized anti-IL-6 receptor antibodies include tocilizumab, sarilumab and the like. Specific examples of anti-IL-6 antibodies include sirukumab, olokizumab, and the like.

As used herein, an "anti-TNF-a agent" referred to a medicament capable of treating rheumatoid arthritis by suppressing a TNF-$\alpha$ signaling pathway. Specific examples of anti-TNF-a agent include anti-TNF-a antibodies, human soluble TNF/LT$\alpha$ receptors consisting of an Fc region of human IgG1 and a subunit dimer of an extracellular domain of a human tumor necrosis factor receptor II, and the like. Specific examples of human soluble TNF/LT$\alpha$ receptors include etanercept. Specific examples of anti-TNF-a antibodies include adalimumab, infliximab, golimumab, certolizumab, and the like.

Examples of preferred biological formulations thereamong which are applied in the determining method of the present invention include, but are not limited to, humanized anti-IL-6 receptor antibodies and humanized soluble TNF/LT$\alpha$ receptors, and still preferably tocilizumab and etanercept.

Each of the specific cytokines, chemokines, and/or soluble receptors employed as a specific marker in the present invention is a molecule associated with rheumatoid arthritis. Rheumatoid arthritis is called a multifactorial disease. It is understood that rheumatoid arthritis can be properly treated by administering an effective therapeutic agent against a cause thereof if the specific cause is identified. Biological formulations utilize this approach. Since the cause of rheumatoid arthritis is generally a cytokine, therapy using a biological formulation is also called "anti-cytokine therapy".

For biological formulations, a target for modifying activity is generally specified. The specified target is modified (e.g., suppress the binding of a binding factor to a target) to suppress the symptom of rheumatoid arthritis. IL-6, TNF-$\alpha$ and the like are known as a therapeutic target for rheumatoid arthritis, and corresponding agents are categorized as an anti-IL-6 agent and anti-TNF-a agent, respectively. IL-6 activates IL-6 signaling in an IL-6 signaling pathway, ultimately resulting in a rheumatoid arthritis symptom. In addition, TNF-$\alpha$ activates TNF-$\alpha$ signaling in a TNF-$\alpha$ signaling pathway, ultimately resulting in a rheumatoid arthritis symptom. An anti-IL-6 agent exerts a therapeutic effect (e.g., effect of causing rheumatoid arthritis to subside) by suppressing IL-6 signaling. An anti-TNF-$\alpha$ agent exerts a therapeutic effect (e.g., effect of causing rheumatoid arthritis to subside) by suppressing TNF-$\alpha$ signaling. Other biological formulations with a different target have a similar relationship. In this manner, a biological formulation exerts a therapeutic effect by counteracting on signaling by a factor, which is a therapeutic target, from causing a symptom of rheumatoid arthritis.

Every anti-IL-6 agent, regardless of the type thereof (e.g., anti-IL-6 antibodies, anti-IL-6 receptor antibodies and the like), exerts a therapeutic effect by similar working mechanism in terms of suppressing binding of IL-6 to an IL-6 receptor in some manner. A reaction after IL-6 signaling is suppressed is understood to be the same regardless of the manner of suppression. Thus, reactions in a living body with regard to the therapeutic process for rheumatoid arthritis are considered the same, regardless of the type of anti-IL-6 agent. Thus, it is understood that the knowledge regarding reactions in a living body for a certain therapeutic agent belonging to anti-IL-6 agents can also be applied to other therapeutic agents belonging to the same category (e.g., if the certain therapeutic agent is a humanized anti-IL-6 receptor antibody, other types of humanized anti-IL-6 receptor antibodies or antibodies against IL-6, fragments and derivatives thereof, and the like).

For example, in IL-6 signaling, it is known that IL-6 binds to IL-6R and the complex thereof binds to gp130 such that a signal of IL-6 is transmitted within a cell via gp130 (Rose-John S (2012) Int. J. Biol. Sci 8:1237-1247). SIL-6R also binds to IL-6 such that a signal is transmitted via gp130. For this reason, blood sIL-6R is not an inhibiting molecule, but an enhancing factor. Meanwhile, sgp130, since it binds to an IL-6/IL-6R complex, is an inhibiting molecule of IL-6. The inventors have revealed that both sIL-6R and sgp130 are associated with IL-6 inhibition therapy in vivo from the results of the present invention. It is possible to derive from the results in the Examples that IL-6 inhibition therapy has low effectiveness when blood baseline value of sIL-6R is high and IL-6 inhibition therapy is highly effective when sgp130 is high.

It has been discovered in the present invention that sgp130 can be used as a marker for predicting anti-IL-6 agent therapy from the knowledge in tocilizumab. In view of the relationship between IL-6 signaling and sgp130 including the knowledge in the art discussed above, those skilled in the art understand that a similar reaction occurs in a living body with any anti-IL-6 agent. Thus, it is the understanding of those skilled in the art that the knowledge related to sgp130 obtained with tocilizumab herein can be applied similarly to other anti-IL-6 agents.

It has been discovered in the present invention that sgp130 can be used in the prediction of a disease activity indicator and remission for a patient due to an anti-IL-6 agent. In addition, it has been discovered that in addition to sgp130, the following markers can be utilized for various predictions related to an anti-IL-6 agent.

*Prediction of possibility of remission: IP-10, sTNFRII, IL-6, IL-7, MCP-1, and IL-1β
*Prediction of level of improvement in symptom: IL-1β, IL-7, TNF-α, and sIL-6R
*Prediction of disease activity indicator: IL-8, Eotaxin, sTNFRI, sTNFRII, IL-6, VEGF, GM-CSF, and IP-10

These markers are factors (cytokine, chemokine, and/or soluble receptor) related to the pathology of rheumatoid arthritis. Thus, these markers, which were found to be highly related in an anti-IL-6 agent tocilizumab, are considered to be common information with respect to prediction of each IL-6 inhibition therapy (tocilizumab, sirukumab, and the like). Accordingly, it is understood that each of the regression equations obtained for tocilizumab obtained in the Examples of the present invention can be used directly or with a minor adjustment for other anti-IL-6 agents. For various anti-IL-6 agents, detailed numerical values disclosed in the present invention with regard to regression equations may not be applicable. Even in such a case, those skilled in the art can create a regression equation for a specific anti-IL-6 agent by conducting further analysis, such as backward looking analysis, based on the description of the present specification and clinical data that was actually obtained as needed.

Considering the conventional knowledge, sgp130 being usable in the prediction of remission and disease activity indicator is recognized as a significant feature in the present invention. In addition thereto, when a prediction for whether remission is reached is examined in more detail, it was revealed in the present invention that precision in which AUC (Area Under Curve) is in excess of about 0.8, in some cases precision of about 0.9, is exhibited when determination is made by combining sgp130 with another maker described above (see Tables 12-13). In this manner, the present invention demonstrates that prediction and determination can be made very accurately at a precision that was impossible with prior art.

Explanations similar to those for anti-IL-6 agents can be provided for anti-TNF-a agents. Based on the knowledge obtained in the present invention, common information for anti-TNF-α agents can be discussed, which is provided below.

Every anti-TNF-α agent, regardless of the type thereof (e.g., anti-TNF-α or human soluble TNF/LTα receptors consisting of an Fc region of human IgG1 and a subunit dimer of an extracellular domain of a human tumor necrosis factor receptor II), exerts a therapeutic effect by similar working mechanism in terms of suppressing the binding of TNF-α to a TNF-α receptor in some manner. A reaction after TNF-α signaling is suppressed is understood to be the same, regardless of the manner of suppression. Thus, reactions in a living body with regard to the therapeutic process for rheumatoid arthritis are the same, regardless of the type of anti-TNF-α agent. Thus, it is understood that the knowledge regarding reactions in a living body for a certain therapeutic agent belonging to anti-TNF-α agents can also be applied to other therapeutic agents belonging to the same category (e.g., if the certain therapeutic agent is a human soluble TNF/LTα receptor, other types of human soluble TNF/LTα receptor or anti-TNF-α antibodies, fragments and derivatives thereof, and the like).

In addition, it has been discovered in the present invention that the following markers can be utilized for various predictions related to an anti-TNF-α agent.

*Prediction of possibility of remission: IL-9, TNF-α, VEGF, MIP-1a, PDGFbb
*Prediction of level of improvement in symptom: IL-2, IL-15, sIL-6R, sTNFRI, IL-6, IL-13
*Prediction of disease activity indicator: IL-9, TNF-α, VEGF, IL-6, IL-13

These markers are known factors (cytokine, chemokine, and/or soluble receptor) related to the pathology of rheumatoid arthritis. Thus, these markers, which were found to be highly related in TNF-α etanercept, are the common information with respect to prediction of each TNF-α inhibition therapy (etanercept, adalimumab, infliximab, golimumab, certolizumab and the like). Accordingly, each of the regression equations obtained for etanercept obtained in the Examples of the present invention can be used directly or with a minor adjustment for other anti-TNF-α agents. For various anti-TNF-α agents, detailed numerical values disclosed in the present invention regarding regression equations may not be applicable. Even in such a case, those skilled in the art can create a regression equation for a specific anti-TNF-α agent by conducting further analysis, such as backward looking analysis, based on the description of the present specification and clinical data that is actually obtained as needed.

It has been discovered in the present invention that in addition to VEGF or MIP-1a, PDGFbb and an indicator for a condition of a rheumatoid arthritis patient prior to therapy, a combination of IL-9 and TNF-α can be used in determining in advance remission of rheumatoid arthritis. In this regard, DAS-28 values prior to administration or the like can be used as an indicator for a condition of a patient prior to therapy, but the indicator is not limited thereto. Even for such a combination of IL-9 and TNF-$\alpha$, a relatively high value of AUC of 0.745 is exhibited. Prediction of remission due to an anti-TNF-$\alpha$ agent using two such specific markers, which are two markers that are different from an anti-IL-6 agent, was not possible with conventional therapeutic techniques.

It has been discovered in the present invention that a marker for predicting a therapeutic effect of an anti-IL-6 agent is completely different from a marker for predicting a therapeutic effect of an anti-TNF-$\alpha$ agent. This was unexpected from the current therapeutic framework, which views rheumatoid arthritis as a single disease and thus provides undifferentiated therapy. Meanwhile, rheumatoid arthritis is a multifactorial disease, and a biological formulation directly targets the cause thereof. It was unknown in the past whether these causes are independent from one another or are associated with one another. However, it has been revealed by the knowledge of the present invention that a marker for predicting a therapeutic effect of an anti-IL-6 agent is different from a marker for predicting a therapeutic effect of an anti-TNF-$\alpha$ agent. Thus, it was revealed that IL-6 signaling is highly likely to be independent from TNF-$\alpha$ signaling as a cause of rheumatoid arthritis. Such knowledge was unknown in the past. As far as the inventors are aware, the present invention is the first to report the presence of a suitable biological formulation for each patient. It has been demonstrated by the knowledge of the present invention that therapy by personalized medicine (method of selecting a therapeutic agent suitable for therapy for each patient) is possible for rheumatoid arthritis.

Patients often do not reach remission with TNF-$\alpha$ inhibition therapy such as etanercept alone. Thus, therapy is generally provided in conjunction with methotrexate administration. In addition, methotrexate is known to suppress IL-6 pathway without suppressing a TNF-$\alpha$ pathway (Nishina N et al., Clin Rheumatol. 2013 Nov; 32(11): 1661-6). In view of the above and the fact that the present invention has conducted a study targeting patients treated by etanercept or the like in conjunction with methotrexate, some of the markers associated with an anti-TNF-$\alpha$ agent discovered in the present invention may overlap with a marker for an anti-IL-6 agent. In such a case, it is preferable that prediction and determination are made by excluding markers that overlap with a marker for an anti-IL-6 agent. Such markers that overlap with a marker for an anti-IL-6 agent can be excluded by multivariable analysis or logistic analysis as needed.

Patients subjected to determination

The determining method of the present invention determines whether administration of a biological formulation is effective in a rheumatoid arthritis patient prior to administration of the biological formulation.

Further, target rheumatoid arthritis patients in the determining method of the present invention are not particularly limited, as long as it is prior to administration of the biological formulation. In addition, whether DMARDs such as methotrexate are administered, past dosing history of anti-cytokine therapy (administration of infliximab etanercept, adalimumab, tocilizumab or the like) are not relevant. When a more detailed determination result is required, a therapeutic effect due to a biological formulation can be predicted and determined by selecting a desired specific marker in accordance with the past dosing history of the biological formulation in the determining method of the present invention.

Specific markers

The determining method of the present invention uses one or two or more types of specific markers (herein, also referred to as a "determination marker") selected from the group consisting of sgp130 (soluble gp130), IP-10 (interferon-inducible protein 10), sTNFRI (soluble receptors for tumor necrosis factor type I), sTNFRII (soluble receptors for tumor necrosis factor type II), GM-CSF (granulocyte macrophage colony-stimulating factor), IL-6 (interleukin-6), IL-7 (interleukin-7), IL-8 (interleukin-8), IL-9 (interleukin-9), IL-10 (interleukin-10), IL-12 (interleukin-12), IL-13 (interleukin-13), IL-15 (interleukin-15), Eotaxin, VEGF (vascular endothelial growth factor), MCP-1 (monocyte chemotactic protein-1), TNF-$\alpha$ (tumor necrosis factor-a), IFN-$\gamma$ (interferon-$\gamma$), FGFbasic (basic fibroblast growth factor), PDGF-bb (platelet-derived growth factor bb), sIL-6R (soluble receptors for interleukin-6), and MIP-1$\alpha$ (macrophage inflammatory protein-1$\alpha$) in the serum of the rheumatoid arthritis patient.

One type of the aforementioned specific cytokine, chemokine, and soluble receptor may be used alone as a specific marker in the determining method of the present invention. However, it is preferable to use two or more types from thereamong in combination as a specific marker, from the viewpoint of predicting and determining a therapeutic effect due to a biological formulation at a higher precision. Furthermore, it is more preferable to use a set of specific markers with a high determination precision shown in the present specification.

The specific marker is appropriately selected and used, depending on the therapeutic effect to be predicted and determined, type of biological formulation to be administered, past dosing history of biological formulation or the like. Specific preferred examples of specific marker are shown below for each therapeutic effect to be predicted and determined (level of improvement in a symptom after therapy, possibility of remission, disease activity indicator).

<Cases where level of improvement in symptom after therapy (level of improvement in value of disease activity indicator; value of disease activity indicator prior to therapy - value of disease activity indicator after therapy) is predicted and determined for biological formulation>

For a naive patient administered with an anti-IL-6 agent (e.g., tocilizumab) (herein, also referred to as an "anti-IL-6 agent therapy naive patient"), it is preferable to use at least one type selected from the group consisting of IL-7, IL-8, IL-12, IL-13, IP-10, VEGF, IL-1β, TNF-α, and sIL-6R as a specific marker. It is more preferable to use IL-1β, IL-7, TNF-α, and sIL-6R in combination as a specific marker.

For a switch patient administered with an anti-IL-6 agent (e.g., tocilizumab) (hereinafter, also referred to as an "anti-IL-6 agent therapy switch patient"), it is preferable to use at least one type selected from the group consisting of IL-1β, IL-5, IL-6, IL-7, IL-10, IL-12, IL-13, IL-15, FGFbasic, GM-CSF, IFN-γ, TNF-α, and VEGF as a specific marker.

For a naive patient administered with an anti-TNF-α agent (e.g., etanercept) (hereinafter, also referred to as an "anti-TNF-α agent therapy naive patient"), it is preferable to use at least one type selected from the group consisting of IL-6, IP-10, IL-2, IL-13, IL-15, sIL-6R, and sTNFRI as a specific marker. It is more preferable to use a combination of IL-2, IL-15, sIL-6R, and sTNFRI as a specific marker.

For a switch patient administered with an anti-TNF-α agent (e.g., etanercept) (herein, also referred to as an "anti-TNF-α agent therapy switch patient"), it is preferable, as is with an "anti-TNF-α agent therapy naive patient", to use at least one type selected from the group consisting of IL-6, IP-10, IL-2, IL-13, IL-15, sIL-6R, and sTNFRI as a specific marker. It is more preferable to use a combination of IL-2, IL-15, sIL-6R, and sTNFRI as a specific marker. A rheumatoid arthritis patient cannot reach remission with an anti-TNF-α agent alone and it is practically required to use methotrexate in conjunction when using an anti-TNF-a agent in recent years. In the present invention, a marker for anti-TNF-α agent therapy naive patients is also information obtained from combined use with methotrexate. Methotrexate is known to have an effect on rheumatoid arthritis through the suppression of an IL-6 signaling pathway. Thus, even an "anti-TNF-α agent therapy naive patient" with respect to biological formulations is recognized as a patient who already has a certain level of modification to the IL-6 signaling pathway. Thus, markers for an "anti-TNF-α agent therapy naïve patient" obtained in the present invention are recognized to be practically in the same state as a marker for an "anti-TNF-α agent therapy switch patient". Considering the above situation, a marker for an "anti-TNF-α agent therapy naive patient" obtained in the present invention can be similarly applied in predicting an effect in an "anti-TNF-α agent therapy switch patient". Further, methotrexate is known to suppress an IL-6 pathway. Thus, a marker utilized in an "anti-TNF-a agent therapy switch patient" may overlap with a marker for an anti-IL-6 agent. In such a case, a marker overlapping with a marker for an anti-IL-6 agent can be excluded for analysis as needed.

<Cases where value of disease activity indicator after therapy itself is predicted and determined for biological formulation>

For an anti-IL-6 agent therapy naive patient, it is preferable to use at least one type selected from the group consisting of sgp130, IL-8, Eotaxin, IP-10, sTNFRI, sTNFRII, IL-6, and VEGF as a specific marker. It is more preferable to use a combination of sgp130, IL-8, Eotaxin, IP-10, sTNFRI, sTNFRII, and IL-6, or a combination of sgp130, IL-8, Eotaxin, IP-10, sTNFRI, sTNFRII, IL-6, and VEGF as a specific marker. Any anti-IL-6 agent may be targeted for prediction and determination. However, tocilizumab is preferred.

For an anti-IL-6 agent therapy switch patient, it is preferable to use at least one type selected from the group consisting of sgp130, IL-1β, IL-2, IL-5, IL-15, GM-CSF, IFN-γ, TNF-α, and IP-10 as a specific marker. It is more preferable to use a combination of sgp130, IP-10, and GM-CSF as a specific marker. Any anti-IL-6 agent may be targeted for prediction and determination for these specific markers. However, tocilizumab is preferred.

For an anti-TNF-α agent therapy naive patient, it is preferable to use at least one type selected from the group consisting of IL-9, IL-6, IL-13, TNF-α, and VEGF as a specific marker. It is more preferable to use a combination of IL-9, TNF-α, and VEGF, or a combination of IL-6 and IL-13 as a specific marker. Any anti-TNF-a agent may be targeted for prediction and determination for these specific markers. However, etanercept is preferred.

<Cases where possibility of remission (whether remission is reached) by therapy is predicted and determined for biological formulation>

For patients administered with an anti-IL-6 agent (including both anti-IL-6 agent therapy naive patients and anti-IL-6 agent therapy switch patients), it is preferable to use at least one type selected from the group consisting of sgp130, IP-10, sTNFRII, IL-6, IL-7, MCP-1 and IL-1β as a specific marker. It is more preferable to use at least sgp130, and even more preferable to use a combination of (i) sgp130, (ii) IP-10, (iii) sTNFRII, and (iv) IL-6, IL-7, MCP-1 or IL-1β. More specifically, for anti-IL-6 agent therapy naive patients, a combination of (i) sgp130, (ii) IP-10, (iii) sTNFRII, and (iv) IL-6, IL-7, MCP-1 or IL-1β is especially preferable as a specific marker. Further, for anti-IL-6 agent therapy switch patients, a combination of (i) sgp130, (ii) IP-10, (iii) sTNFRII, and (iv) IL-6 or IL-1β is especially preferable as a specific marker. For such specific markers, any anti-IL-6 agent may be targeted for prediction and determination. However, tocilizumab is preferred.

For an anti-TNF-a agent therapy naive patient, it is preferable to use at least one type selected from the group consisting of IL-9, TNF-$\alpha$, VEGF, PDGF-bb, and MIP-1$\alpha$ as a specific marker. It is especially preferable to use a combination of IL-9 and TNF-$\alpha$, a combination of VEGF and PDGF-bb, or a combination of MIP-1$\alpha$ and PDGF-bb as a specific marker. Any anti-TNF-$\alpha$ agent may be targeted for prediction and determination for such specific markers. However, etanercept is preferred. These specific markers can be used similarly for anti-TNF-$\alpha$ agent therapy switch patients and for anti-TNF-$\alpha$ agent therapy naive patients.

It is known that serum concentration of each of the cytokines, chemokines, and soluble receptors used as a specific marker can be measured by a measurement system utilizing an antigen-antibody reaction such as ELISA. Such measuring kits are commercially available. Thus, the cytokines, chemokines, and soluble receptors can be measured with a known measuring kit by a known method in the determining method of the present invention. A reagent used in such a measurement system utilizing an antigen-antibody reaction can be provided individually or in a set as a diagnostic agent for determining each biological formulation.

Prediction and determination of therapeutic effect due to biological formulation

A therapeutic effect due to a specific biological formulation such as an anti-IL-6 agent or an anti-TNF-$\alpha$ agent can be predicted and determined based on a measured value of the specific marker. For example, the prediction and determination include a method in which the specific marker is measured in advance for patients in full remission and patients who are not in remission from therapy with a specific biological formulation; a regression equation of a measured value of the specific marker (explanatory variable) and a therapeutic effect of biological formulation (objective variable) are found by regression analysis; and a measured value of a specific marker of a rheumatoid arthritis patient targeted for determination is applied to said regression equation. When finding a regression equation, it is preferable to use a log value of serum concentration (pg/ml) for the specific markers other than sgp130. For sgp130, a value of serum concentration (pg/ml) is preferably used. Such log values or concentration values are selected by the inventors by considering whether concentration values should be directly used or log values should be employed as a result of comparing and studying the normal distribution of healthy individuals, resulting in high correlation as in the result of the study. Further, it is preferable that a regression equation is derived by multiple regression analysis. The objective variable in the above-described regression equation may be appropriately determined based on the therapeutic effect to be predicted and determined.

For example, when predicting and determining the level of improvement in a symptom after therapy for a specific biological formulation, the objective variable may be set to "a value obtained by subtracting a value of a disease activity indicator after a predetermined period of therapy from a value of a disease activity indicator prior to therapy" for analysis by multiple linear regression analysis. For example, when predicting and determining a value of a disease activity indicator after therapy for a specific biological formulation, the objective variable may be set to "a value of disease activity indicator after a predetermined period of therapy" for analysis by multiple linear regression analysis. In this regard, specific examples of a value of a disease activity indicator include a DAS (Disease activity score)-28 value, CDAI (Clinical Disease Activity Index) value, SDAI (Simple Disease Activity Index) value and the like. A DAS-28 value, CDAI value and SDAI value are correlated with one another and reflect a symptom of rheumatoid arthritis. Thus, any of such disease activity indicator values may be used in the determining method of the present invention. Further, a disease activity indicator used in the determining method of the present invention is not limited to those exemplified above. Indicators that may be newly advocated in the future can be used. For DAS28, the present specification demonstrates that DAS28-CRP and DAS28-ESR can be similarly used. The level of improvement may be 0 (no change) or have a negative value. In such a case, improvement is not recognized. When the value is unchanged, the value indicates no improvement, and a negative value indicates exacerbation. If the value is negative, those skilled in the art would naturally decide not to use the biological formulation.

Further, when predicting or determining the possibility of remission due to therapy with a biological formulation (result of whether there is remission or no remission), multiple logistic regression analysis may be used for analysis.

For regression analysis utilizing a measured value of the specific marker as an explanatory variable, a value of a disease activity indicator prior to therapy (DAS-28 value, CDAI value, SDAI value or the like) or a result of evaluation by a Boolean method may be utilized as an explanatory variable.

Hereinafter, therapeutic effects to be predicted and determined are separated into a level of improvement in a symptom after therapy, DAS-28 value after therapy, and possibility of remission to disclose specific methods for the determining method of the present invention. However, the determining method of the present invention should not be interpreted to be limited to the following specific methods.

<Prediction and determination of level of improvement in symptom after therapy>

A level of improvement in a symptom after therapy due to a biological formulation can be predicted and determined by multiple linear regression analysis while setting an objective variable as "a value obtained by subtracting a value of a disease activity indicator after a predetermined period of therapy from a value of a disease activity indicator prior to therapy" and an explanatory variable as "a measured value of the specific marker".

In Examples described below, the following equations (1) and (2) have been discovered as regression equations for predicting and determining a level of improvement in a symptom after 16 weeks of therapy due to a specific biological formulation such as an anti-IL-6 agent or an anti-TNF-α agent (level of improvement in DAS-28 value; DAS-28 value prior to therapy - DAS-28 value after 16 weeks of therapy), separated by the past dosing history of a rheumatoid arthritis patient and type of biological formulation. A level of improvement in a symptom after 16 weeks of therapy can be predicted and determined by finding an objective variable from applying values to one of the following regression equations (1) and (2) depending on the past dosing history of a rheumatoid arthritis patient subjected to determination and type of biological formulation. A level of improvement in a symptom due to a specific biological formulation is predicted and determined to be large for the patient for larger values of the objective variable calculated by the following regression equation.

[Cases where level of improvement in symptom after 16 weeks of therapy (level of improvement in DAS-28 value; DAS-28 value prior to therapy - DAS28-value after 16 weeks of therapy) is predicted and determined for tocilizumab therapy naive patient]

Specific markers: IL-1β, IL-7, TNF-α, and sIL-6R

Regression equation (1):

```
Objective function (DAS-28 value prior to therapy - DAS28-
value after 16 weeks of therapy) = 5.505 + (-3.618 × A) +
(3.255 × B) + (1.475 × C) + (-1.841 × D)
```

A: log value of serum IL-1β concentration (pg/ml)
B: log value of serum IL-7 concentration (pg/ml)
C: log value of serum TNF-α concentration (pg/ml)
D: log value of serum sIL-6R concentration (pg/ml)

When another anti-IL-6 agent is used, a regression equation for another selected anti-IL-6 agent can be created by using the same method as the method performed in the Examples or the like while referring to parameters such as coefficients, each variable of the above-described regression equation and the above-described types of specific marker.

[Cases where level of improvement in symptom after 16 weeks of therapy (level of improvement in DAS-28 value; DAS-28 value prior to therapy - DAS28-value after 16 weeks of therapy) is predicted and determined for etanercept therapy naive patient]

Specific markers: IL-2, IL-15, sIL-6R, and sTNFRI

Regression equation (2):

```
Objective function (DAS-28 value prior to therapy - DAS-28
value after 16 weeks of therapy) = 7.325 + (-1.567 × E) +
(1.632 × F) + (-2.540 × D) + (1.973 × G)
```

E: log value of serum IL-2 concentration (pg/ml)
F: log value of serum IL-15 concentration (pg/ml)
D: log value of serum sIL-6R concentration (pg/ml)
G: log value of serum sTNFRII concentration (pg/ml)

When another anti-TNF-α agent is used, a regression equation for another selected anti-TNF-α agent can be created by using the same method as the method performed in the Examples or the like while referring to parameters such as coefficients, each variable of the above-described regression equation and the above-described types of specific marker.

The regression equations (1) and (2) demonstrate an example of a regression equation used to predict and determine a level of improvement in DAS-28 value after 16 weeks of therapy due to a specific biological formulation such as an anti-IL-6 agent or an anti-TNF-α agent. However, a level of improvement in a CDAI value or an SDAI value after 16 weeks of therapy due to a biological formulation (level of improvement in CDAI value or SDAI value; CDAI value or SDAI value prior to therapy - CDAI value or SDAI value after 16 weeks of therapy) can naturally be predicted and determined by multiple linear regression analysis by the same method using a CDAI value or SDAI value. Further, since a therapeutic effect stabilizes and appears after 16 weeks of therapy by a biological formulation, regression equations for predicting and determining a level of improvement in a symptom after 16 weeks of therapy are shown in the above-described regression equations (1) and (2). Naturally, a level of improvement in a symptom before or after 16 weeks of therapy due to each of the specific biological formulations can be predicted and determined by multiple linear regression analysis using the same method.

In Examples described below, the following equations (3)-(7) have been discovered as regression equations for predicting and determining a DAS-28 value of a symptom after 16 weeks of therapy due to a specific biological formulation such as an anti-IL-6 agent or an anti-TNF-α agent, separated by the past dosing history of a rheumatism patient and type of biological formulation. A DAS-28 value after 16 weeks of therapy can be predicted and determined by finding an objective variable from applying values to one of the following regression equations (3)-(7), depending on the past dosing history of a rheumatoid arthritis patient subjected to determination and type of biological formulation. When the objective variable calculated by the following regression equation is 2.3 or less, the patient is predicted and determined to reach remission due to a specific biological formulation.

[Cases where DAS-28 value after 16 weeks of therapy is predicted and determined for tocilizumab therapy naive patient]

Specific markers: sgp130, IL-8, Eotaxin, IP-10, sTNFRI, sTNFRII, IL-6, and VEGF

Regression equation (3):

```
Objective function (DAS-28 value after 16 weeks of therapy)
= 6.909 + (-5.341 × H) + (3.940 × I)(-1.039 × J) + (-1.002
× K) + (-2.580 × L) + (1.407 × G) + (0.744 × M) + (-0.850 ×
N)
```

H: serum sgp130 concentration (pg/ml)
I: log value of serum IL-8 concentration (pg/ml)
J: log value of serum Eotaxin concentration (pg/ml)
K: log value of serum IP-10 concentration (pg/ml)
L: log value of serum sTNFRII concentration (pg/ml)
G: log value of serum sTNFRII concentration (pg/ml)
M: log value of serum IL-6 concentration (pg/ml)
N: log value of serum VEGF concentration (pg/ml)

When another anti-IL-6 agent is used, a regression equation for another selected anti-IL-6 agent can be created by using the same method as the method performed in the Examples or the like while referring to parameters such as coefficients, each variable of the above-described regression equation and the above-described types of specific marker.

[Cases where DAS-28 value after 16 weeks of therapy is predicted and determined for tocilizumab therapy naive patient]

Specific markers: sgp130, IL-8, Eotaxin, IP-10, sTNFRI, sTNFRII, and IL-6

Regression equation (4):

```
Objective function (DAS-28 value after 16 weeks of therapy)
= 4.731 + (-5.433 × H) + (2.551 × I)(-0.937 × J) + (-1.116
× K) + (-2.010 × L) + (1.630 × G) + (0.577 × M)
```

H: serum sgp130 concentration (μg/ml)
I: log value of serum IL-8 concentration (pg/ml)
J: log value of serum Eotaxin concentration (pg/ml)
K: log value of serum IP-10 concentration (pg/ml)
L: log value of serum sTNFRII concentration (pg/ml)
G: log value of serum sTNFRII concentration (pg/ml)
M: log value of serum IL-6 concentration (pg/ml)

When another anti-IL-6 agent is used, a regression equation for another selected anti-IL-6 agent can be created by using the same method as the method performed in the Examples or the like while referring to parameters such as coefficients, each variable of the above-described regression equation and the above-described types of specific marker.

[Cases where DAS-28 value after 16 weeks of therapy is predicted and determined for tocilizumab therapy switch patient]

Specific markers: sgp130, IP-10, and GM-CSF

Regression equation (5):

```
Objective function (DAS-28 value after 16 weeks of therapy)
= 2.837 + (-6.037 × H) + (0.714 × K) + (-0.622 × O)
```

H: serum sgp130 concentration (μg/ml)
K: log value of serum IP-10 concentration (pg/ml)
O: log value of serum GM-CSF concentration (pg/ml)

When another anti-IL-6 agent is used, a regression equation for another selected anti-IL-6 agent can be created by using the same method as the method performed in the Examples or the like while referring to parameters such as coefficients, each variable of the above-described regression equation and the above-described types of specific marker.

[Cases where DAS-28 value after 16 weeks of therapy is predicted and determined for etanercept therapy naïve patient]

Specific markers: IL-6 and IL-13, DAS-28 value prior to etanercept administration is also used as an explanatory variable

Regression equation (6):

```
Objective function (DAS-28 value after 16 weeks of therapy)
= 0.081 + (0.522 × a) + (-0.969 × M) + (1.409 × P)
```

a: DAS-28 value prior to etanercept administration
M: log value of serum IL-6 concentration (pg/ml)
P: log value of serum IL-13 concentration (pg/ml)

When another anti-TNF-$\alpha$ agent is used, a regression equation for another selected anti-TNF-$\alpha$ agent can be created by using the same method as the method performed in the Examples or the like while referring to parameters such as coefficients, each variable of the above-described regression equation and the above-described types of specific marker.

[Cases where DAS-28 value after 16 weeks of therapy is predicted and determined for etanercept therapy naive patient]

Specific markers: IL-9, TNF-$\alpha$ and VEGF

Regression equation (7):

$$\text{Objective function (DAS-28 value after 16 weeks of therapy)}$$
$$= 0.703 + (0.646 \times S) + (-0.551 \times C) + (0.858 \times N)$$

S: log value of serum IL-9 concentration (pg/ml)
C: log value of serum TNF-$\alpha$ concentration (pg/ml)
N: log value of serum VEGF concentration (pg/ml)

When another anti-TNF-$\alpha$ agent is used, a regression equation for another selected anti-TNF-$\alpha$ agent can be created by using the same method as the method performed in the Examples or the like while referring to parameters such as coefficients, each variable of the above-described regression equation and the above-described types of specific marker.

Since regression equation (7) does not use a DAS-28 value prior to etanercept administration as an explanatory variable, a DAS-28 value after 16 weeks of therapy can be predicted while eliminating a subjective opinion of a physician. Thus, regression equation (7) is considered preferable over regression equation (6).

The regression equations (3)-(7) show examples of a regression equation used to predict and determine a DAS-28 value after 16 weeks of therapy due to a specific biological formulation such as an anti-IL-6 agent or an anti-TNF-$\alpha$ agent. However, a CDAI value or SDAI value itself after 16 weeks of therapy due to each specific biological formulation can naturally be predicted and determined by multiple linear regression analysis by the same method using a CDAI value or SDAI value. Further, as discussed above, since a therapeutic effect stabilizes and appears after 16 weeks of therapy due to each specific biological formulation, regression equations for predicting and determining a value of a disease activity indicator after 16 weeks of therapy are shown in the above-described regression equations (3)-(7). However, a value of disease activity indicator prior to or after 16 weeks of therapy due to each of the specific biological formulations can naturally be predicted and determined by multiple linear regression analysis using the same method.

In Examples described below, the following equations (8)-(16) have been discovered as regression equations for predicting and determining the possibility of remission (either remission or not in remission) after 16 weeks of therapy due to a specific biological formulation such as an anti-IL-6 agent or an anti-TNF-$\alpha$ agent, separated by the past dosing history of a rheumatoid arthritis patient and type of biological formulation. It is possible to predict and determine whether remission is reached after 16 weeks of therapy by finding the probability (p) of remission after 16 weeks of therapy from applying values to one of the following regression equations (8)-(16) depending on the past dosing history of a rheumatoid arthritis patient subjected to determination and type of biological formulation. The probability of remission estimated from the following regression equations (8)-(16) refers to the probability of a DAS-28 value being 2.3 or less. A p value computed from regression equations (8)-(16) closer to 1 indicates a higher possibility of remission after 16 weeks of therapy. For example, the p value of 0.5 or higher can predict and determine remission and less than 0.5 can predict and determine no remission for convenience's sake. In this regard, a DAS-28 value of 2.3 is used as the boundary between remission and non-remission to enhance the precision of prediction and determination of remission because a CRP value tends to decrease and DAS-28 value may decreases regardless of inflammation by inhibiting IL-6. The value is set at a lower value of DAS-28 value (2.6), which is generally considered the boundary between remission and non-remission.

[Cases where possibility of remission is predicted and determined for tocilizumab therapy naive patient]

Specific markers: sgp130, IP-10, sTNFRII, and IL-6

Regression equation (8):

```
p/(1-p) = exp{(-5.095) + (-36.648 × H) + (-4.004 × K) +
(5.632 × G) + (1.658 × M)}
```

p: probability of remission after 16 weeks of therapy
H: serum sgp130 concentration ($\mu$g/ml)
K: log value of serum IP-10 concentration (pg/ml)
G: log value of serum sTNFRII concentration (pg/ml)
M: log value of serum IL-6 concentration (pg/ml)

When another anti-IL-6 agent is used, a regression equation for another selected anti-IL-6 agent can be created by using the same method as the method performed in the Examples or the like while referring to parameters such as coefficients, each variable of the above-described regression equation and the above-described types of specific marker.

[Cases where possibility of remission is predicted and determined for tocilizumab therapy naive patient]

Specific markers: sgp130, IP-10, sTNFRII, and IL-7

Regression equation (9):

```
p/(1-p) = exp{(-3.467) + (-42.849 × H) + (-4.430 × K) +
(5.736 × G) + (2.705 × B)}
```

p: probability of remission after 16 weeks of therapy
H: serum sgp130 concentration ($\mu$g/ml)
K: log value of serum IP-10 concentration (pg/ml)
G: log value of serum sTNFRII concentration (pg/ml)
M: log value of serum IL-7 concentration (pg/ml)

When another anti-IL-6 agent is used, a regression equation for another selected anti-IL-6 agent can be created by using the same method as the method performed in the Examples or the like while referring to parameters such as coefficients, each variable of the above-described regression equation and the above-described types of specific marker.

[Cases where possibility of remission is predicted and determined for tocilizumab therapy naive patient]

Specific markers: sgp130, IP-10, sTNFRII, and MCP-1

Regression equation (10):

```
p/(1-p) = exp{(-2.834) + (-38.721 × H) + (-4.664 × K) +
(5.369 × G) + (2.502 × B)}
```

p: probability of remission after 16 weeks of therapy
H: serum sgp130 concentration ($\mu$g/ml)

K: log value of serum IP-10 concentration (pg/ml)
G: log value of serum sTNFRII concentration (pg/ml)
P: log value of serum MCP-1 concentration (pg/ml)

When another anti-IL-6 agent is used, a regression equation for another selected anti-IL-6 agent can be created by using the same method as the method performed in the Examples or the like while referring to parameters such as coefficients, each variable of the above-described regression equation and the above-described types of specific marker.

[Cases where possibility of remission is predicted and determined for tocilizumab therapy naive patient]

Specific markers: sgp130, IP-10, sTNFRII, and IL-1$\beta$

Regression equation (11):

$$p/(1-p) = \exp\{(-1.269) + (-39.538 \times H) + (-3.807 \times K) + (5.086 \times G) + (1.647 \times A)\}$$

p: probability of remission after 16 weeks of therapy
H: serum sgp130 concentration ($\mu$g/ml)
K: log value of serum IP-10 concentration (pg/ml)
G: log value of serum sTNFRII concentration (pg/ml)
A: log value of serum IL-1$\beta$ concentration (pg/ml)

When another anti-IL-6 agent is used, a regression equation for another selected anti-IL-6 agent can be created by using the same method as the method performed in the Examples or the like while referring to parameters such as coefficients, each variable of the above-described regression equation and the above-described types of specific marker.

[Cases where possibility of remission is predicted and determined for tocilizumab therapy switch patient]

Specific markers: sgp130, IP-10, sTNFRII, and IL-6

Regression equation (12):

$$p/(1-p) = \exp\{(-10.935) + (-29.051 \times H) + (4.466 \times K) + (2.067 \times G) + (-2.757 \times M)\}$$

p: probability of remission after 16 weeks of therapy
H: serum sgp130 concentration ($\mu$g/ml)
K: log value of serum IP-10 concentration (pg/ml)
G: log value of serum sTNFRII concentration (pg/ml)
M: log value of serum IL-6 concentration (pg/ml)

When another anti-IL-6 agent is used, a regression equation for another selected anti-IL-6 agent can be created by using the same method as the method performed in the Examples or the like while referring to parameters such as coefficients, each variable of the above-described regression equation and the above-described types of specific marker.

[Cases where possibility of remission is predicted and determined for tocilizumab therapy switch patient]

Specific markers: sgp130, IP-10, sTNFRII, and IL-1$\beta$

Regression equation (13):

$$p/(1-p) = exp\{(-9.671) + (-27.150 \times H) + (3.205 \times K) + (1.914 \times G) + (-2.540 \times A)\}$$

p: probability of remission after 16 weeks of therapy
H: serum sgp130 concentration ($\mu$g/ml)
K: log value of serum IP-10 concentration (pg/ml)
G: log value of serum sTNFRII concentration (pg/ml)
A: log value of serum IL-1$\beta$ concentration (pg/ml)

When another anti-IL-6 agent is used, a regression equation for another selected anti-IL-6 agent can be created by using the same method as the method performed in the Examples or the like while referring to parameters such as coefficients, each variable of the above-described regression equation and the above-described types of specific marker.

[Cases where possibility of remission is predicted and determined for etanercept therapy naive patient]

Specific markers: VEGF and PDGF-bb, DAS-28 value prior to etanercept administration is also used as an explanatory variable.

Regression equation (14):

$$p/(1-p) = exp\{(-19.058) + (1.390 \times a) + (-2.763 \times E) + (4.962 \times Q)$$

p: probability of remission after 16 weeks of therapy
a: DAS-28 value prior to etanercept administration
E: log value of serum VEGF concentration (pg/ml)
Q: log value of serum PDGF-bb concentration (pg/ml)

When another anti-TNF-$\alpha$ agent is used, a regression equation for another selected anti-TNF-$\alpha$ agent can be created by using the same method as the method performed in the Examples or the like while referring to parameters such as coefficients, each variable of the above-described regression equation and the above-described types of specific marker.

[Cases where possibility of remission is predicted and determined for etanercept therapy naive patient]

Specific markers: MIP-1$\alpha$ and PDGF-bb, DAS-28 value prior to etanercept administration is also used as an explanatory variable.

Regression equation (15):

$$p/(1-p) = exp\{(-18.491) + (1.107 \times a) + (-1.808 \times R) + (3.930 \times Q)\}$$

p: probability of remission after 16 weeks of therapy
a: DAS-28 value prior to etanercept administration
R: log value of serum MIP-1$\alpha$ concentration (pg/ml)
Q: log value of serum PDGF-bb concentration (pg/ml)

When another anti-TNF-α agent is used, a regression equation for another selected anti-TNF-α agent can be created by using the same method as the method performed in the Examples or the like while referring to parameters such as coefficients, each variable of the above-described regression equation and the above-described types of specific marker.

[Cases where possibility of remission is predicted and determined for etanercept therapy naive patient]

Specific markers: IL-9 and TNF-α

Regression equation (16):

$$p/(1-p) = \exp\{(-1.004) + (1.711 \times S) + (-1.031 \times C)\}$$

p: probability of remission after 16 weeks of therapy
S: log value of serum IL-9 concentration (pg/ml)
C: log value of serum TNF-α concentration (pg/ml)

When another anti-TNF-α agent is used, a regression equation for another selected anti-TNF-α agent can be created by using the same method as the method performed in the Examples or the like while referring to parameters such as coefficients, each variable of the above-described regression equation and the above-described types of specific marker.

Since the regression equation (16) does not use a DAS-28 value prior to etanercept administration as an explanatory variable, the possibility of remission can be predicted while eliminating a subjective opinion of a physician. Thus, regression equation (16) is considered preferable over regression equations (14) and (15).

The regression equations (6)-(16) show examples of a regression equation for predicting and determining the possibility of remission after 16 weeks of therapy, with a DAS-28 value after 16 weeks of therapy of 2.3 or lower considered remission and the value over 2.3 as non-remission. However, the possibility of remission after 16 weeks of therapy due to a specific biological formulation such as an anti-IL-6 agent or an anti-TNF-α agent can naturally be predicted and determined by multiple logistic regression analysis with the same method using a CDAI value or SDAI value. Further, as discussed above, since a therapeutic effect stabilizes and appears after 16 weeks of therapy by a biological formulation, regression equations for predicting and determining the possibility of remission after 16 weeks of therapy are shown in the above-described regression equations (6)-(16). However, the possibility of remission prior to or after 16 weeks of therapy due to a biological formulation can be predicted and determined by multiple logistic regression analysis using the same method.

Selection of biological formulation to be administered

The determining method of the present invention can predict the therapeutic effectiveness of a biological formulation prior to the administration thereof. Thus, the method can be utilized in selecting the optimal biological formulation that should be administered prior to starting therapy.

Thus, in one embodiment, the method of selecting a biological formulation of the present invention includes a method of selecting a biological formulation that is effective to a patient by determining in advance remission due to a specific biological formulation in accordance with the method of the present invention to select a specific biological formulation with a high probability of remission. In another embodiment, the method of selecting a biological formulation of the present invention includes a method of selecting a biological formulation that is effective for a patient by determining in advance a level of improvement in a symptom after therapy due to a specific biological formulation in accordance with the method of the present invention and selecting a biological formulation with a high level of improvement in a symptom after the therapy. In another embodiment, the method of selecting a biological formulation of the present invention includes a method of selecting a biological formulation that is effective for a patient by determining in advance a disease activity indicator after therapy for the patient due to a specific biological formulation in accordance with the present invention and selecting a biological formulation with a disease activity indicator which is lower than a predetermined baseline.

For example, for a level of improvement in a symptom after therapy of a naive patient, cases in which an anti-IL-6 agent (e.g., tocilizumab) is administered and cases in which an anti-TNF-α agent (e.g., etanercept) is administered are each predicted by the aforementioned method and a biological formulation with a higher level of improvement is selected, so that an optimal biological formulation can be administered to the patient. Specifically, a level of improvement in a symptom after therapy due to anti-IL-6 agent (e.g., tocilizumab) therapy, which is predicted by using regression equation (1) or an equation adjusted based thereupon is compared to a level of improvement in a symptom after therapy with etanercept anti-TNF-$\alpha$ agent (e.g., etanercept) therapy, which is predicted by using regression equation (2) or an equation adjusted based thereupon, so that the biological formulation with a higher level of improvement can be selected as the optimal biological formulation.

Alternatively, when the focus is on individual biological formulations, it is possible to determine a baseline for whether each biological formulation such as an anti-IL-6 agent and an anti-TNF-$\alpha$ agent is employed in advance or based on experience for a level of improvement in a symptom after therapy to determine that a specific biological formulation is to be administered when the actually calculated level of improvement in a symptom after therapy is at or above a predetermined baseline. Specifically, if a level of improvement in a symptom after therapy calculated by using regression equation (1), (2) or the like is expressed by DAS-28 value prior to therapy - DAS-28 value after 16 weeks of therapy, a specific biological formulation can be determined to be administered when the value is 0 (i.e., no exacerbation) or when the value is 0.1 or greater, 0.2 or greater, 0.3 or greater, 0.4 or greater, 0.5 or greater, 1.0 or greater, 1.5 or greater, 2.0 or greater, 2.5 or greater, 3.0 or greater, or the like. Such a specific value of a baseline can be set to any value, such as the specific numerical values described herein, numerical values therebetween (e.g., 0.6 or greater or the like) or a value exceeding such numerical values (e.g., 3.5 or greater). Meanwhile, when the level of improvement is negative, determination can be made that such a medicament should not be administered.

For example, for a DAS-28 value after 16 weeks of therapy of a naive patient, cases in which an anti-IL-6 agent (e.g., tocilizumab) is administered and cases in which an anti-TNF-$\alpha$ agent (e.g., etanercept) is administered are each predicted by the aforementioned method and a biological formulation with a lower DAS-28 value after 16 weeks of therapy is selected so that an optimal biological formulation can be administered to the patient. Specifically, a DAS-28 value after 16 weeks of therapy due to anti-IL-6 agent (e.g., tocilizumab) therapy, which is predicted by using one of regression equations (3)-(5) and an equation adjusted based thereupon is compared to a DAS-28 value after 16 weeks of therapy due to anti-TNF-$\alpha$ agent (e.g., etanercept) therapy, which is predicted by using regression equation (6), (7) or an equation adjusted based thereupon, so that the biological formulation with a smaller DAS-28 value can be selected as the optimal biological formulation.

In another embodiment, when the focus is on individual biological formulations, it is possible to determine a baseline for whether each biological formulation such as an anti-IL-6 agent and an anti-TNF-$\alpha$ agent is employed for a disease activity indicator in advance or based on experience to determine a specific biological formulation is to be administered when the actually calculated disease activity indicator is at or below a predetermined baseline. Specifically, if a disease activity indicator predicted by using regression equation (3)-(7) or the like is expressed in a DAS-28 value after 16 weeks of therapy, a specific biological formulation can be determined to be administered when the value is 2.3 or lower (remission), 2.6 or lower (non-remission, but low level of activity), 4.1 or lower (non-remission, but medium level of activity), or the like. For such a specific value of a baseline, the specific numerical values described herein or slightly adjusted values based on experiment or the like can be used. As a baseline of remission, a value higher than 2.3 or lower than 2.3 may be used as needed.

For example, for the possibility of remission of a naive patient, cases in which an anti-IL-6 agent (e.g., tocilizumab) is administered and cases in which an anti-TNF-$\alpha$ agent (e.g., etanercept) is administered are each predicted by the aforementioned method to select a biological formulation with a higher possibility of remission, so that the optimal biological formulation can be administered to the patient. Specifically, the possibility of remission due to anti-IL-6 agent (e.g., tocilizumab) therapy, which is predicted by using one of regression equations (8)-(11) or an equation adjusted based thereupon, is compared to the possibility of remission by anti-TNF-$\alpha$ agent (e.g., etanercept) therapy, which is predicted by using one of regression equations (14)-(16) or an equation adjusted based thereupon, so that the biological formulation with a higher possibility of remission can be selected as the optimal biological formulation.

Alternatively, when the focus is on individual biological formulations, it is possible to determine a baseline for whether each biological formulation such as an anti-IL-6 agent and an anti-TNF-$\alpha$ agent is employed for the probability of remission in advance or based on experience to determine a specific biological formulation is to be administered when the actually calculated probability of remission is at or above a predetermined baseline. Specifically, a specific biological formulation can be determined to be administered when a probability of remission predicted by using regression equation (8)-(16) or the like is 30% or higher, 40% or higher, 50% or higher, 60% or higher, 70% or higher, 80% or higher, 85% or higher, 90% or higher, or 95% or higher. For such a specific value of a baseline, the specific numerical values described herein or values slightly adjusted based on experience of the like can be used. In addition, any value other than the specific numerical values described herein may be used.

2. Diagnostic agent

The present invention further provides a diagnostic agent for carrying out the above-described detection method.

Specifically, the diagnostic agent of the present invention is a diagnostic agent for determining the effectiveness of therapy due to a biological formulation targeting an inflammatory cytokine for a rheumatoid arthritis patient, characterized by comprising a reagent capable of detecting at least one type of specific marker selected from the group consisting of sgp130, IP-10, sTNFRI, sTNFRII, GM-CSF, IL-1β, IL-2, IL-5, IL-6, IL-7, IL-8, IL-9, IL-10, IL-12, IL-13, IL-15, Eotaxin, VEGF, MCP-1, TNF-α, IFN-γ, FGFbasic, PDGF-bb, sIL-6R, and MIP-1α.

In one embodiment, the diagnostic agent of the present invention is a diagnostic agent used in a method of measuring a concentration of a specific marker in a body sample of a rheumatoid arthritis patient to determine in advance remission of the patient due to a specific biological formulation, the diagnostic agent comprising a reagent for detecting the specific marker. In another embodiment, the diagnostic agent of the present invention is a diagnostic agent used in a method of selecting a biological formulation that is effective for a rheumatoid arthritis patient by measuring a concentration of a specific marker in a body sample of the patient, calculating in advance the probability of remission for the patient due to a plurality of specific biological formulations, and selecting a specific biological formulation with a high probability of remission, where the diagnostic agent comprises a reagent for detecting the specific marker. After remission is determined in advance, a predetermined level of probability of remission is set in advance so that the level can be used for determination to administer the biological formulation. When used for a plurality of specific biological formulations, a probability of remission for each of the plurality of specific biological formulations can be calculated and compared to select which specific biological formulation should be administered. Methods specifically disclosed in other parts of the specification, including the section of "1. Determination method" can be used for a specific method for each specific marker used in these embodiments.

In further embodiments, the diagnostic agent of the present invention is a diagnostic agent used in a method of measuring a concentration of a specific marker in a body sample of a rheumatoid arthritis patient to determine in advance a level of improvement in a symptom after therapy for the patient due to a specific biological formulation, where the diagnostic agent comprises a reagent for detecting the specific marker. In another embodiment, the diagnostic agent of the present invention is a diagnostic agent used in a method of selecting a biological formulation that is effective on a rheumatoid arthritis patient by measuring a concentration of a specific marker in a body sample of the patient, determining in advance a level of improvement in a symptom after therapy for the patient due to a plurality of specific biological formulations, and selecting a specific biological formulation with a high level of improvement, where the diagnostic agent comprises a reagent for detecting the specific marker. After a level of improvement in a symptom after therapy is determined in advance, a predetermined level of improvement is set in advance so that the level can be used for determination to administer the biological formulation. When used for a plurality of specific biological formulations, a level of improvement for each of the plurality of specific biological formulations can be calculated and compared to select which specific biological formulation should be administered. Methods specifically disclosed in other parts of the specification, including the section of "1. Determining method", can be used for a specific method for each specific marker used in these embodiments.

In further embodiments, the diagnostic agent of the present invention is a diagnostic agent used in a method of measuring a concentration of a specific marker in a body sample of a rheumatoid arthritis patient to determine in advance a disease activity indicator after therapy for the patient due to a specific biological formulation, where the diagnostic agent comprises a reagent for detecting the specific marker. In yet another embodiment, the diagnostic agent of the present invention is a diagnostic agent used in a method of selecting a biological formulation that is effective on a rheumatoid arthritis patient by measuring a concentration of a specific marker in a body sample of the patient, determining in advance a disease activity indicator after therapy for the patient due to a plurality of specific biological formulation, and selecting a specific biological formulation with a low disease activity indicator, where the diagnostic agent comprises a reagent for detecting the specific marker. After a disease activity indicator is determined in advance, a predetermined level of disease activity indicator is set in advance so that the level can be used for determination to administer the biological formulation. When used for a plurality of specific biological formulations, a disease activity indicator for each of the plurality of specific biological formulations can be calculated and compared to select which specific biological formulation should be administered. Methods specifically disclosed in other parts of the specification, including the section of "1. Determining method", can be used for a specific method for each specific marker used in these embodiments.

In a specific embodiment, a reagent required for a specific marker can be selected and used based on information described herein in accordance with the type of biological formulation or items for determining effectiveness (remission, level of improvement in a symptom after therapy, or disease activity indicator itself). When there are a plurality of such reagents, the reagents may be provided separately, collectively as a set, or as a kit with other required reagents (e.g., color producing agent).

The specific marker can be measured by a measurement system utilizing an antigen-antibody reaction such as ELISA. Specific examples of reagents capable of detecting the specific marker include antibodies that can specifically bind to the specific marker and fragments thereof. Further, antibodies that can specifically bind to the specific marker may be bound on a suitable support to be provided as an antibody array.

Furthermore, the diagnostic agent of the present invention may comprise a reagent (secondary antibody, color producing substance or the like) required for detecting the specific marker by an antigen-antibody reaction.

3. Therapeutic agent

The present invention further provides a therapeutic agent (also called personalized medicine or companion therapeutic agent), which is selected or determined to be suitable by the application of the detection method, diagnostic method, and selection method. More specifically, the therapeutic agent of the present invention is a therapeutic agent for the treatment of a rheumatoid arthritis patient, comprising a specific biological formulation, characterized in that a concentration of a specific marker in a body sample of the patient (e.g., serum) is measured to determine the effectiveness of the specific biological formulation and the specific biological formulation is selected or determined to be suitable based on the determined matter is administered. Alternatively, the present invention provides a set of therapeutic agents for treating a rheumatoid arthritis patient comprising a plurality of specific biological formulations. Such a set of therapeutic agents is characterized in that a concentration of a specific marker in a body sample of the patient is measured to calculate in advance a probability of remission for the patient due to the specific biological formulation, and a specific biological formulation with a high probability of remission is administered to the patient. Such a therapeutic agent comprises at least one biological formulation selected from the group consisting of an anti-IL-6 agent and an anti-TNF-$\alpha$ agent. A package insert or the like may be included, which explains that a concentration of a specific marker in a body sample of a patient (e.g., serum) is measured to determine the effectiveness due to the specific biological formulation and the specific biological formulation is administered when selected or determined to be suitable based on the determined matter. A package insert may be provided in a paper medium. However, a package insert may be provided in an electronic medium, via the internet or the like. Examples of anti-IL-6 agent that can be used as the therapeutic agent or the set of therapeutic agents of the present invention include, but are not limited to, tocilizumab, sarilumab, olokizumab, sirukumab and the like. Examples of anti-TNF-a agent that can be used as the therapeutic agent or the set of therapeutic agents of the present invention include, but are not limited to, etanercept, adalimumab, infliximab, golimumab, certolizumab and the like.

When the present invention is provided as a set of therapeutic agents, the set of therapeutic agents is characterized in that a concentration of a specific marker in a body sample of a patient is measured to calculate in advance a probability of remission for the patient due to the specific biological formulation, and a specific biological formulation with a high probability of remission is administered to the patient. When therapeutic agents are provided as a set, each biological formulation may be provided together or separately. Thus, when therapeutic agents are provided as a set, each therapeutic agent (e.g., anti-IL-6 agent, anti-TNF-$\alpha$ agent or the like) is provided individually, while a package insert is provided for the therapeutic agent. The package insert comprises a description explaining that, for example, a biological formulation is determined as to whether it should be administered in comparison to other biological formulations based on the determinement on effectiveness (e.g., possibility of remission, level of improvement in a symptom after therapy, or disease activity indicator itself) based on a method of the present invention. A package insert may be provided in a paper medium. However, a package insert may be provided in an electronic medium, via the internet or the like.

In one embodiment, the method of treating a rheumatoid arthritis patient of the present invention comprises (A) measuring a concentration of a specific marker in a body sample of the rheumatoid arthritis patient to determine in advance remission for the patient due to a specific biological formulation such as an anti-IL-6 agent or anti-TNF-$\alpha$ agent and (B) when it is determined that remission would occur for the patient due to the specific biological formulation by step (A), administering to the patient the specific biological formulation. Determination for remission is made when the probability of remission from a regression equation for predicting remission is at or above a certain baseline. Such a determination is made by using the methods specifically discussed in other portions of the specification, including the sections of "1. Determining method" and "2. Diagnostic agent".

Alternatively, the method of treating a rheumatoid arthritis of the present invention comprises (A) measuring a concentration of a specific marker in a body sample of a rheumatoid arthritis patient to calculate in advance a probability of remission for the patient by a plurality of specific biological formulations comprising an anti-IL-agent, anti-TNF-$\alpha$ agent or the like, (B) administering to the patient a specific biological formulation with a high probability of remission obtained from step (A). In this regard, a specific biological formulation is selected by calculating the probability of remission in a regression equation for predicting remission for a plurality of specific biological formulations and selecting a biological formulation corresponding to that with a high calculated probability of remission. For example, a specific marker for predicting remission due to an anti-I-6 agent and a specific marker for predicting remission due to an anti-TNF-$\alpha$ agent are selected, and a concentration of the specific markers in serum or the like of a target patient is measured to calculate the probability of remission for the anti-IL-6 agent and the probability of remission for the anti-TNF-$\alpha$ agent from a regression equation using the concentration of the markers, log values or the like. In this regard, in addition to such values, an indicator for a state prior to therapy of the patient such as a DAS-28 value prior to administration can be used in conjunction therewith. In addition, it is possible to compare the probability of remission for an anti-IL-6 agent with the probability of remission for an anti-TNF-$\alpha$ agent to determine that a biological formulation providing a high probability should be administered. In this regard, it is understood that any type of biological formulation may be used as an anti-IL-6 agent and an anti-TNF-$\alpha$ agent, as long as it is a biological formulation in the same category. This is because, as discussed in another section of the present specification, it is understood that the specific marker of the present invention can be applied as long as the category of biological formulation (e.g., anti-IL-6 agent, anti-TNF-$\alpha$ agent or the like) is the same. For specific selection and determination methods, methods specifically discussed in other portions of the present specification, including the above-described sections of "1. Determining method" and "2. Diagnostic agent", can be used.

In other embodiments, the method of treating a rheumatoid arthritis patient of the present invention comprises (A) measuring a concentration of a specific marker in a body sample of the rheumatoid arthritis to determine in advance a level of improvement in a symptom after therapy for the patient due to a specific biological formulation such as an anti-IL-6 agent or an anti-TNF-$\alpha$ agent and (B) when the level of improvement determined by step (A) is at or above a predetermined baseline, administering to the patient the specific biological formulation. A certain biological formulation is determined to be administered when a level of improvement in a symptom after therapy explained herein is calculated and the resulting value is at or above a predetermined value. Such determination can be made by using the method specifically discussed in other parts of the present specification, including the sections of "1. Determining method" and "2. Diagnostic agent".

In yet another embodiment, the method of treating a rheumatoid arthritis patient of the present invention comprises (A) measuring a concentration of a specific marker in a body sample of the rheumatoid arthritis patient to determine in advance a level of improvement in a symptom after therapy for the patient due to a plurality of specific biological formulations including an anti-IL-6 agent, an anti-TNF-$\alpha$ agent, or the like and (B) administering to the patient a specific biological formulation with a high level of improvement obtained from step (A). In this regard, a specific biological formulation is selected by calculating a level of improvement in a symptom after therapy in a regression equation for predicting a level of improvement in a symptom after therapy for a plurality of specific biological formulations and selecting a biological formulation corresponding to a biological formulation with a high level of calculated improvement in a symptom after therapy. For example, a specific marker for predicting a level of improvement in a symptom after therapy for an anti-IL-6 agent and a specific marker for predicting a level of improvement in a symptom after therapy for an anti-TNF-$\alpha$ agent are selected, and a concentration of the specific markers in a serum of a target patient or the like is measured with respect to the specific marker to calculate a level of improvement in a symptom after therapy for the anti-IL-6 agent and a level of improvement in a symptom after therapy for the anti-TNF-a agent from a regression equation by using the concentration, log value or the like. In this regard, in addition to these values, an indicator for a state prior to therapy of a patient such as a DAS-28 value can be used in conjunction therewith. In addition, it is possible to compare a level of improvement in a symptom after therapy for an anti-IL-6 agent and a level of improvement in a symptom after therapy for an anti-TNF-$\alpha$ agent to determine that a biological formulation providing a high level of improvement in a symptom after therapy should be administered. In this regard, it is understood that any type of biological formulation may be used as an anti-IL-6 agent or an anti-TNF-$\alpha$ agent, as long as it is a biological formulation in the same category. This is because, as discussed in another section of the present specification, it is understood that the specific marker of the present invention can be applied as long as the category of biological formulation (e.g., anti-IL-6 agent, anti-TNF-$\alpha$ agent or the like) is the same. For specific selection and determination methods, methods specifically discussed in other parts of the present specification, including the above-described sections of "1. Determining method" and "2. Diagnostic agent", can be used.

In yet another embodiment, the method of treating a rheumatoid arthritis patient of the present invention comprises (A) measuring a concentration of a specific marker in a body sample of the rheumatoid arthritis patient to determine in advance a disease activity indicator after therapy for the rheumatoid arthritis patient due to a specific biological formulation, such as an anti-IL-6 agent or an anti-TNF-$\alpha$ agent and (B) when the disease activity indicator from step (A) is at or below a predetermined baseline, administering to the patient the specific biological formulation. It is determined that a certain biological formulation should be administered when a value obtained by calculating a disease activity indicator explained herein is at or below a predetermined baseline. Such determination can be made by using methods specifically discussed in other parts of the present specification, including the above-described sections of "1. Determining method" and "2. Diagnostic agent".

In still another embodiment, the method of treating a rheumatoid arthritis patient of the present invention comprises (A) measuring a concentration of a specific marker in a body sample of the rheumatoid arthritis patient to determine in advance a disease activity indicator after therapy for the rheumatoid arthritis patient due to a plurality of specific biological formulations including an anti-IL-6 agent, an anti-TNF-$\alpha$ agent or the like and (B) administering to the patient a specific biological formulation with a low disease activity indicator obtained in step (A). In this regard, a specific biological formulation is selected by calculating a level of improvement in a symptom after therapy in a regression equation for predicting a disease activity indicator after therapy for a plurality of specific biological formulations and selecting a biological formulation corresponding to a lower calculated disease activity indicator after therapy. For example, a specific marker for predicting a disease activity indicator after therapy for an anti-IL-6 agent and a specific marker for predicting a disease activity indicator after therapy for an anti-TNF-α agent are selected, and concentrations of the specific markers in a serum of a target patient or the like are measured to calculate a disease activity indicator after therapy for the anti-IL-6 agent and a disease activity indicator after therapy for the anti-TNF-α agent from a regression equation by using the concentrations, log values or the like. In this regard, in addition to such values, an indicator for a state prior to therapy of a patient such as a DAS-28 value prior to administration can also be used in conjunction therewith. In addition, a disease activity indicator after therapy for an anti-IL-6 agent can be compared to a disease activity indicator after therapy for an anti-TNF-α agent to determine that a biological formulation providing a low disease activity indicator after therapy should be administered. In this regard, it is understood that any type of biological formulation may be used as an anti-IL-6 agent or an anti-TNF-α agent, as long as it is a biological formulation in the same category. This is because, as discussed in another section of the present specification, it is understood that the specific marker of the present invention can be applied as long as the category of biological formulation (e.g., anti-IL-6 agent, anti-TNF-α agent or the like) is the same. For specific selection and determination methods, methods specifically discussed in other parts of the present specification, including the above-described sections of "1. Determining method" and "2. Diagnostic agent", can be used.

Various embodiments have been used to disclose the present invention. It is understood that all patents, published patent applications and publications cited herein to explain the present invention are incorporated by reference as if set forth fully herein.

Examples

Hereinafter, the present invention is disclosed in detail while using Examples to facilitate the understanding thereof. However, the Examples are not provided to limit the present invention, but only for illustrative purposes. Thus, it is not intended that specifically described embodiments and examples be construed as limitations on the scope of the invention except as set forth in the appended claims.

1. Patient and Experimental Method

(Patient)

Hereinafter, a rheumatism patient who has not received anti-cytokine therapy (administration of infliximab, etanercept, adalimumab, tocilizumab or the like) in the past is referred to as a naive patient, and a rheumatism patient who has received anti-cytokine therapy in the past is referred to as a switch patient.

155 rheumatoid arthritis patients, to whom methotrexate therapy was ineffective, were registered at the Higashihiroshima Memorial Hospital from March 2008 to June 2013. Among the 155 patients, 98 patients received therapy with tocilizumab, which is an anti-IL-6 agent, and the remaining 57 patients received therapy with etanercept, which is an anti-TNF-α agent. Among the 98 patients who received therapy with tocilizumab, 58 patients were naive patients who had not previously received anti-cytokine therapy and 40 patients were switch patients who had previously received anti-cytokine therapy 1-3 times. Among 57 patients who received etanercept therapy, 49 patients were naive patients who had not previously received anti-cytokine therapy, and the remaining 8 patients were switch patients who had previously received anti-cytokine therapy. Informed consent was obtained prior to receiving a blood sample supply from all patients. Further, the tests were conducted with permission prior to the study from the ethics committee of the Higashihiroshima Memorial Hospital.

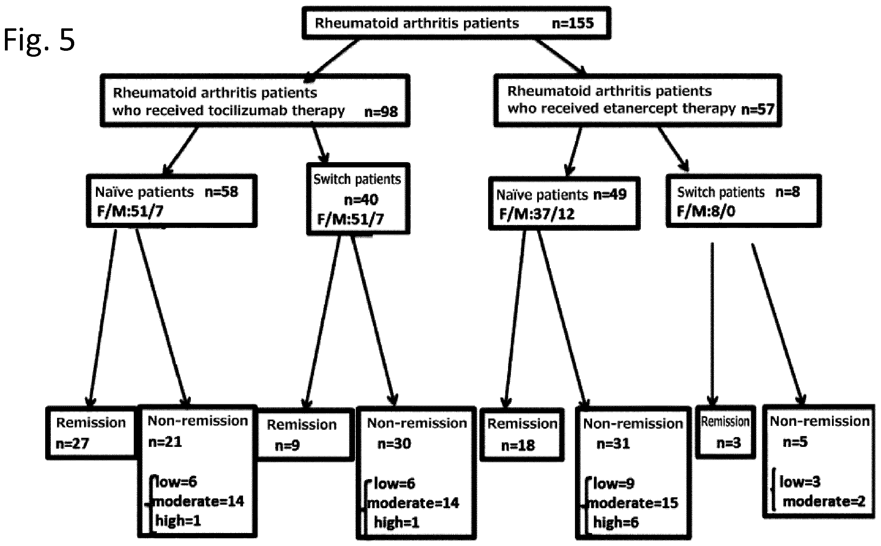

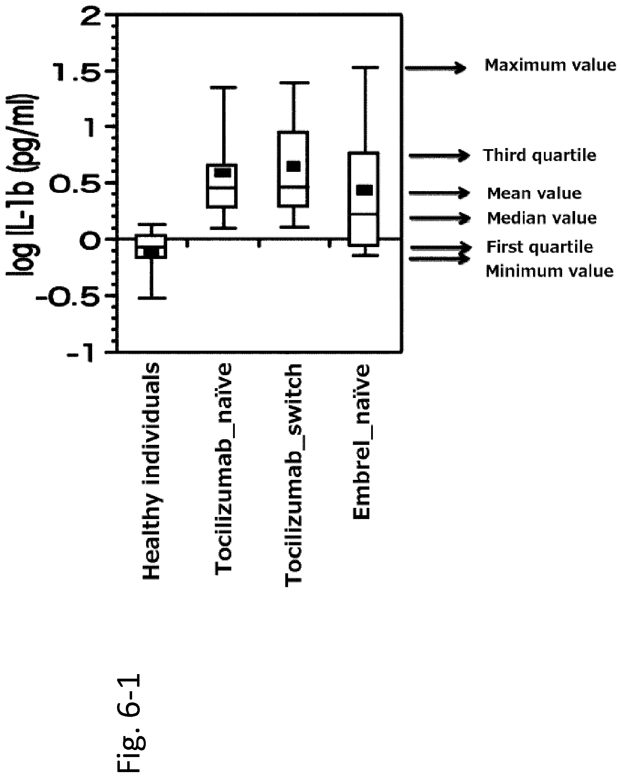

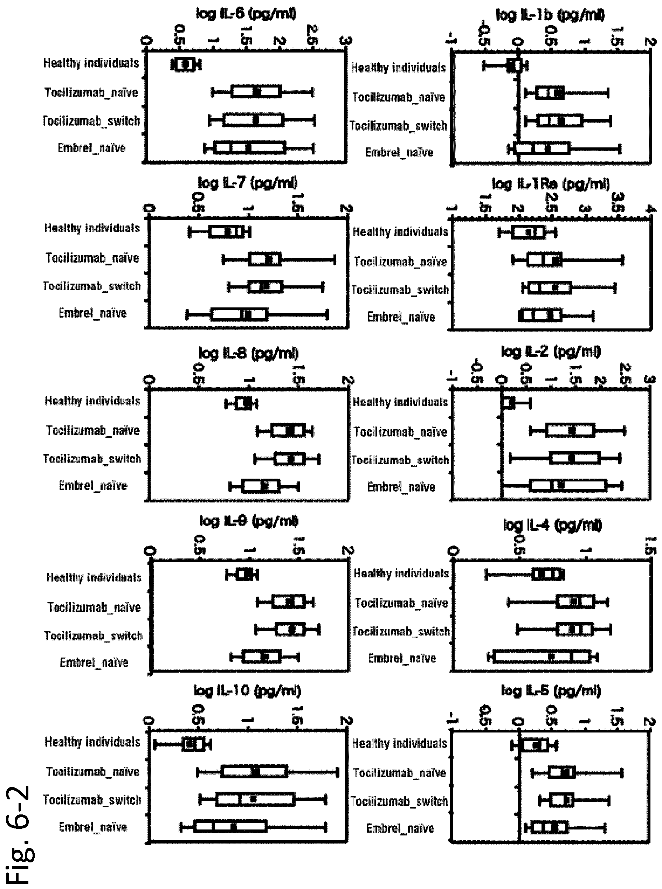

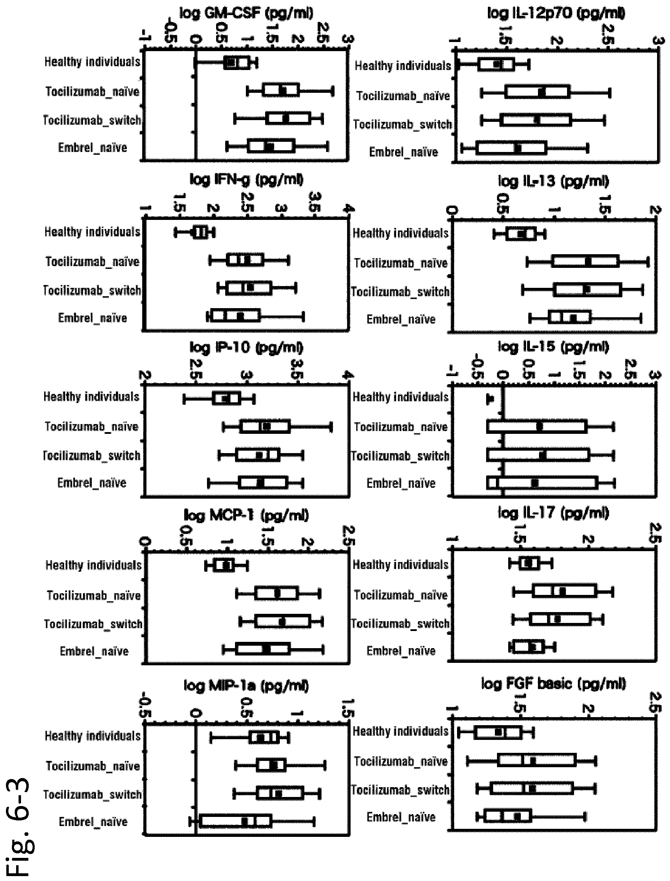

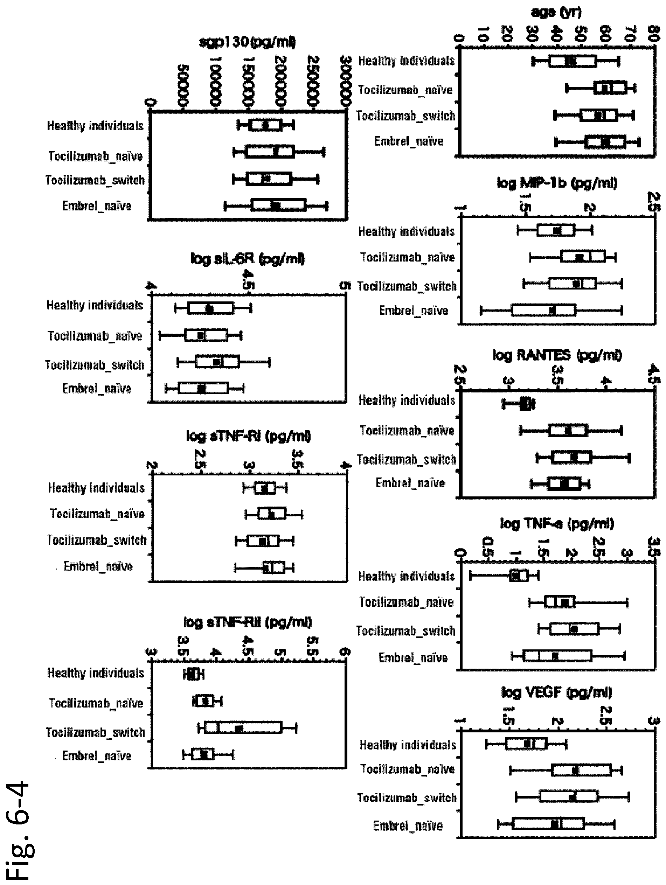

Table 1 shows the clinical baseline individual group statistics for group of individuals and clinical diagnosis. Further, Figure **5** shows a trial profile of patients receiving therapy with tocilizumab, which is an anti-IL-6 agent, and patients receiving therapy with etanercept, which is an anti-TNF-α agent. Figures **6-1** to **6-4** show serum concentration of cytokine/chemokine/soluble receptor prior to therapy. The total number is not consistent because 9 naive patients (8 patients who suffered from side effects or other diseases and 1 patient for whom data could not be obtained for the entire 16 weeks) among patients treated with tocilizumab were eliminated. Further, the total number is not consistent because 1 switch patient suffering from a side effect (patient for whom data could not be obtained for the entire 16 weeks) among patients treated with tocilizumab was eliminated. There was hardly any difference in DAS-28 value, CRP, swollen joint count, tender joint count, Stage, and Class among the groups (Table 1). Further, the duration of disease was shorter for patients treated with etanercept in comparison to patients treated with tocilizumab (Table 1).

In order to create a baseline concentration of cytokines, serum was collected from healthy individuals (56 individual; 20 males and 36 females) without a history of suffering from hepatitis C or cancer. The healthy individuals underwent medical examination by the Louis Pasteur Center for Medical Research or the Higashihiroshima Memorial Hospital and informed consent was received in writing from the healthy individuals. The baseline concentration was used to find a distribution pattern of cytokines/chemokines/soluble receptors.

(Experimental Method)

Prior to therapy, concentrations of cytokines, chemokines, and soluble receptors in the serum of rheumatoid arthritis patients were measured.

Figure **5** shows clinical results for naive patients and switch patients administered with 8 mg/kg tocilizumab or 50 mg/kg etanercept once every 4 weeks. After 16 weeks of therapy (after 4 administrations), a therapeutic effect was determined based on DAS-28-CRP values and whether the patient is in remission or non-remission. Results for non-remission were further classified into low, medium and high based on DAS-28-CRP values of the patients. DAS-28-ESR values are extensively used to determine the symptom of rheumatoid arthritis patients. However, it is reported that DAS-28-CRP values are almost interchangeable with DAS-28-ESR values and the same results are derived therefrom (Ann Rheum Dis.2007, March 407-409 Comparison of Disease Activity Score (DAS)28-erythrocyte Sedimentation rate and DAS-C-reactive protein threshold votes. Inoue E, Yamanaka H, et al.)

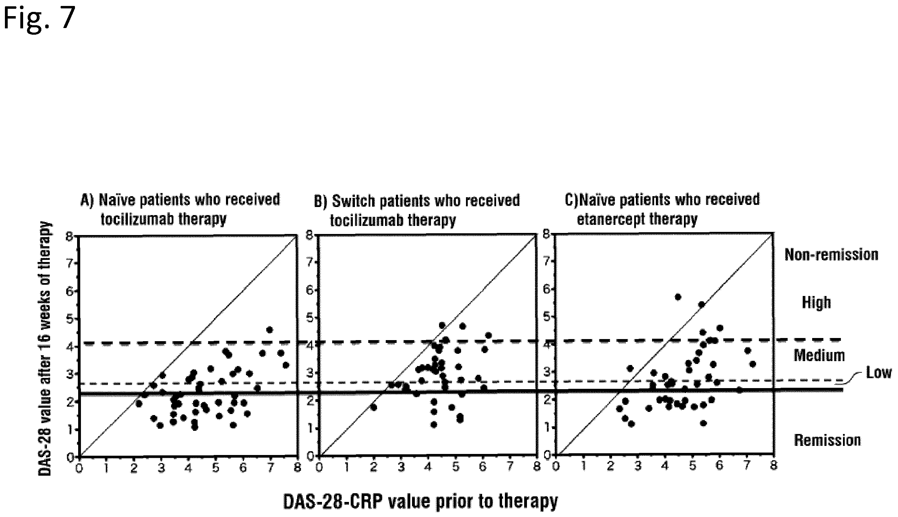

In the present tests, DAS-28-CRP values were used to determine the symptoms of rheumatoid arthritis patients. Remission was classified as DAS-28-CRP value < 2.3 and non-remission was classified as DAS-28-CRP value $\geq$ 2.3. Furthermore, DAS-28-CRP classification system developed by Inoue et al was used to classify non-remission patients as low (DAS-28-CRP value = 2.3-2.6), medium (DAS-28-CRP value = 2.7-4.1) and high (DAS-28-CRP value > 4.1) depending of the severity of the symptoms. To obtain consistent determination of symptoms, the same physician at the Higashihiroshima Memorial Hospital determined the final symptoms of all patients. Further, Figure **7** shows detailed clinical results of each patient shown in Figure 5, i.e., results of determining DAS-28-CRP values prior to therapy and after 16 weeks of therapy for naive patients who received anti-IL-6 (tocilizumab) therapy, switch patients who received anti-IL-6 (tocilizumab) therapy, and naive patients who received anti-TNF-$\alpha$ (etanercept) therapy. Hereinafter, DAS-28-CRP values may be denoted simply as DAS-28 values.

(Analysis of cytokine/chemokine/soluble receptor)

For all measurements of cytokines, a multiplex cytokine array system (Bio-Plex 200, Bio-Rad Laboratories) was used in accordance with the product protocol thereof. 1600 g of serum for all patients and healthy individuals were collected by 10 minutes of centrifugation. All serum samples were stored at -80°C. Bio-Plex Human Cytokine 27-Plex Panel is configured such that 27 types of cytokines (IL-1$\beta$, IL-1RA, IL-2, IL-4, IL-5, IL-6, IL-7, IL-8, IL-9, IL-10, IL-12 (p70), IL-13, IL-15, IL-17, basic FGF, eotaxin, G-CSF, GM-CSF, IFN-$\gamma$, IP-10, MCP-1, MTP-1$\alpha$, MIP-1$\beta$, PDGF-bb, RANTES, TNF-$\alpha$, and VEGF) can be analyzed. In addition thereto, sIL-6R, sgp130, sTNF-RI and sTNF-RII were also analyzed (Milliplex®MAP, Human Soluble Cytokine Receptor Panel: Millipore Co. MA). In the present test, concentrations of cytokines, chemokines, and soluble receptors of 56 healthy individuals were simultaneously measured to find the distribution patterns thereof. Bio-Plex Manager software version 5.0 was used to conduct data collection and analysis.

(Statistical Analysis)

The distribution of cytokine/chemokine values in healthy individuals was analyzed. The log values of the values of the concentration (pg/ml) of cytokines, chemokines, and soluble receptors, other than sgp130, were used for the analysis. The values of concentration ($\mu$g/ml) were directly used for sgp130.

First, simple linear regression analysis and multiple linear regression analysis were performed to investigate the association between cytokine/chemokine/soluble receptor concentration or clinical test values and values obtained by subtracting DAS-28 values after 16 weeks from DAS-28 values of patients at 0 weeks. Next, DAS-28 values after 16 weeks were estimated from a value computed from regression introduced with the clinical test values. Furthermore, simple logistic regression analysis and multiple logistic regression analysis were preformed to analyze the relationship between serum cytokine concentration and remission or non-remission. The resulting parameter p value of <0.05 indicates the presence of a significant difference. All statistical analysis was conducted by using the JMP 9.0 software.

2. Results

(Clinical evaluation)

Tables 1 and 2 show clinical baseline individual group statistics, clinical diagnosis and cytokine/chemokine/soluble receptor characteristics. Figures **6-1** to **6-4** shows clinical baseline individual group statistics for healthy individuals and rheumatoid arthritis patients with respect to serum concentrations of cytokines/chemokines/soluble receptors prior to therapy. In Table 1, Stage and Class are results of determination with respect to functional classification criteria for rheumatoid arthritis based on Steinbrocker (1949) classification (I-IV; Steinbrocker O et al: Therapeutic criteria in rheumatoid arthritis. JAMA 140:659,1949) and Hochberg (1992) classification (I-IV; Hochberg MC et al, The American College

**EP 3 309 553 A1**

of Rheumatology 1991 revised criteria for the Classification of global functional status in rheumatoid arthritis. Arthritis and Rheumatism, 35:498-502, 1992), respectively. The results indicate that there is no clinically significant difference among the three rheumatism patient groups and the serum concentrations of cytokines/chemokines/soluble receptors in most rheumatism patients are significantly higher in comparison to healthy individuals.

[Table 1]

## Basic information data for patients

*Table of basic information data for naive patients who received tocilizumab therapy, switch patients who received tocilizumab therapy, and naive patients who received etanercept therapy (clinical parameters: Age (years), Duration of disease (years), WBC, Fe, Ferritin, RBC, Hb, Ht, Plt, CRP, DAS28-CRP, RF (IU/mℓ), VAS, Swollen joint count, Tender joint count, Stage, Class).*

WBC  White blood cell count  ($\times 10^3/\mu\ell$)

Fe  Serum iron  ($\mu$g/d$\ell$)

Ferritin  Ferritin (ng/d$\ell$) CLIA method

RBC  Red blood cell count  ($\times 10^4/\mu\ell$)

Hb  Hemoglobin value  (g/d$\ell$)

Ht  Hematocrit value  (%)

Plt  Platelet count  ($\times 10^3/\mu\ell$)

CRP  C-reactive protein  (mg/d$\ell$)

DAS28-CRP  Disease activity score obtained by changing a variable for the erythrocyte sedimentation rate to a variable for CRP

RF  Rheumatoid factor concentration  (IU/m$\ell$)

VAS  Level of pain with 100 mm as the maximum pain experienced up to date  (mm)

Stage  Functional classification criteria for rheumatoid arthritis (mainly level of radiological progression) (I~IV)

Class  Functional classification criteria for rheumatoid arthritis (mainly level of difficulty in terms of daily living) (I~IV)

DAS is an evaluation method recommended by EULAR (The European League Against Rheumatism). Absolute values of disease activity are calculated.

DAS28 assessment is narrowed down to 28 joints.

DAS28 is calculated with the formula* by measuring the following 4 items.

(1) Tender joints
(2) Swollen joints
(3) Patient global health condition (in terms of VAS)
(4) CRP or ESR

Formula*

$$DAS28 = 0.56 \times \sqrt{T28} + \sqrt{s28} + 0.7 \times \ln(CRP) + 0.014 \times GH$$

<References> Van Der Heijde DMFM et al. Ann Rheum Dis 49, 916-920, 1990
Van Der Heijde DMFM et al. Ann Rheum Dis 51, 177-181, 1992

49 naive patients received 16 weeks of tocilizumab therapy. 56% of the patients thereof (27 patients) exhibited remission and the rest of the 21 patients exhibited non-remission (Figure 5). Furthermore, 39 switch patients received 16 weeks of tocilizumab therapy, of which 9 patients exhibited remission and the rest of the 30 patients exhibited non-remission.

Further, 49 naive patients received 16 weeks of etanercept therapy, which is anti-TNF-$\alpha$ therapy. 18 patients thereamong exhibited remission and the remaining 31 patients exhibited non-remission (Figure **5**). In the present test, the number of switch patients was extremely low. Thus, only naive patients were used in the analysis for etanercept therapy. It is understood that data for naive patients can be analogically inferred to be applicable to switch patients.

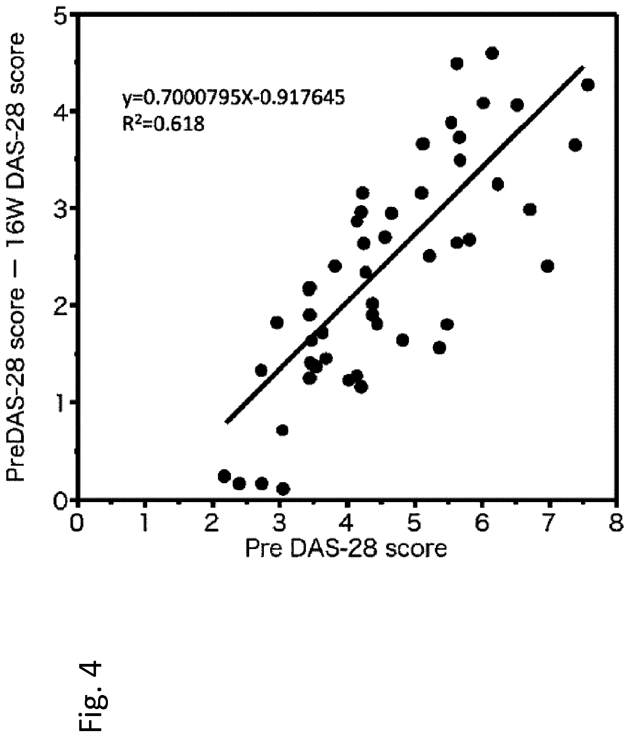

Figure **4** shows the relationship between a DAS-28 value prior to therapy and a value obtained from subtracting a DAS-28 value after 16 weeks from the DAS-28 value prior to therapy (PreDAS-28score - 16W DAS-28 score) in a naive patient who has received tocilizumab therapy (PreDAS-28 score). According to Figure **4**, improvement in DAS-28 values was observed after tocilizumab therapy in most naive patients.

(Search for biomarkers based on DAS-28 value after 16 weeks of therapy by using serum concentration of cytokines/chemokines/soluble receptors in rheumatism patient prior to therapy)

According to the current results, about 55% of naive patients and about 23% of switch patients are expected to exhibit remission after tocilizumab therapy. Although the final symptoms are not identical, some improvement in the symptom is observed in about 45% of naive patients and about 77% of switch patients. Further, about 36.7% of naive patients are expected to exhibit remission after etanercept therapy. In addition, some improvement in the symptom is observed after etanercept therapy in about 60% of the remaining patients.

It has been reported by Pers Y.M. et al (Rheumatology (2013) doi: 10.1093/rheumatology/ket301 First published online: September 19, 2013) that when DAS-28 values after 12-24 weeks of tocilizumab therapy were assessed from general medical examination, 40% of patients exhibited remission, and the main prediction markers were young patients, high CRP value, and patients without a cardiovascular disorder. Further, Koike T (J. Rheumatology, November 2013) et al reported 47.6% remission with respect to DAS28-ESR after 28 weeks of tocilizumab therapy. Meanwhile for etanercept, it is reported by Markenson JA et al (J. Rheumatology, July 2011, p. 1273-81) in the RADIUS study and Curtis JR et al (Ann RheumDis. 2012. 71. 206-212) in the TEMPO study that patients achieving a low disease activity of DAS-28 value $\leq$ 3.2 after 52 weeks and remission were 53%, and 63% when methotrexate is added. Further, Koike T et al (J. Rheumatology, October 2013, p. 1658-1668) have demonstrated that therapy of etanercept adding with methotrexate, when assessing DAS-28 values after 24 weeks, was more effective than therapy with etanercept alone or therapy adding an anti-rheumatism agent (DMARD) other than methotrexate. Furthermore, it is reported by Cannon GW et al (Clin Exp Rheumatol. November 2013) that 35% reached remission in a 3 year observation from TEMPO and RADIUS studies. Furthermore, Cannon GW et al demonstrated that patients with low disease activity are more likely exhibit remission. However, these reports do not reveal a marker for predicting and determining a therapeutic effect due to a biological formulation.

In this regard, serum concentrations of cytokines/chemokines were used to investigate whether it is possible to estimate the level of improvement in rheumatoid arthritis based on DAS-28 values after 16 weeks of therapy.

Figures **6-1** to **6-4** show comparisons of baseline individual group statistics of cytokines/chemokines/soluble receptors prior to therapy for healthy individuals and three groups (naive patients who received tocilizumab therapy, switch patients who received tocilizumab therapy, naive patients who received etanercept therapy). As can be seen from Figures **6-1** to **6-4**, serum concentration other than those for sgp130, sIL-6R and sTNFRI in rheumatoid arthritis patients were significantly higher in comparison to healthy individuals. Further, serum concentrations of cytokines/chemokines were lower in naive patients who received etanercept therapy in comparison to naive patients who received tocilizumab therapy. Further, it was revealed from this result that naive patients who received anti-IL-6 (tocilizumab) therapy have a higher CRP value prior to therapy in comparison to naive patients who received anti-TNF-$\alpha$ (etanercept) therapy.

Simple linear regression analysis was performed to find the cytokine/chemokine involved in the level of improvement in DAS-28 values. The level of improvement in a DAS-28 value (DAS-28 value prior to therapy - DAS-28 value after 16 weeks of therapy) was used as an objective variable and serum concentration of cytokines/chemokines/soluble receptors were used directly, or by converting into a log value, as an independent variable. The results are shown in Table 2. As shown in Table 2, logIL-7, logIL-8, logIL-12, logIL-13, logIP-10 and logVEGF exhibiting p < 0.05 significantly matched the level of improvement in DAS-28 values in naive patients who received anti-IL-6 therapy (tocilizumab therapy). Further, for switch patients who received anti-IL-6 therapy (tocilizumab therapy), logIL-1$\beta$, logIL-5, logIL-6, logIL-7, logIL-10, logIL-12, logIL-13, logIL-15, logFGF, logGM-CSF, logIFN-$\gamma$, logTNF-$\alpha$ and logVEGF significantly matched the level of improvement in DAS-28 values. Meanwhile, logIL-6 and logIP-10 significantly matched the level of improvement in DAS-28 values for naive patients who received anti-TNF-$\alpha$ therapy (etanercept therapy).

[Table 2]

Simple linear regression analysis  Level of improvement in DAS-28

Objective variable : DAS-28 improvement   (=0 week DAS-28 value - 16 week DAS-28 value)

| Cytokine/Chemokine | Naïve patients who received tocilizumab therapy | | Switch patients who received tocilizumab therapy | | Naïve patients who received etanercept therapy | |
|---|---|---|---|---|---|---|
| | Estimates | p value | Estimates | p value | Estimates | p value |
| logHu IL-1b | 0.211 | 0.513 | 0.944 | 0.008 | 0.280 | 0.321 |
| logHu IL-1ra | 0.298 | 0.189 | 0.388 | 0.142 | 0.240 | 0.341 |
| logHu IL-2 | 0.294 | 0.213 | 0.522 | 0.278 | 0.240 | 0.246 |
| logHu IL-4 | 0.836 | 0.158 | 0.788 | 0.238 | 0.453 | 0.344 |
| logHu IL-5 | 0.634 | 0.133 | 1.337 | 0.003 | 0.198 | 0.698 |
| logHu IL-6 | 0.519 | 0.057 | 0.701 | 0.018 | 0.572 | 0.040 |
| logHu IL-7 | 0.890 | 0.035 | 1.204 | 0.011 | 0.207 | 0.578 |
| logHu IL-8 | 1.803 | 0.043 | 0.447 | 0.439 | 0.743 | 0.230 |
| logHu IL-9 | 0.345 | 0.136 | 0.327 | 0.169 | 0.182 | 0.460 |
| logHu IL-10 | 0.589 | 0.059 | 0.860 | 0.011 | 0.054 | 0.865 |
| logHu IL-12 | 0.918 | 0.010 | 1.059 | 0.008 | 0.004 | 0.990 |
| logHu IL-13 | 0.755 | 0.036 | 0.930 | 0.016 | -0.023 | 0.958 |
| logHu IL-15 | 0.278 | 0.098 | 0.433 | 0.010 | 0.306 | 0.073 |
| logHu IL-17 | 0.438 | 0.453 | 0.536 | 0.431 | -0.174 | 0.673 |
| logHu Eotaxin | 0.574 | 0.084 | 0.763 | 0.068 | 0.570 | 0.122 |
| logHu FGF basic | 0.333 | 0.398 | 0.978 | 0.045 | 0.276 | 0.589 |
| logHu G-CSF | 0.290 | 0.578 | 1.331 | 0.084 | 0.347 | 0.479 |
| logHu GM-CSF | 0.143 | 0.573 | 0.692 | 0.002 | 0.115 | 0.875 |
| logHu IFN-g | 0.297 | 0.397 | 1.069 | 0.005 | 0.289 | 0.381 |
| logHu IP-10 | 1.119 | 0.009 | 0.582 | 0.256 | 0.969 | 0.049 |
| logHu MCP-1 | 0.610 | 0.135 | 0.543 | 0.208 | 0.659 | 0.103 |
| logHu MIP-1a | 0.862 | 0.057 | 0.751 | 0.099 | 0.451 | 0.262 |
| logHu PDGF-bb | 0.858 | 0.104 | 0.258 | 0.650 | -0.845 | 0.323 |
| logHu MIP-1b | 1.108 | 0.108 | 0.124 | 0.842 | 0.461 | 0.258 |
| logHu RANTES | 0.664 | 0.144 | 0.297 | 0.519 | -0.826 | 0.348 |
| logHu TNF-a | 0.384 | 0.167 | 0.810 | 0.010 | 0.398 | 0.099 |
| logHu VEGF | 0.998 | 0.007 | 0.899 | 0.028 | 0.208 | 0.626 |
| sgp130 | 0.000 | 0.216 | 0.000 | 0.382 | 0.000 | 0.669 |
| logHu-sIL-6R | -0.881 | 0.292 | 0.783 | 0.370 | -0.798 | 0.332 |
| logHu-sTNFRI | -0.360 | 0.817 | -0.555 | 0.439 | 0.131 | 0.828 |
| logHu-sTNFRII | -0.855 | 0.312 | -0.111 | 0.889 | -0.083 | 0.863 |
| CRP | 0.081 | 0.025 | 0.064 | 0.265 | 0.014 | 0.841 |
| 0wDAS28-CRP | 0.893 | <0.0001 | 0.741 | <0.0001 | 0.597 | <0.0001 |
| MMP | 0.001 | 0.384 | 0.002 | 0.098 | -0.001 | 0.473 |
| RF | 0.001 | 0.090 | 0.004 | 0.039 | 0.001 | 0.220 |
| VAS | 0.029 | <0.0001 | 0.020 | 0.024 | 0.025 | <0.0001 |
| Swollen joint count | 0.082 | 0.002 | 0.133 | 0.026 | 0.102 | 0.009 |
| Tender joint count | 0.106 | <0.0001 | 0.166 | 0.009 | 0.121 | 0.000 |

Multiple linear regression analysis was performed to find the correlation between the level of improvement in DAS-28 value and cytokine/chemokine/soluble receptor concentration. As a result, it was found by phased multiple regression analysis that a combination of logIL-1β, logIL-7, logTNF-α and logsIL-6R is significantly correlated with the level of improvement in DAS-28 values in naive patients who received anti-IL-6 therapy (tocilizumab therapy) (Table 3).
Meanwhile, a combination of logIL-2, logIL-15, logIL-6R, and logTNFRI was found to have significant correlation with the level of improvement in DAS-28 values in naive patients who received anti-TNF-α therapy (etanercept therapy) (Table 4).

[Table 3]

Multiple linear regression analysis on naïve patients who received tocilizumab therapy
Level of improvement in DAS-28

Objective variable : DAS-28 improvement
(=0 week DAS-28 value - 16 week DAS-28 value)

| Naïve patients who received tocilizumab therapy | | |
|---|---|---|
| Multiple regression analysis  (Objective value=0w~16wDAS28) | | |
| R^2 | 0.376 | |
| ANOVA (Analysis of variance) | p=0.0004 | |
| Cytokine/Chemokine/soluble receptor | Estimate | p value |
| intercept | 5.505 | 0.1216 |
| logHu IL-1b | -3.618 | 0.0002 |
| logHu IL-7 | 3.255 | 0.0002 |
| logHu TNF-a | 1.475 | 0.0221 |
| logHu-sIL-6R | -1.814 | 0.0264 |

[Table 4]

## Naïve patients who received etanercept therapy
## Multiple linear regression analysis　Level of improvement in DAS-28

Objective variable : DAS-28 improvement
(=0 week DAS-28 value - 16 week DAS-28 value)

| Naïve patients who received etanercept therapy | | |
|---|---|---|
| Multiple regression analysis　(Objective value=0w-16wDAS28) | | |
| $R^2$ | 0.343 | |
| ANOVA(Analysis of variance) | p=0.0037 | |
| Cytokine/Chemokine/soluble receptor | Estimate | p value |
| intercept | 7.325 | 0.0231 |
| logHu IL-2 | -1.567 | 0.0058 |
| logHu IL-15 | 1.632 | 0.0008 |
| logHusIL-6R | -2.540 | 0.0130 |
| logHu-sTNFRI | 1.973 | 0.0115 |

Simple linear regression analysis was performed to find the cytokine/chemokine/soluble receptor involved in the final assessment of a DAS-28 value after 16 weeks of therapy (16wDAS28). The DAS-28 value after 16 weeks of therapy was used as an objective variable and serum concentration of cytokines/chemokines/soluble receptors were used directly, or by converting into a log value, as an independent variable. As shown in Table 5, sgp130 exhibiting p < 0.05 significantly matched DAS-28 values after 16 weeks of therapy in naive patients who received anti-IL-6 therapy (tocilizumab therapy). Further, for switch patients who received anti-IL-6 therapy (tocilizumab therapy), logIL-1β, logIL-2, logIL-5, logIL-15, logGM-CSF, logIFN-γ, logTNF-α and sgp130 significantly matched DAS-28 values after 16 weeks of therapy. Meanwhile, logIL-9 significantly matched DAS-28 values after 16 weeks of therapy for naive patients who received anti-TNF-α therapy (etanercept therapy).

[Table 5]

Simple linear regression analysis 16-week DAS-28

Objective variable: 16-week DAS-28
Simple linear regression anlysis of cytokine /chemokine /soluble receptor based on DAS-28 16w
Simple linear regression anlysis were performed to find
the parameters related to 16wDAS-28 (=16wDAS28),

| Cytokine/Chemokine | | Naïve Tocilizumab Therapy | | Switch Tocilizumab Therapy | | Naïve Etanercept Therapy | |
| --- | --- | --- | --- | --- | --- | --- | --- |
| | | Tocilizumab naïve | | Tocilizumab switch | | Etanercept naïve | |
| | | Estimates | p value | Estimates | p value | Estimates | p value |
| logHu IL-1b | pg/ml | 0.094 | 0.681 | -0.604 | 0.035 | -0.047 | 0.860 |
| logHu IL-1ra | pg/ml | -0.178 | 0.269 | 0.041 | 0.850 | 0.053 | 0.817 |
| logHu IL-2 | pg/ml | -0.078 | 0.644 | -0.482 | 0.012 | 0.179 | 0.341 |
| logHu IL-4 | pg/ml | 0.335 | 0.426 | -0.798 | 0.140 | -0.190 | 0.661 |
| logHu IL-5 | pg/ml | -0.131 | 0.606 | -0.832 | 0.025 | 0.119 | 0.724 |
| logHu IL-6 | pg/ml | 0.286 | 0.156 | -0.301 | 0.216 | 0.095 | 0.712 |
| logHu IL-7 | pg/ml | 0.026 | 0.933 | -0.617 | 0.119 | 0.199 | 0.550 |
| logHu IL-8 | pg/ml | 0.568 | 0.319 | 0.168 | 0.721 | -0.175 | 0.756 |
| logHu IL-9 | pg/ml | -0.174 | 0.291 | -0.190 | 0.330 | 0.545 | 0.011 |
| logHu IL-10 | pg/ml | -0.232 | 0.298 | -0.395 | 0.163 | 0.351 | 0.217 |
| logHu IL-12 | pg/ml | -0.202 | 0.438 | -0.529 | 0.115 | 0.413 | 0.177 |
| logHu IL-13 | pg/ml | -0.100 | 0.699 | -0.533 | 0.096 | 0.467 | 0.236 |
| logHu IL-15 | pg/ml | -0.053 | 0.660 | -0.325 | 0.019 | 0.092 | 0.557 |
| logHu IL-17 | pg/ml | -0.578 | 0.158 | -0.619 | 0.262 | -0.578 | 0.556 |
| logHu Eotaxin | pg/ml | -0.363 | 0.124 | -0.360 | 0.291 | 0.056 | 0.868 |
| logHu FGF basic | pg/ml | -0.168 | 0.546 | -0.688 | 0.085 | 0.493 | 0.281 |
| logHu G-CSF | pg/ml | -0.321 | 0.380 | -0.978 | 0.120 | -0.032 | 0.943 |
| logHu GM-CSF | pg/ml | -0.036 | 0.839 | -0.589 | 0.001 | 0.191 | 0.368 |
| logHu IFN-g | pg/ml | 0.038 | 0.879 | -0.709 | 0.024 | 0.015 | 0.960 |
| logHu IP-10 | pg/ml | -0.048 | 0.877 | 0.241 | 0.568 | 0.104 | 0.818 |
| logHu MCP-1 | pg/ml | 0.144 | 0.623 | -0.113 | 0.738 | 0.009 | 0.981 |
| logHu MIP-1a | pg/ml | 0.198 | 0.591 | -0.388 | 0.301 | 0.051 | 0.890 |
| logHu PDGF-bb | pg/ml | 0.097 | 0.798 | -0.165 | 0.720 | 0.794 | 0.301 |
| logHu MIP-1b | pg/ml | 0.351 | 0.477 | -0.284 | 0.573 | -0.396 | 0.281 |
| logHu RANTES | pg/ml | 0.249 | 0.444 | -0.452 | 0.224 | 0.382 | 0.631 |
| logHu TNF-a | pg/ml | -0.033 | 0.865 | -0.646 | 0.012 | 0.035 | 0.875 |
| logHu VEGF | pg/ml | 0.400 | 0.139 | -0.042 | 0.902 | 0.573 | 0.132 |
| sgp130 | μ g/ml | -3.785 | 0.046 | -7.801 | 0.001 | -3.005 | 0.207 |
| logHu-sIL-6R | pg/ml | -0.866 | 0.187 | -1.246 | 0.075 | -0.754 | 0.336 |
| logHu-sTNFRI | pg/ml | -1.028 | 0.039 | 0.033 | 0.955 | 0.094 | 0.902 |
| logHu-sTNFRII | pg/ml | -0.179 | 0.766 | 0.078 | 0.728 | 0.690 | 0.115 |
| DAS-28 0w | | 0.306 | 0.000 | 0.259 | 0.097 | 0.403 | 0.003 |
| MMP | | 0.000 | 0.645 | 0.000 | 0.464 | 0.002 | 0.023 |
| RF | | 0.001 | 0.378 | 0.000 | 0.818 | 0.000 | 0.504 |
| VAS | | 0.003 | 0.642 | 0.004 | 0.560 | 0.008 | 0.334 |
| Swollen joint count | | 0.069 | 0.000 | 0.066 | 0.183 | 0.081 | 0.022 |
| Tender joint count | | 0.067 | 0.000 | 0.074 | 0.166 | 0.042 | 0.193 |
| Stage | | 0.092 | 0.418 | 0.393 | 0.162 | -0.197 | 0.232 |
| Class | | 0.188 | 0.483 | 0.130 | 0.680 | 0.453 | 0.096 |

Multiple linear regression analysis was performed to find the correlation between DAS-28 value after 16 weeks of therapy and cytokine/chemokine/soluble receptor concentration. As a result thereof, it was found by phased multiple regression analysis that a combination of sgp130, logIL-8, logEotaxin, logIP-10, logTNFRI, logTNFRII, logIL-6, and logIL-VEGF is significantly correlated with a DAS-28 value after 16 weeks of therapy in naive patients who received anti-IL-6 therapy (tocilizumab therapy) as shown in Table 6. Further, it was found that there is a very significant correlation even without using logIL-VEGF (Table 7).

Further, it was found that a combination of sgp130, logIP-10, and logGM-CSF is significantly correlated with a DAS-28 value after 16 weeks of therapy in switch patients who received anti-IL-6 therapy (tocilizumab therapy) (Table 8).

Meanwhile, a combination of DAS-28 value prior to therapy, logIL-6 and logIL-13 was also found to be significantly correlated with the level of improvement in DAS-28 value for naive patients who received anti-TNF-α therapy (etanercept therapy) (Table 9). Further, it was found that a combination of logIL-9, logTNF-α, and logVEGF, even without using a DAS-28 value prior to therapy, is significantly correlated with a DAS-28 value after 16 weeks of therapy naive patients who received anti-TNF-α therapy (etanercept therapy) (Table 10).

[Table 6]

Tocilizumab naïve multiple linear regression analysis
Objective variable 16-week DAS-28

Multiple linear regression anlysis of cytokine /chemokine
/soluble receptor based on 16w DAS-28
A.Multiple regression anlysis were performed to find the
parameters related to 16wDAS-28 (=16wDAS28).

| Naïve Tocilizumab Therapy | Tocilizumab naïve | |
|---|---|---|
| Multiple regression analysis (Objective value=16wDAS28) | | |
| R^2 | 0.646 | |
| ANOVA(Analysis of variance) | p<0.0001 | |
| Cytokine/Chemokine/soluble receptor | Estimate | p value |
| intercept | 6.909 | 0.001 |
| sgp130# | −0.534 | 0.002 |
| log IL−8 | 3.940 | <.0001 |
| log Eotaxin | −1.039 | <.0001 |
| log IP−10 | −1.002 | 0.002 |
| log sTNFRI | −2.580 | <.0001 |
| log sTNFRII | 1.407 | 0.030 |
| log IL−6 | 0.744 | 0.002 |
| log VEGF | −0.850 | 0.039 |

sgp130# : μ g/ml
others : pg/ml

[Table 7]

Tocilizumab naïve multiple linear regression analysis
Objective variable 16-week DAS-28

Multiple linear regression anlysis of cytokine /chemokine
/soluble receptor based on 16w DAS-28
A.Multiple regression anlysis were performed to find the
parameters related to 16wDAS-28 (=16wDAS28).

| Naïve Tocilizumab Therapy | Tocilizumab naïve | |
|---|---|---|
| Multiple regression analysis  (Objective value=16wDAS28) | | |
| R^2 | 0.605 | |
| ANOVA(Analysis of variance) | p<0.0001 | |
| Cytokine/Chemokine/soluble receptor | Estimate | p value |
| intercept | 4.731 | 0.0127 |
| sgp130# | −0.543 | 0.003 |
| log IL-8 | 2.551 | <.0001 |
| log Eotaxin | −0.937 | 0.0004 |
| log IP-10 | −1.116 | 0.0007 |
| log sTNFRI | −2.010 | 0.0004 |
| log sTNFRII | 1.630 | 0.0152 |
| log IL-6* | 0.577 | 0.0096 |

sgp130# : μ g/ml
others : pg/ml

[Table 8]

Tocilizumab switch multiple linear regression analysis
Objective variable 16-week DAS-28

Multiple linear regression anlysis of cytokine /chemokine
/soluble receptor based on 16w DAS-28
A.Multiple regression anlysis were performed to find the
parameters related to 16wDAS-28 (=16wDAS28).

| Tocilizumab switch | | |
|---|---|---|
| Multiple regression analysis  (Objective value=16wDAS28) | | |
| R^2 | 0.486 | |
| ANOVA(Analysis of variance) | p<0.0001 | |
| Cytokine/Chemokine/soluble receptor | Estimate | p value |
| intercept | 2.837 | 0.011 |
| sgp130# | −0.604 | 0.003 |
| log IP-10 | 0.714 | 0.003 |
| log GM-CSF | −0.622 | 0.0003 |

sgp130# : μg/ml
others : pg/ml

[Table 9]

## Multiple linear regression analysis on naïve patients who received etanercept therapy

Multiple linear regression anlysis of cytokine/chemokine/soluble receptor and DAS28-CRP before therapy on 16week Das-28.

## Objective variable: 16-week DAS-28

| Naïve Etanercept Therapy | | |
|---|---|---|
| Multiple regression analysis (Objective value=16wDAS28) | | |
| R^2 | 0.321 | |
| ANOVA(Analysis of variance) | p=0.0016 | |
| Cytokine/Chemokine/soluble receptor | estimate | p value |
| intercept | 0.081 | 0.907 |
| DAS28-CRP (Prior to therapy) | 0.522 | 0.000 |
| logHu IL-6 | -0.969 | 0.015 |
| log HuIL-13 | 1.409 | 0.015 |

[Table 10]

## Etanercept naïve multiple linear regression analysis Objective variable 16-week DAS-28

Multiple linear regression anlysis of cytokine /chemokine /soluble receptor based on 16w DAS-28
A.Multiple regression anlysis were performed to find the parameters related to 16wDAS-28 (=16wDAS28).

| Tocilizumab switch | | |
|---|---|---|
| Multiple regression analysis (Objective value=16wDAS28) | | |
| R^2 | 0.264 | |
| ANOVA(Analysis of variance) | p=0.0093 | |
| Cytokine/Chemokine/soluble receptor | Estimate | p value |
| intercept | 0.703 | 0.348 |
| log IL-9 | 0.646 | 0.007 |
| log TNF-α | -0.551 | 0.039 |
| log VEGF | 0.858 | 0.053 |

IL-9, TNF-α, VEGF : pg/ml

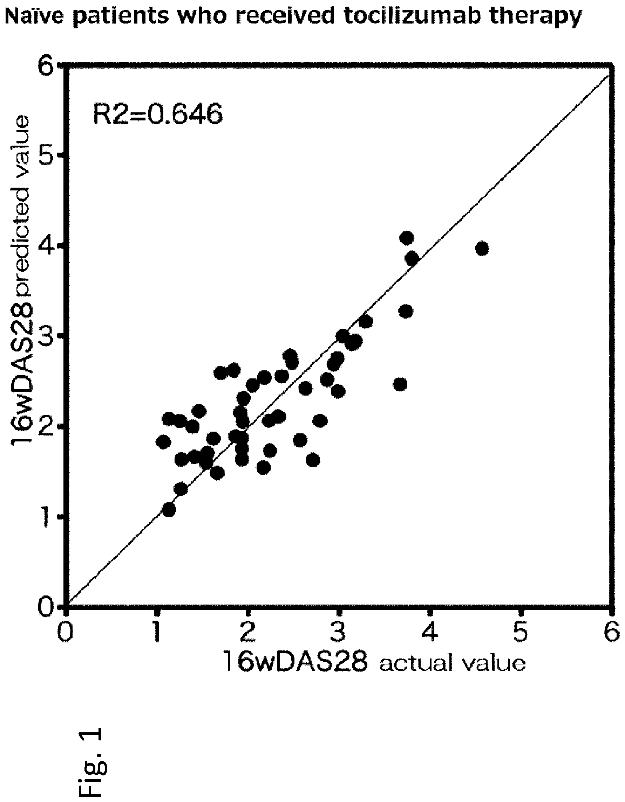

Further, regression equation (4) found based on the multiple linear regression analysis shown in Table 7 was used to find a predicted value of DAS-28 value after 16 weeks of therapy in naive patients who received anti-IL-6 therapy (tocilizumab therapy). Figure 1 shows the results of comparing predicted values of DAS-28 values after 16 weeks of therapy calculated by regression equation (4) and actual values of DAS-28 values after 16 weeks of therapy. It was confirmed from the results that DAS-28 values after 16 weeks of therapy estimated from the results of multiple linear regression analysis shown in Table 7 are very consistent with actual values of DAS-28 values after 16 weeks of therapy.

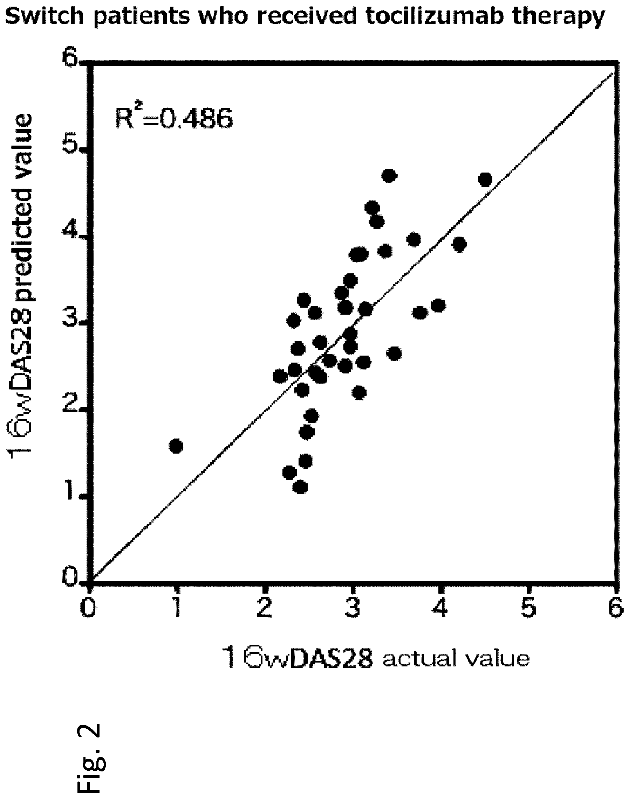

Further, regression equation (5) found based on the multiple linear regression analysis shown in Table 8 was used to find a predicted value of DAS-28 value after 16 weeks of therapy in switch patients who received anti-IL-6 therapy (tocilizumab therapy). Figure **2** shows the results of comparing the predicted values of DAS-28 values after 16 weeks of therapy calculated by regression equation (5) and actual values of DAS-28 values after 16 weeks of therapy. It was confirmed from the results that DAS-28 values after 16 weeks of therapy estimated from the results of multiple linear regression analysis shown in Table 8 are very consistent with actual values of DAS-28 values after 16 weeks of therapy.

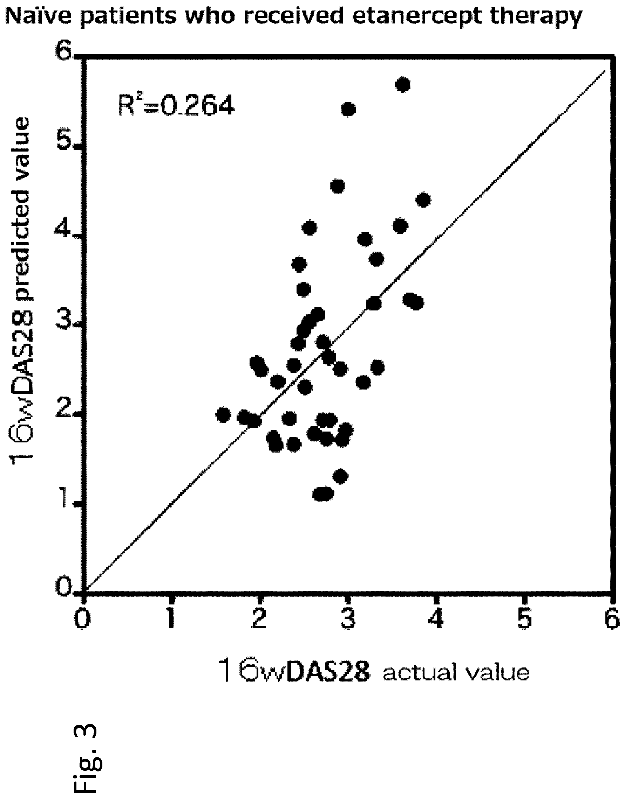

Regression equation (7) found based on the multiple linear regression analysis shown in Table 10 was used to find a predicted value of DAS-28 value after 16 weeks of therapy in naive patients who received anti-TNF-$\alpha$ therapy (etanercept therapy). Figure **3** shows the results of comparing predicted values of DAS-28 values after 16 weeks of therapy calculated by regression equation (7) and actual values of DAS-28 values after 16 weeks of therapy. It was confirmed from the results that DAS-28 values after 16 weeks of therapy can be estimated to a certain extent from the results of multiple linear regression analysis shown in Table 10.

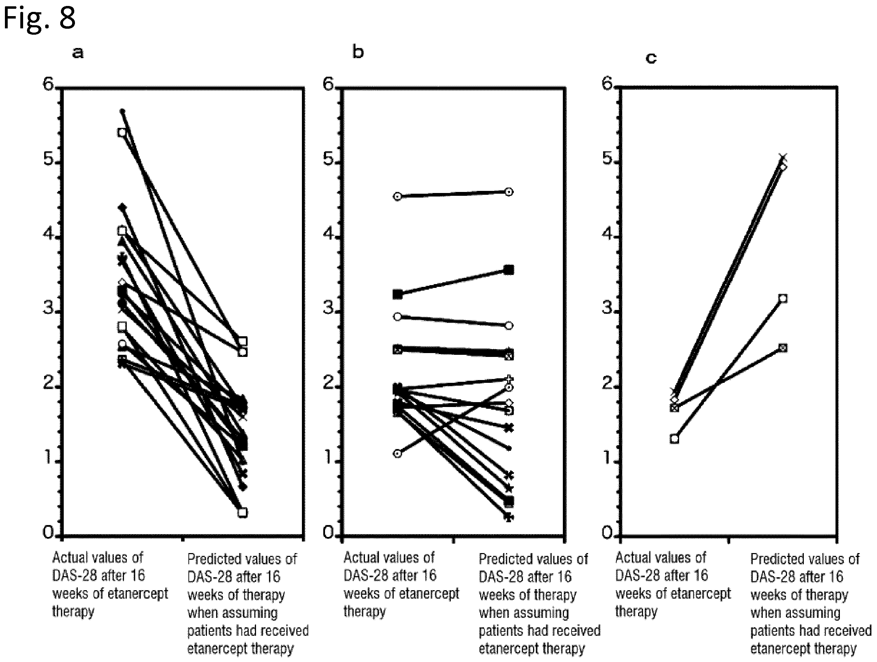

Further, a predicted value of DAS-28 after 16 weeks of therapy was found by using the aforementioned regression equation (4) while assuming that naive patients who received anti-TNF-$\alpha$ therapy (etanercept therapy) had received anti-IL-6 therapy (tocilizumab therapy) without receiving anti-TNF-$\alpha$ therapy (etanercept therapy). Figure **8** shows the actual values of DAS-28 after 16 weeks of anti-TNF-$\alpha$ therapy (etanercept therapy) and predicted values of DAS-28 values after 16 weeks of therapy while assuming that anti-IL-6 therapy (tocilizumab therapy) was received. From this result, naive patients who received anti-TNF-$\alpha$ therapy (etanercept therapy) are classified into patients who are predicted to have a higher therapeutic effect when receiving anti-IL-6 therapy (tocilizumab therapy) (Figure **8 a**), patients who are predicted to have barely any difference observed between anti-TNF-$\alpha$ therapy (etanercept therapy) and anti-IL-6 therapy (tocilizumab therapy) (Figure **8 b**), and patients who are predicted to have a higher therapeutic effect observed when receiving anti-TNF-$\alpha$ therapy (etanercept therapy) (Figure **8 c**). For patients shown in Figure **8 a**, anti-IL-6 therapy (tocilizumab therapy) is estimated to be more effective than anti-TNF-$\alpha$ therapy (etanercept therapy) that was actually received. Thus, it was found that a more effective therapeutic agent can be selected by estimating DAS-28 values due to anti-IL-6 therapy and anti-TNF-$\alpha$ therapy prior to therapy by the present invention.

(Search for biomarkers for predicting and determining the possibility of remission by using serum concentration of cytokines/chemokines/soluble receptors in rheumatism patient prior to therapy)

In therapy of rheumatoid arthritis, it is desirable that even a partial improvement is observed in the symptom of a patient. However, it is most desirable to reach complete remission. In this regard, in addition to a search for various factors for estimating the final DAS-28 value, a search was conducted for cytokines/chemokines/soluble receptors for predicting whether a patient reaches complete remission.

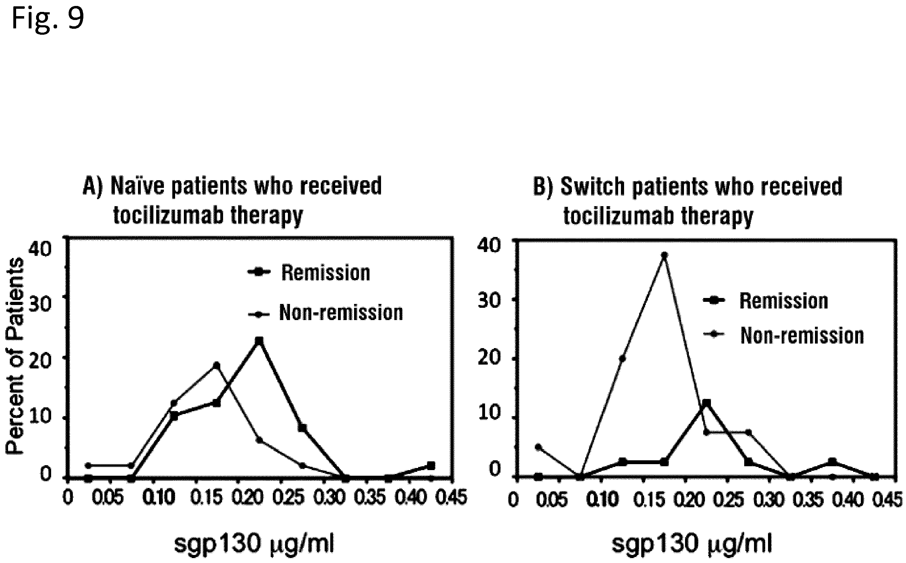

Data for cytokine/chemokine/soluble receptor concentrations was analyzed for complete remission and non-remission patient groups by simple logistic regression analysis. Further, Table 10 shows the results of analyzing data for cytokine/chemokine/soluble receptor concentrations for naive patients and switch patients who received anti-IL-6 therapy (tocilizumab therapy) and naïve patients who received anti-TNF-$\alpha$ therapy (etanercept therapy). It was found by simple logistic regression analysis that swollen joint count and tender joint count and DAS-28 values were significantly different between complete remission and non-remission groups. Furthermore, sgp130 was significantly different between complete remission and non-remission groups in naive and switch patients who received anti-IL-6 therapy (tocilizumab therapy) (Table 11). Meanwhile, significant difference in sgp130 was not observed between remission and non-remission groups in naive patients who received anti-TNF-$\alpha$ therapy (etanercept therapy) (Table 11). Further, Figure **9** shows the results of analyzing the relationship between serum sgp130 concentration and DAS-28 value prior to therapy for remission and non-remission patients. As is clear from Figure **9**, many patients who have reached remission had a high sgp130 concentration.

[Table 11]

## Simple logistic regression analysis

| Cytokine/Chemokine | | Naïve patients who received tocilizumab therapy (n=48) | | Switch patients who received tocilizumab therapy (n=40) | | Naïve patients who received etanercept therapy (n=43) | |
|---|---|---|---|---|---|---|---|
| | | Whole Model Test — Single logistic analysis, p value | Parameter Estimates — Estimates | Whole Model Test — Single logistic analysis, p value | Parameter Estimates — Estimates | Whole Model Test — Single logistic analysis, p value | Parameter Estimates — Estimates |
| logHu IL-1b | pg/ml | 0.378 | 0.486 | 0.147 | −1.081 | 0.577 | −0.274 |
| logHu IL-1ra | pg/ml | 0.148 | −0.628 | 0.087 | 1.573 | 0.323 | 0.478 |
| logHu IL-2 | pg/ml | 0.856 | 0.074 | 0.080 | −1.009 | 0.857 | 0.064 |
| logHu IL-4 | pg/ml | 0.534 | 0.638 | 0.420 | −1.241 | 0.965 | −0.035 |
| logHu IL-5 | pg/ml | 0.814 | 0.143 | 0.189 | −1.276 | 0.896 | −0.083 |
| logHu IL-6 | pg/ml | 0.184 | 0.663 | 0.270 | −0.710 | 0.950 | −0.030 |
| logHu IL-7 | pg/ml | 0.585 | 0.401 | 0.233 | −1.231 | 0.660 | 0.280 |
| logHu IL-8 | pg/ml | 0.432 | 1.088 | 0.864 | −0.207 | 0.751 | −0.333 |
| logHu IL-9 | pg/ml | 0.545 | −0.242 | 0.289 | −0.540 | 0.020 | 1.075 |
| logHu IL-10 | pg/ml | 0.604 | −0.281 | 0.196 | −0.948 | 0.572 | 0.310 |
| logHu IL-12 | pg/ml | 0.773 | −0.181 | 0.113 | −1.457 | 0.833 | 0.123 |
| logHu IL-13 | pg/ml | 0.963 | 0.029 | 0.432 | −0.669 | 0.671 | 0.322 |
| logHu IL-15 | pg/ml | 0.924 | 0.027 | 0.173 | −0.519 | 0.942 | 0.021 |
| logHu IL-17 | pg/ml | 0.197 | −1.332 | 0.920 | 0.147 | 0.691 | −0.730 |
| logHu Eotaxin | pg/ml | 0.447 | −0.441 | 0.512 | −0.590 | 0.639 | 0.299 |
| logHu FGF basic | pg/ml | 0.792 | −0.177 | 0.725 | −0.371 | 0.402 | 0.773 |
| logHu G-CSF | pg/ml | 0.599 | −0.471 | 0.786 | −0.450 | 0.901 | −0.104 |
| logHu GM-CSF | pg/ml | 0.910 | 0.104 | 0.099 | −0.930 | 0.798 | −0.102 |
| logHu IFN-g | pg/ml | 0.536 | 0.369 | 0.190 | −1.081 | 0.681 | −0.228 |
| logHu IP-10 | pg/ml | 0.647 | −0.344 | 0.604 | 0.557 | 0.393 | 0.733 |
| logHu MCP-1 | pg/ml | 0.402 | 0.593 | 0.696 | −0.366 | 0.960 | 0.035 |
| logHu MIP-1a | pg/ml | 0.428 | 0.698 | 0.306 | −0.963 | 0.885 | −0.098 |
| logHu PDGF-bb | pg/ml | 0.751 | 0.290 | 0.458 | 0.894 | 0.356 | 1.357 |
| logHu MIP-1b | pg/ml | 0.709 | 0.444 | 0.608 | −0.882 | 0.161 | −0.991 |
| logHu RANTES | pg/ml | 0.748 | 0.252 | 0.866 | 0.166 | 0.823 | −0.335 |
| logHu TNF-a | pg/ml | 0.787 | 0.127 | 0.143 | −1.020 | 0.694 | −0.182 |
| logHu VEGF | pg/ml | 0.400 | 0.558 | 0.389 | −0.793 | 0.967 | 0.030 |
| sgp130 | μ g/ml | −18.182 | 0.003 | −24.159 | 0.003 | 0.212 | −5.882 |
| logHu-sIL-6R | pg/ml | 0.118 | −2.590 | 0.023 | −5.922 | 0.879 | 0.590 |
| logHu-sTNFRI | pg/ml | 0.302 | −1.284 | 0.843 | −0.306 | 0.566 | 0.591 |
| logHu-sTNFRII | pg/ml | 0.719 | −0.519 | 0.064 | 1.210 | 0.390 | 0.775 |
| age | | 0.139 | 0.039 | 0.064 | −0.073 | 0.444 | 0.019 |
| Duration of disease | | 0.228 | 0.041 | 0.221 | −0.059 | 0.414 | 0.033 |
| WBC | | 0.173 | 0.000 | 0.434 | 0.000 | 0.057 | 0.000 |
| DAS28-CRP | | 0.011 | 0.608 | 0.689 | 0.165 | 0.005 | 0.845 |
| VAS | | 0.328 | 0.013 | 0.810 | 0.005 | 0.419 | 0.011 |
| CRP | | 0.993 | 0.001 | 0.939 | −0.009 | 0.019 | 0.342 |
| RF | | 0.121 | 0.002 | 0.995 | 0.000 | 0.015 | 0.006 |
| Swollen joint count | | 0.015 | 0.123 | 0.193 | 0.182 | 0.012 | 0.218 |
| Tender joint count | | 0.014 | 0.123 | 0.363 | 0.137 | 0.048 | 0.147 |
| Stage | | 0.237 | 0.328 | 0.352 | 0.651 | 0.615 | −0.158 |
| Class | | 0.459 | 0.481 | 0.806 | −0.201 | 0.403 | 0.438 |

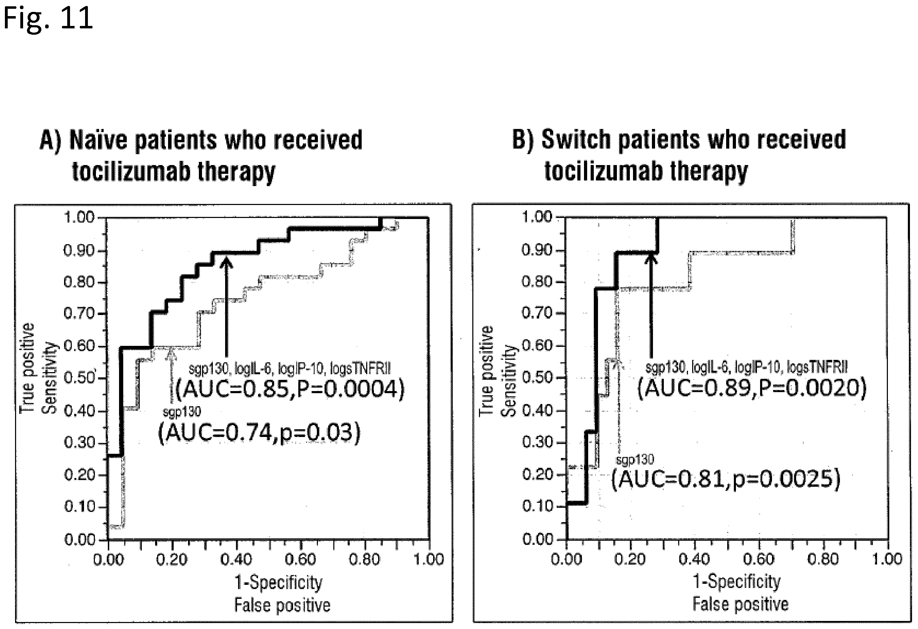

A multivariable model was examined as a prediction biomarker for remission and non-remission by phased multiple forward logistic regression analysis based on serum concentration of cytokines/chemokines/soluble receptors in patients prior to administration of an anti-IL-6 agent (tocilizumab). Tables 12 and 13 show preferred combinations of prediction biomarkers for remission and non-remission found based on phased multiple forward logistic regression analysis and ROC curves. It was found from the results of analysis that sgp130, logIP-10, logsTNFRII and logIL-6 can be prediction biomarkers for determining with high precision whether remission is reached for naive patients who received anti-IL-6 therapy (tocilizumab therapy) (p = 0.0004) (Table 11a). Further, it was found that logIL-7 (p = 0.0003), logIL-1β (p = 0.0005) or logMCP-1 (p = 0.0004), in combination with sgp130, logIP-10, and logsTNFRII, can be a prediction biomarker for determining with high precision whether remission is reached for naive patients who received anti-IL-6 therapy (tocilizumab therapy) (Tables 12b-12d). Figure **11** shows a graph comparing sgp130 alone to a ROC curve combining sgp130 with logIL-6, logIP-10 and logTNFRII. As shown in Figure **11**, it is demonstrated that prediction precision is far higher with a ROC curve combining sgp130 with logIL-6, logIP-10 and logTNFRII than sgp130 alone. In fact, a high value of 0.85 or 0.89 was exhibited in terms of AUC level.

Further, it was found that a combination of sgp130, log IP-10, log sTNFRII and log IL-6 can be a prediction biomarker for determining whether remission is reached for switch patients who received anti-IL-6 therapy (tocilizumab therapy) (p = 0.002) (Table 13a). Furthermore, it was also found that a combination of sgp130, log IP-10, log sTNFRII and log IL-1β can also be a predication biomarker for determining with high precision whether remission is reached (p = 0.003) (Table 13b).

Meanwhile, p value was 0.257 for biomarker groups for predicting and determining the possibility of remission found based on the ROC curve and multiple logistic regression analysis obtained in anti-IL-6 therapy, or tocilizumab therapy, for naïve patients who received anti-TNF-α therapy (etanercept therapy), thus demonstrating that this biomarker group cannot predict whether remission is reached (Table 14). Meanwhile, it was demonstrated that a combination of DAS-28 value prior to therapy (0wDAS-28), log VEGF, and log PDGF-bb can also predict and determine the possibility of remission to a certain extent, as shown in Table 15, by another multiple logistic regression analysis. Furthermore, it was found that a combination of log IL-9 and log TNF-α can also predict and determine the possibility of remission to a certain extent without using DAS-28 value (OwDAS-28) for naïve patients who received anti-TNF-α therapy (etanercept therapy) as shown in Table 16. That is, it is suggested that the pathology of rheumatoid arthritis patients is diverse, and a biomarker for predicting and determining the possibility of remission is different for patients to whom IL-6 inhibition is effective and patients for whom TNF-α inhibition is effective.

[Table 12]

## Tocilizumab naïve Multiple logistic regression analysis multiple logistic analysis. Objective variable :remission vs non-remission

**a.**

| Whole Model Test | | p=0.0004 |
|---|---|---|
| **Parameter Estimates** | | |
| Term | Estimates | p value(Prob>ChiSq) |
| Intercept | -5.092 | 0.466 |
| sgp130 | -35.848 | 0.001 |
| logHu IP-10 | -4.004 | 0.007 |
| logHu-sTNFRII | 5.632 | 0.016 |
| logHu IL-6 | 1.658 | 0.034 |

Area Under
Curve=0.85009

**b.**

| Whole Model Test | | p=0.0003 |
|---|---|---|
| **Parameter Estimates** | | |
| Term | Estimates | p value(Prob>ChiSq) |
| Intercept | -3.467 | 0.621 |
| sgp130 | -42.849 | 0.001 |
| logHu IP-10 | -4.430 | 0.005 |
| logHu-sTNFRII | 5.735 | 0.017 |
| logHu IL-7 | 2.705 | 0.035 |

Area Under
Curve=0.84832

**c.**

| Whole Model Test | | p=0.0004 |
|---|---|---|
| **Parameter Estimates** | | |
| Term | Estimates | p value(Prob>ChiSq) |
| Intercept | -2.834 | 0.684 |
| sgp130 | -38.721 | 0.001 |
| logHu IP-10 | -4.864 | 0.007 |
| logHu-sTNFRII | 5.363 | 0.020 |
| logHu MCP-1 | 2.502 | 0.040 |

Area Under
Curve=0.84832

**d.**

| Whole Model Test | | p=0.0005 |
|---|---|---|
| **Parameter Estimates** | | |
| Term | Estimates | p value(Prob>ChiSq) |
| Intercept | -1.269 | 0.853 |
| sgp130 | -39.538 | 0.001 |
| logHu IP-10 | -3.807 | 0.008 |
| logHu-sTNFRII | 5.088 | 0.028 |
| logHu IL-1b | 1.647 | 0.050 |

Area Under
Curve=0.85362

[Table 13]

Tocilizumab switch Multiple logistic regression analysis multiple logistic analysis, Objective variable remission vs non-remission

| a. Whole Model Test | | p=0.0020 |
| --- | --- | --- |
| Parameter Estimates | | |
| Term | Estimates | p value(Prob>ChiSq) |
| Intercept | -10.935 | 0.190 |
| sgp130# | -29.051 | 0.020 |
| logHu IP-10 | 4.456 | 0.060 |
| logHu-sTNFRII | 2.067 | 0.084 |
| logHu IL-8 | -2.757 | 0.047 |

| b. Whole Model Test | | p=0.0030 |
| --- | --- | --- |
| Parameter Estimates | | |
| Term | Estimate | Prob>ChiSq |
| Intercept | -9.671 | 0.217 |
| sgp130# | -27.150 | 0.025 |
| logHu IP-10 | 3.205 | 0.095 |
| logHu-sTNFRII | 1.914 | 0.080 |
| logHu IL-1b | -2.540 | 0.055 |

Area Under Curve =0.892

sgp130#:μg/ml

Area Under Curve = 0.899

sgp130#:μg/ml

[Table 14]

| Results of multiple logistic regression analysis on naïve patients who received etanercept therapy by using biomarkers for predicting and determining the possibility of remission found based on results of multiple logistic regression analysis obtained from patients who received tocilizumab therapy | | |
| --- | --- | --- |
| Whole Model Teat | | p=0.257 |
| Parameter Estimates | | |
| Term | Estimates | p value(Prob>ChiSq) |
| Intercept | -6.489 | 0.179 |
| sgp130 | -9.591 | 0.150 |
| logHu IL-6 | -0.422 | 0.467 |
| logHu IP-10 | 0.893 | 0.435 |
| logHu-sTNFR II | 1.789 | 0.235 |

[Table 15]

Etanercept naïve Multiple logistic regression analysis multiple logistic analysis, Objective variable :remission vs non-remission

| Whole Model Test | | p=0.0034 |
|---|---|---|
| Parameter Estimates | | |
| Term | Estimates | p value(Prob>ChiSq) |
| Intercept | −19.058 | 0.025 |
| 0w DAS28-CRP | 1.390 | 0.007 |
| log VEGF | −2.763 | 0.045 |
| log PDGF-bb | 4.962 | 0.042 |

Area Under
Curve=0.8055

| Whole Model Test | | p=0.0071 |
|---|---|---|
| Parameter Estimates | | |
| Term | Estimates | p value(Prob>ChiSq) |
| Intercept | 4.491 | 0.031 |
| 0w DAS28-CRP | 1.107 | 0.007 |
| log MIP-1a | −1.808 | 0.082 |
| log PDGF-bb | 3.930 | 0.089 |

Area Under Curve=0.7986

[Table 16]

| Etanercept naive Multiple logistic regression analysis multiple logistic analysis, Objective variable:remission vs non-remission | | |
|---|---|---|
| Whole Model Test | | p=0.0115 |
| Parameter Estimates | | |
| Term | Estimates | p value(Prob>ChiSq) |
| Intercept | -1.004 | 0.337 |
| log IL-9 | 1.711 | 0.012 |
| log TNF-$\alpha$ | -1.031 | 0.079 |

Area Under Curve: 0.745

(Comparison of DAS28-CRP to DAS28-ESR)

As discussed above, it is reported that DAS-28-CRP values are almost interchangeable with DAS-28-ESR values, and the same results are derived therefrom (Ann Rheum Dis.2007, March 407-409 Comparison of Disease Activity Score(DAS)28-erythrocyte Sedimentation rate and DAS-C-reactive protein threshold votes. Inoue E, Yamanaka H, et al.) For verification thereof, multivariable linear regression analysis was performed on DA28-ESR scores after 16 weeks with respect to cytokine/chemokine/soluble receptor levels. The results thereof are shown in the following table.

[Table 17]

| | Naïve patients who received tocilizumab therapy | | Switch patients who received tocilizumab therapy | |
|---|---|---|---|---|
| n (F/M) | n=45 (42/3) | | n=37 (31/6) | |
| $R^2$ | 0.437 | | 0.486 | |
| | P=0.0003 | | P<0.0001 | |
| Cytokine/Chemokine/ Soluble receptor | Estimate | pvalue | Estimate | pvalue |
| Intercept | 3.260 | 0.027 | 2.28 | 0.189 |
| Sgp130 | -7.317 | 0.020 | -8.18 | 0.003 |
| logIP-10 | -0.887 | 0.048 | 0.97 | 0.083 |
| logIL-6 | 0.601 | 0.051 | | |
| logIL-8 | 3.171 | 0.000 | | |
| logEotaxin | -1.026 | 0.006 | | |
| logGN-CSF | | | -0.68 | 0.003 |

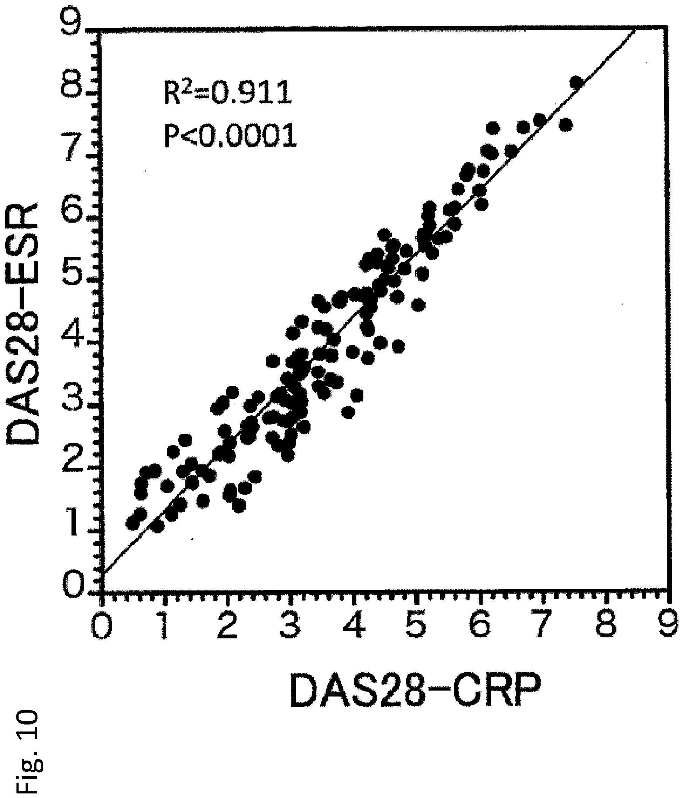

Further, Figure 10 shows a diagram plotting DAS28-CRP and DAS28-ESR scores prior to therapy and after therapy for naive and switch patients who received tocilizumab therapy. It is shown that the values of sgp130, logIL-6, logIL-8, logEotaxin, and logIP-10 are prediction markers (biomarkers) for naive patients, and the values of sgp130, logGM-CSF and logIP-10 are prediction markers (biomarkers) for switch patients.

[Industrial Applicability]

The present invention is applicable in the field of health industry (medical, pharmaceutical) or the like.

**Claims**

1. A method of determining in advance remission for a rheumatoid arthritis patient by a specific biological formulation by measuring a concentration of a specific marker in a body sample of the patient.

2. The method of claim 1, wherein the specific biological formulation is an anti-IL-6 agent, and the specific marker comprises a combination of sgp130 and at least one selected from the group consisting of IP-10, sTNFRII, IL-6, IL-7, MCP-1 and IL-1β.

3. The method of claim 1, wherein the specific biological formulation is an anti-IL-6 agent, and the specific marker comprises a combination of (i) sgp130, (ii) IP-10, (iii) sTNFRII, and (iv) IL-6, IL-7, MCP-1 or IL-Iβ.

4. The method of claim 1, wherein the specific biological formulation is an anti-IL-6 agent, the patient is a rheumatoid arthritis patient who has received anti-cytokine therapy in the past, and the marker is a combination of (i) sgp130, (ii) IP-10, (iii) sTNFRII, and (iv) IL-6 or IL-1β.

5. The method of claim 1, wherein the specific biological formulation is an anti-TNF-α agent, and the specific marker comprises a combination of IL-9 and TNF-α or a combination of VEGF or MIP-1a, PDGFbb and an indicator of a condition prior to therapy of the patient.

6. The method of claim 1, wherein the specific biological formulation is an anti-TNF-α and the specific marker comprises a combination of IL-9 and TNF-α.

7. The method of claim 1, wherein the body sample is a serum.

8. The method of any one of claims 1-7, wherein remission for the patient is determined in advance based on a probability of remission calculated from a regression equation using a value of a concentration of the specific marker or a log value thereof or an indicator of a condition of the patient prior to therapy.

9. The method of claim 8, wherein calculation with the regression equation is performed by using a value of a concentration of the sgp130 and a log value of a concentration for the other specific markers.

10. The method of claim 9, wherein the regression equation is selected from one of regression equations (8)-(16).

11. A method of selecting a biological formulation that is effective for the patient by determining in advance remission due to the specific biological formulation in accordance with the method of any one of claims 1-10 and selecting a specific biological formulation with a high probability of remission.

12. A method of treating a rheumatoid arthritis patient comprising (A) measuring a concentration of a specific marker in a body sample of the rheumatoid arthritis patient to determine in advance remission for the patient due to a specific biological formulation, and (B) when it is determined that remission would occur due to the specific biological formulation by step (A), administering to the patient the specific biological formulation.

13. A method of treating a rheumatoid arthritis patient comprising (A) measuring a concentration of a specific marker in a body sample of the rheumatoid arthritis patient to calculate in advance a probability of remission of the patient due to a plurality of specific biological formulations, and (B) administering to the patient a specific biological formulation with a high probability of remission obtained from step (A).

14. A diagnostic agent comprising a reagent for detecting a specific marker, wherein the diagnostic agent is used in a method of measuring a concentration of the specific marker in a body sample of a rheumatoid arthritis patient to determine in advance remission for the patient due to a specific biological formulation.

15. A diagnostic agent comprising a reagent for detecting a specific marker, wherein the diagnostic agent is used in a method of selecting a biological formulation that is effective for a rheumatoid arthritis patient by measuring a concentration of the specific marker in a body sample of the patient, calculating in advance a probability of remission for the patient due to a plurality of specific biological formulations, and selecting a specific biological formulation with a high probability of remission.

16. A therapeutic agent for treating a rheumatoid arthritis patient comprising a specific biological formulation, **characterized in that** a concentration of a specific marker in a body sample of the patient is measured to determine in advance remission of the patient due to the specific biological formulation and when it is determined that remission would occur, the specific biological formulation is administered.

17. A method of measuring a concentration of a specific marker in a body sample of a rheumatoid arthritis patient to determine in advance a level of improvement in a symptom after therapy for the patient due to a specific biological formulation.

18. The method of claim 17, wherein the specific biological formulation is an anti-IL-6 agent and the specific marker comprises a combination of IL-1β, IL-7, TNF-α, and sIL-6R.

19. The method of claim 17, wherein the specific biological formulation is an anti-TNF-α agent and the specific marker comprises a combination of IL-2, IL-15, sIL-6R, and sTNFRI or a combination of IL-6 and IL-13.

20. The method of any one of claims 17-19, wherein the body sample is a serum.

21. The method of any one of claims 17-20, wherein the level of improvement in a symptom after therapy is determined in advance based on the level of improvement in a symptom after therapy calculated from a regression equation using a value of the concentration of the specific marker or a log value thereof or an indicator of a condition of the patient prior to therapy.

22. The method of claim 21, wherein calculation with the regression equation is performed by using a value of a concentration of the sgp130 and a log value of a concentration for the other specific markers.

23. The method of claim 22, wherein the regression equation is selected from one of regression equations (1)-(2).

24. A method of selecting a biological formulation that is effective for the patient by determining in advance a level of improvement in a symptom after therapy due to the specific biological formulation in accordance with the method of any one of claims 17-23 and selecting a specific biological formulation with a high level of improvement in a symptom after therapy.

25. A method of treating a rheumatoid arthritis patient comprising (A) measuring a concentration of a specific marker in a body sample of the rheumatoid arthritis patient to determine in advance a level of improvement in a symptom after therapy for the patient due to a specific biological formulation, and (B) when the level of improvement determined by step (A) is at or above a predetermined baseline, administering to the patient the specific biological formulation.

26. A method of treating a rheumatoid arthritis patient comprising (A) measuring a concentration of a specific marker in a body sample of the rheumatoid arthritis patient to determine in advance a level of improvement in a symptom after therapy for the patient due to a plurality of specific biological formulations, and (B) administering to the patient a specific biological formulation with a high level of improvement obtained from step (A).

27. A diagnostic agent comprising a reagent for detecting a specific marker, wherein the diagnostic agent is used in a method of measuring a concentration of the specific marker in a body sample of a rheumatoid arthritis patient to determine in advance a level of improvement in a symptom after therapy for the patient due to a specific biological formulation.

28. A diagnostic agent comprising a reagent for detecting a specific marker, wherein the diagnostic agent is used in a method of selecting a biological formulation that is effective for a rheumatoid arthritis patient by measuring a concentration of the specific marker in a body sample of the patient, determining in advance a level of improvement in a symptom after therapy for the patient due to a plurality of specific biological formulations, and selecting a specific biological formulation with a high level of improvement.

29. A therapeutic agent for treating a rheumatoid arthritis patient comprising a specific biological formulation, **characterized in that** a concentration of a specific marker in a body sample of the rheumatoid arthritis patient is measured to determine in advance a level of improvement in a symptom after therapy for the patient due to the specific biological formulation and when the level of improvement is at or above a predetermined baseline, the specific biological formulation is administered.

30. A method of measuring a concentration of a specific marker in a body sample of a rheumatoid arthritis patient to determine in advance the level of improvement in a symptom after therapy due to a specific biological formulation to the patient.

31. The method of claim 30, wherein the specific biological formulation is an anti-IL-6 agent and the specific marker comprises a combination of sgp130, IP-10, and at least one selected from IL-8, Eotaxin, sTNFRI, sTNFRII, IL-6, VEGF, and GM-CSF.

32. The method of claim 30, wherein the specific biological formulation is an anti-IL-6 agent and the specific marker comprises a combination of sgp130, IL-8, Eotaxin, IP-10, sTNFRI, sTNFRII, and IL-6, or a combination of sgp130, IL-8, Eotaxin, IP-10, sTNFRI, sTNFRII, IL-6, and VEGF, and wherein the patient is a rheumatism patient who has not received anti-cytokine therapy in the past

33. The method of item 30, wherein the specific biological formulation is an anti-IL-6 agent and the specific marker comprises a combination of sgp130, IL-10, and GM-CSF, and wherein the patient is a rheumatism patient who has received anti-cytokine therapy in the past.

34. The method of claim 30, wherein the specific biological formulation is an anti-TNF-a agent and the specific marker comprises a combination of IL-9, TNF-$\alpha$, and VEGF or a combination of IL-6 and IL-13.

35. The method of any one of items 30-34, wherein the body sample is a serum.

36. The method of any one of claims 30-35, wherein the disease activity indicator after therapy is determined in advance based on a disease activity indicator after therapy calculated with a regression equation using a value of a concentration of the specific marker or a log value thereof or an indicator of a condition of the patient prior to therapy.

37. The method of claim 36, wherein calculation with the regression equation is performed by using a value of a concentration of the sgp130 and a log value of a concentration for the other specific markers.

38. The method of claim 37, wherein the regression equation is selected from one of regression equations (3)-(7).

39. A method of selecting a biological formulation that is effective for the patient by determining in advance a disease activity indicator after therapy for the patient due to a specific biological formulation in accordance with the method of any one of claims 30-38 and selecting a specific biological formulation with the disease activity indicator that is lower than a predetermined baseline.

40. A method of treating a rheumatoid arthritis patient comprising (A) measuring a concentration of a specific marker in a body sample of the rheumatoid arthritis patient to determine in advance a disease activity indicator after therapy for the patient due to a specific biological formulation, and (B) when the disease activity indicator is at or below a predetermined baseline according to step (A), administering to the patient the specific biological formulation.

41. A method of treating a rheumatoid arthritis patient comprising (A) measuring a concentration of a specific marker in a body sample of the rheumatoid arthritis patient to determine in advance a disease activity indicator after therapy for the patient due to a plurality of specific biological formulations, and (B) administering to the patient a specific biological formulation with a low disease activity indicator obtained from step (A).

42. A diagnostic agent comprising a reagent for detecting a specific marker, wherein the diagnostic agent is used in a method of measuring a concentration of the specific marker in a body sample of a rheumatoid arthritis patient to determine in advance a disease activity indicator after therapy for the patient due to a specific biological formulation.

43. A diagnostic agent comprising a reagent for detecting a specific marker, wherein the diagnostic agent is used in a method of selecting a biological formulation that is effective for a rheumatoid arthritis patient by measuring a concentration of the specific marker in a body sample of the patient, determining in advance a disease activity indicator after therapy for the patient due to a plurality of specific biological formulations, and selecting a specific biological formulation with a low disease activity indicator.

44. A therapeutic agent for treating a rheumatoid arthritis patient comprising a specific biological formulation, **characterized in that** a concentration of a specific marker in a body sample of the patient is measured to determine in advance a disease activity indicator after therapy for the patient due to the specific biological formulation and when the disease activity indicator is at or below a predetermined baseline, the specific biological formulation is administered.

**Naïve patients who received tocilizumab therapy**

Fig. 1

**Switch patients who received tocilizumab therapy**

Fig. 2

Naïve patients who received etanercept therapy

$R^2=0.264$

16wDAS28 predicted value

16wDAS28 actual value

Fig. 3

Fig. 4

Fig. 5

Rheumatoid arthritis patients    n=155

Rheumatoid arthritis patients who received etanercept therapy n=57

Rheumatoid arthritis patients who received tocilizumab therapy    n=98

Switch patients  n=8
F/M:8/0

Naïve patients  n=49
F/M:37/12

Switch patients
n=40
F/M:51/7

Naïve patients  n=58
F/M:51/7

Non-remission
n=5
low=3
moderate=2

Remission
n=3

Non-remission
n=31
low=9
moderate=15
high=6

Remission
n=18

Non-remission
n=30
low=6
moderate=14
high=1

Remission
n=9

Non-remission
n=21
low=6
moderate=14
high=1

Remission
n=27

Fig. 6-1

Fig. 6-2

Fig. 6-3

log GM-CSF (pg/ml)

log IL-12p70 (pg/ml)

log IFN-g (pg/ml)

log IL-13 (pg/ml)

log IP-10 (pg/ml)

log IL-15 (pg/ml)

log MCP-1 (pg/ml)

log IL-17 (pg/ml)

log MIP-1a (pg/ml)

log FGF basic (pg/ml)

Healthy individuals
Tocilizumab_naïve
Tocilizumab_switch
Embrel_naïve

Fig. 6-4

# Fig. 7

A) Naïve patients who received tocilizumab therapy

B) Switch patients who received tocilizumab therapy

C) Naïve patients who received etanercept therapy

DAS-28 value after 16 weeks of therapy

DAS-28-CRP value prior to therapy

Non-remission

High

Medium

Low

Remission

EP 3 309 553 A1

## Fig. 8

a

Actual values of DAS-28 after 16 weeks of etanercept therapy

Predicted values of DAS-28 after 16 weeks of therapy when assuming patients had received etanercept therapy

b

Actual values of DAS-28 after 16 weeks of etanercept therapy

Predicted values of DAS-28 after 16 weeks of therapy when assuming patients had received etanercept therapy

c

Actual values of DAS-28 after 16 weeks of etanercept therapy

Predicted values of DAS-28 after 16 weeks of therapy when assuming patients had received etanercept therapy

EP 3 309 553 A1

Fig. 9

A) Naïve patients who received tocilizumab therapy

B) Switch patients who received tocilizumab therapy

EP 3 309 553 A1

Fig. 10

Fig. 11

**A) Naïve patients who received tocilizumab therapy**

**B) Switch patients who received tocilizumab therapy**

## INTERNATIONAL SEARCH REPORT

| International application No. |
| --- |
| PCT/JP2015/002892 |

| A. | CLASSIFICATION OF SUBJECT MATTER |
| --- | --- |

*G01N33/68*(2006.01)i

According to International Patent Classification (IPC) or to both national classification and IPC

| B. | FIELDS SEARCHED |
| --- | --- |

Minimum documentation searched (classification system followed by classification symbols)
G01N33/68

Documentation searched other than minimum documentation to the extent that such documents are included in the fields searched

| Jitsuyo Shinan Koho | 1922–1996 | Jitsuyo Shinan Toroku Koho | 1996–2015 |
| Kokai Jitsuyo Shinan Koho | 1971–2015 | Toroku Jitsuyo Shinan Koho | 1994–2015 |

Electronic data base consulted during the international search (name of data base and, where practicable, search terms used)
JSTPlus/JMEDPlus/JST7580(JDreamIII), CAplus/MEDLINE/BIOSIS(STN)

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| X<br><br><br><br>A | Kensei TSUZAKA et al., "Kecchu ADAMTS5 Hatsugenryo de Kansetsu Rheumatism ni Okeru Etanercept no Yukosei ga Yosoku Dekiru", Annual General Assembly and Scientific Meeting of the Japan College of Rheumatology Program Shorokushu, 20 March 2015 (20.03.2015), vol.59th, page.482, P1-052 | 1,7,8,14,15, 17,20,21,24, 27,28,30,35, 36,39,42,43<br>2-4,9-11, 31-33,37,38 (Partial) |
| X<br><br><br><br>A | Shusaku NAKASHIMA et al., "Tocilizumab no Chiryo Hannosei o Shimesu Biomarker no Kento", Annual General Assembly and Scientific Meeting of Japan College of Rheumatology/International Rheumatology Symposium Program Shorokushu, 2013, vol.57th-22nd, page 419 | 1,7,8,14,15, 17,20,21,24, 27,28,30,35, 36,39,42,43<br>2-4,9-11, 31-33,37,38 (Partial) |

| ☒ | Further documents are listed in the continuation of Box C. | ☐ | See patent family annex. |
| --- | --- | --- | --- |

| * | Special categories of cited documents: |
| --- | --- |
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
| --- | --- |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
| --- | --- |
| 19 August 2015 (19.08.15) | 01 September 2015 (01.09.15) |

| Name and mailing address of the ISA/ | Authorized officer |
| --- | --- |
| Japan Patent Office<br>3-4-3,Kasumigaseki,Chiyoda-ku,<br>Tokyo 100-8915,Japan | |
| | Telephone No. |

Form PCT/ISA/210 (second sheet) (July 2009)

**INTERNATIONAL SEARCH REPORT**

| International application No. |
|---|
| PCT/JP2015/002892 |

C (Continuation).   DOCUMENTS CONSIDERED TO BE RELEVANT

| Category* | Citation of document, with indication, where appropriate, of the relevant passages | Relevant to claim No. |
|---|---|---|
| X | Tomohiro KAMEDA et al., "Kansetsu Rheumatism ni Okeru Bio-naive Shorei ni Taisuru Tocilizumab no Koka Yosoku no Kento", Annual General Assembly and Scientific Meeting of the Japan College of Rheumatology Program Shorokushu, 2014, vol.58th, page 447 | 1,7,8,14,15, 17,20,21,24, 27,28,30,35, 36,39,42,43 |
| A | | 2-4,9-11, 31-33,37,38 (Partial) |
| A | Misato HASHIZUME et al., "Hitoka Ko IL-6R Kotai tocilizumab no IL-6 Kanren Fukugotai eno Eikyo", Annual General Assembly and Scientific Meeting of Japan College of Rheumatology/International Rheumatology Symposium Program Shorokushu, 2008, vol.52nd-17th, page 317 | 1-4,7-11,14, 15,17,20,21, 24,27,28, 30-33,35-39, 42,43 (Partial) |
| A | US 2010/0298236 A1  (Conaris Research Institute AG), 25 November 2010 (25.11.2010), paragraph [0006] & JP 2011-501944 A      & WO 2009/049881 A1 & EP 2050759 A1 | 1-4,7-11,14, 15,17,20,21, 24,27,28, 30-33,35-39, 42,43 (Partial) |
| A | Avdeev A S et al., The importance of cytokine profile characteristics for evaluating the therapeutic effectiveness of monoclonal antibodies against IL-6 receptors in patients with rheumatoid arthritis, Klinicheskaia meditsina, 2014, Vol. 92, No. 1, pp. 28-34, Abstract | 1-4,7-11,14, 15,17,20,21, 24,27,28, 30-33,35-39, 42,43 (Partial) |
| E,X | WO 2015/083765 A1  (Osaka University), 11 June 2015 (11.06.2015), entire text (Family: none) | 1-4,7-11,14, 15,17,20,21, 24,27,28, 30-33,35-39, 42,43 (Partial) |

Form PCT/ISA/210 (continuation of second sheet) (July 2009)

**EP 3 309 553 A1**

**INTERNATIONAL SEARCH REPORT**

International application No.

PCT/JP2015/002892

---

**Box No. II**  **Observations where certain claims were found unsearchable (Continuation of item 2 of first sheet)**

This international search report has not been established in respect of certain claims under Article 17(2)(a) for the following reasons:

1. ☒ Claims Nos.: 12,13,25,26,40,41
   because they relate to subject matter not required to be searched by this Authority, namely:
   Claims 12, 13, 25, 26, 40 and 41 pertain to therapeutic methods and thus relate to a subject matter on which it is not required to carry out an international search.

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

---

**Box No. III**  **Observations where unity of invention is lacking (Continuation of item 3 of first sheet)**

This International Searching Authority found multiple inventions in this international application, as follows:
   See extra sheet.

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☒ No required additional search fees were timely paid by the applicant.   Consequently, this international search report is restricted to the invention first mentioned in the claims; it is covered by claims Nos.:
   1-4, 7-11, 14, 15, 17, 20, 21, 24, 27, 28, 30-33, 35-39, 42, and 43 (partial)

**Remark on Protest**
☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (July 2009)

**INTERNATIONAL SEARCH REPORT**

International application No.

PCT/JP2015/002892

<u>Continuation of Box No.III of continuation of first sheet(2)</u>

Document 1: Kensei TSUZAKA et al., "Kecchu ADAMTS5 Hatsugenryo de Kansetsu Rheumatism ni Okeru Etanercept no Yukosei ga Yosoku Dekiru", Annual General Assembly and Scientific Meeting of the Japan College of Rheumatology Program Shorokushu, 20 March 2015 (20.03.2015), vol.59th, page 482, P1-052

As disclosed in document 1, it is publicly known that the efficacy of a biological preparation for rheumatoid arthritis is predicted employing a blood biomarker level in a patient before the biological preparation is administered to the patient. Therefore, the matter cannot be regarded as a special technical feature of an invention.

In document 1, an anti-IL-6 agent and an anti-TNF-α agent are disclosed as biological preparations of which the therapeutic effect is to be predicted. Therefore, a matter that the efficacy of an anti-IL-6 agent or an anti-TNF-α agent is evaluated cannot be regarded as a special technical feature, either.

Based on the above-said content, claims of the present application involve fourteen inventions which are classified by the following special technical features of inventions.

Meanwhile, claims 1, 7, 8 (the part referring to claim 1), 14, 15, 17, 20 (the part referring to claim 17), 21 (the part referring to claim 17), 24 (the part referring to claim 17), 27, 28, 30, 35 (the part referring to claim 30), 36 (the part referring to claim 30), 39 (the part referring to claim 30), 42 and 43, which have no special technical feature, are classified into Invention 1.

(Invention 1)

Claim 1, claim 2 (a part which relates to a marker comprising a combination of IP-10 and sgp130), claim 3, claim 4, claim 7, claims 8 to 11 (parts which relate to a marker comprising a combination of IP-10 and sgp130), claim 14, claim 15, claim 17, claim 20 (a part which refers to claim 17), claim 21 (a part which refers to claim 17), claim 24 (a part which refers to claim 17), claim 27, claim 28, claim 30, claims 31 to 33, claim 35 (a part which refers to claims 30 to 33), claim 36 (a part which refers to claims 30 to 33), claims 37 to 38 (parts which refer to claims 31 to 33), claim 39 (a part which refers to claims 30 to 33), claim 42 and claim 43

[STF1] A matter that a marker which comprises a combination of at least IP-10 and sgp130 in a sample from a patient is measured for the purpose of evaluating the efficacy of an anti-IL-6 agent in advance.

(Inventions 2-6)

Claims 2 and 8 to 11 (parts relating to the alternative markers mentioned below)

[STFs 2 to 6] A matter that a marker which comprises a combination of at least IP-10 and sgp130 is measured (STF2), a matter that a marker which comprises a combination of at least IL-6 and sgp130 is measured (STF3), a matter that a marker which comprises a combination of at least IL-7 and sgp130 is measured (STF4), a matter that a marker which comprises a combination of at least MCP-1 and sgp130 is measured (STF5), and a matter that a marker which comprises a combination of at least IL-1β and sgp130 is measured (STF6), each for the purpose of evaluating the efficacy of an anti-IL-6 agent in advance.

(Inventions 7-10)

Claims 5, 6 and 8 to 11 (parts relating to the alternative markers mentioned below), and claim 34 (a part relating to a marker which comprises a combination of IL-9, TNF-α and VEGF)

(Continued to next extra sheet)

Form PCT/ISA/210 (extra sheet) (July 2009)

**INTERNATIONAL SEARCH REPORT**

International application No.

PCT/JP2015/002892

[STFs 7 to 10] A matter that a marker which comprises a combination of IL-9 and TNF-α is measured for the purpose of evaluating the efficacy of an anti-TNF-α agent in advance (STF7); and a matter that a marker which comprises a combination of VEGF and a measure for the condition of the patient before the treatment is measured (STF8), a matter that a marker which comprises a combination of MIP-1a and a measure for the condition of the patient before the treatment is measured (STF9) and a matter that a combination of PDGFbb and a marker which comprises a measure for the condition of the patient before the treatment is measured (STF10), each for the purpose of evaluating the efficacy of an anti-TNF-α agent in advance.

(Invention 11)

Claim 16, claim 29 and claim 44

[STF 11] A therapeutic agent for treating rheumatoid arthritis, which comprises a specific biological preparation.

(Invention 12)

Claim 18 and claims 20-24 (the parts referring to claim 18)

[STF 12] A matter that a marker which comprises a combination of IL-1β, IL-7, TNF-α and sIL-6R is measured for the purpose of evaluating the degree of amelioration of the condition after the treatment with an anti-IL-6 agent in advance.

(Inventions 13-14)

Claim 19, claims 20 to 24 (parts referring to claim 19), and claim 34 (a part relating to a marker which comprises a combination of IL-6 and IL-13)

[STFs 13 to 14] A matter that a marker which comprises a combination of IL-2, IL-15, sIL-6R and sTNFRI is measured for the purpose of evaluating the degree of amelioration of the condition after the treatment with an anti-TNF-α agent in advance (STF13), and a matter that a marker which comprises a combination of IL-6 and IL-13 is measured for the purpose of evaluating the degree of amelioration of the condition after the treatment with an anti-TNF-α agent in advance (STF14).

Meanwhile, Inventions 2-14 are not relevant to inventions which involve all of the invention definition matter of Invention 1 and which have a same category.

Further, as a result of the search which has been carried out with respect to claims classified into Invention 1, Inventions 2-14 are not relevant to inventions on which it is substantially possible to carry out a search without an additional prior-art search and judgment, and there is no other reason for that it can be considered that it is efficient to carry out a search on these Inventions together with Invention 1, and consequently, it is impossible to classify Inventions 2-14 into Invention 1.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2011128096 A
- JP 2011182780 A
- WO 201241332 A
- JP 2009225713 A
- JP 2010088432 A


**Non-patent literature cited in the description**

- **ROSE-JOHN S.** *Int. J. Biol. Sci,* 2012, vol. 8, 1237-1247
- **NISHINA N et al.** *Clin Rheumatol.,* November 2013, vol. 32 (11), 1661-6
- *Ann Rheum Dis.,* March 2007, 407-409
- **STEINBROCKER O et al.** Therapeutic criteria in rheumatoid arthritis. *JAMA,* 1949, vol. 140, 659
- **HOCHBERG MC et al.** *The American College of Rheumatology,* 1991
- *Arthritis and Rheumatism,* 1992, vol. 35, 498-502
- **PERS Y.M. et al.** *Rheumatology,* 2013
- **KOIKE T.** *J. Rheumatology,* November 2013
- **MARKENSON JA et al.** *J. Rheumatology,* July 2011, 1273-81
- **CURTIS JR et al.** *Ann RheumDis.,* 2012, vol. 71, 206-212
- **KOIKE T et al.** *J. Rheumatology,* October 2013, 1658-1668